# EUROPEAN PATENT APPLICATION

(11) **EP 4 349 373 A1**
(43) Date of publication of application: **10.04.2024**
(21) Application number: 22816170.9
(22) Date of filing: 01.06.2022
(51) Int. Cl.: A61K 47/68, A61P 35/00

(54) **CONJUGATE OF ANTIBODY AND FUNCTIONAL SUBSTANCE OR SALT OF SAID CONJUGATE, AND COMPOUND FOR USE IN PRODUCTION OF SAID CONJUGATE OR SALT OF SAID COMPOUND**

(30) Priority: 01.06.2021 US 202163195448 P; 22.02.2022 US 202263312489 P
(71) Applicant: AJINOMOTO CO., INC., Chuo-ku Tokyo 104-8315 (JP)
(72) Inventor: MATSUDA, Yutaka, Kawasaki-shi, Kanagawa 210-8681 (JP); FUJII, Tomohiro, Kawasaki-shi, Kanagawa 210-8681 (JP); HIRAMA, Ryusuke, Kawasaki-shi, Kanagawa 210-8681 (JP); MARKOTAN, Thomas, Princeton, New Jersey 08540 (US); PRIOR, Allan, Princeton, New Jersey 08540 (US); WONG, Christopher, Princeton, New Jersey 08540 (US); PLAKAS, Konstantinos, Princeton, New Jersey 08540 (US); WATANABE, Tomohiro, Kawasaki-shi, Kanagawa 210-8681 (JP)
(74) Representative: Mewburn Ellis LLP
(86) International application number: PCT/JP2022/022393
(87) International publication number: WO 2022/255425

(57) **Abstract**

[PROBLEM] To provide a conjugate of an antibody and a functional substance or a salt thereof excellent in desired properties while controlling a binding ratio between the antibody and the functional substance within a desired range.

[MEANS FOR SOLVING PROBLEM] A conjugate of an antibody and a functional substance or a salt thereof, which comprises a structural unit represented by the following formula (I): 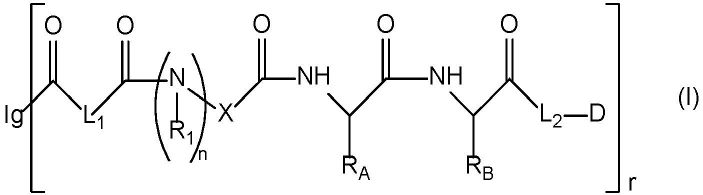 wherein Ig represents an immunoglobulin unit comprising two heavy chains and two light chains, and regioselectively forms an amide bond with a carbonyl group adjacent to Ig via an amino group in side chains of lysine residues in the two heavy chains,
L₁ and L₂ each represent a divalent group,
R₁ represents a monovalent group optionally comprising a hydrophilic group,
X represents a predetermined divalent group,
D represents a functional substance,
R_{A} represents a side chain of a valine residue,
R_{B} represents a side chain of a citrulline residue or an alanine residue,
n is 0 or 1, and
an average ratio r of the amide bonds per two heavy chains is 1.5 to 2.5, and
wherein at least one hydrophilic group is present in the structural unit.

## Description

### TECHNICAL FIELD

The present invention relates to a conjugate of an antibody and a functional substance or a salt thereof, a compound used in production of the same or a salt thereof, and the like.

### BACKGROUND

In recent years, research and development of an antibody drug conjugate (ADC) have been actively performed. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) jointly developed by Immunogene Inc. and Roche Inc.

An ADC is produced by causing a functional group in a side chain of a specific amino acid residue present in an antibody to bind to a drug. Examples of such a functional group used in producing an ADC include an amino group in a side chain of a lysine residue present in an antibody. Several techniques have been reported as a technique for regioselectively modifying a lysine group (e.g., a lysine residue at position 246/248, position 288/290, or position 317) in an antibody (e.g., Patent Literatures 1 to 4).

In an ADC, an antibody and a drug are linked with each other via a linker. There are various linkers in an ADC. For example, in an ADC used as an anti-cancer agent, there is a linker comprising a dipeptide consisting of valine-citrulline (Val-Cit: VC structure) as a linker that is stable in human plasma and has a structure cleavable by a specific enzyme for releasing a drug in cancer cells. A linker comprising such a dipeptide is stable in human plasma as illustrated in the following (A). However, as illustrated in the following (B), a VC structure is recognized by cathepsin B in lysosomes in human cancer cells, and an amide bond present on a carboxy terminal side of citrulline is cleaved. Therefore, an ADC having a linker comprising such a dipeptide can release a drug and exhibit drug efficacies in human cancer cells.

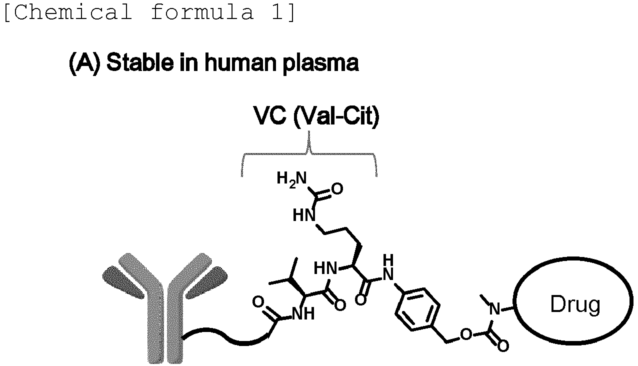

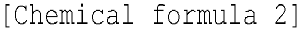

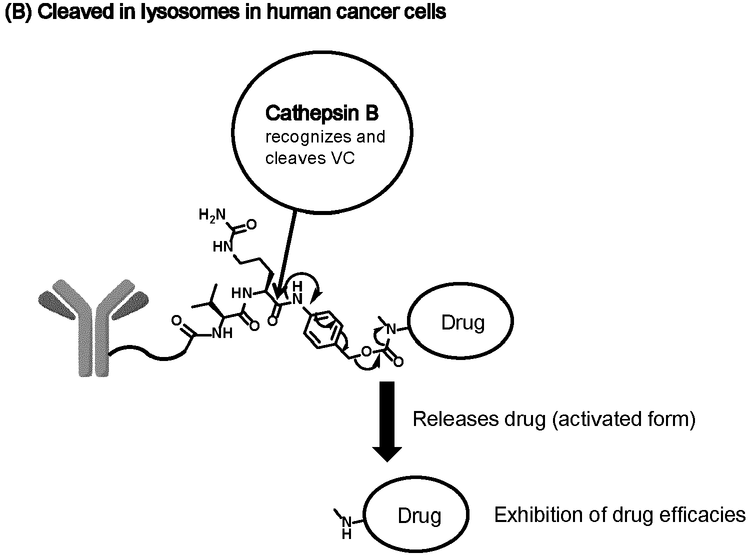

However, an ADC having a linker comprising such a dipeptide is unstable in mouse plasma (Non-Patent Literatures 1 and 2). This is because Cesic, which is a carboxylase that recognizes a VC structure and cleaves an amide bond present on a carboxy terminal side of citrulline, is present in mouse plasma, and therefore a linker comprising such a dipeptide is cleaved in the plasma by Ceslc. Therefore, the ADC having a linker comprising such a dipeptide largely differs in pharmacokinetics between mice and humans. Therefore, with mice, there is a problem that it is difficult to evaluate a drug efficacy in humans.

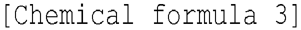

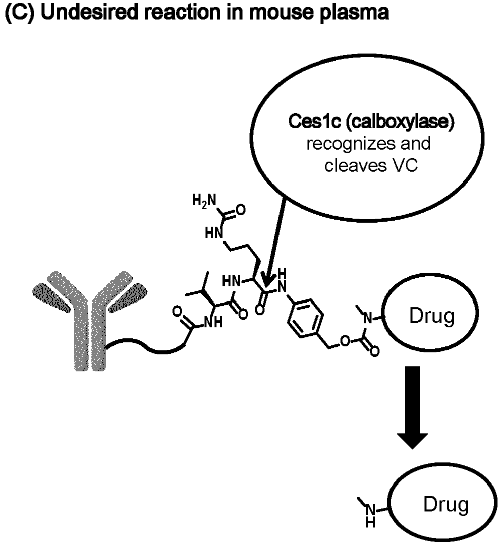

In order to improve instability in mouse plasma for an ADC having a structure "antibody-spacer-VC structure-spacer-drug" as described above, attempts have been made to stabilize the ADC by modifying a linker (that is, spacer-VC structure-spacer).

For example, Patent Literature 5 and Non-Patent Literature 3 disclose that a linker is stabilized by introducing a hydrophilic group (PEG) into a spacer present between a VC structure and a drug (in the vicinity of a cleavage site by Cesic).

On the other hand, a technique for stabilizing a linker by introducing a specific group into a spacer present between an antibody and a VC structure has also been reported. Examples of a linker stabilized by such a technique include the following linkers 1) to 4) characterized by comprising a structure in which at least one α-amino acid residue (that is, X or NH-C(R)-CO) is linked to a position of a VC structure on an antibody side (amino group of V) by an amide bond (that is, a structure represented by X-Val-Cit or NH-C(R)-CO-Val-Cit):
1) a linker comprising a tripeptide structure (Glu-Val-Cit) in which a glutamic acid residue is linked to an N-terminal of Val (Patent Literature 6);
2) a linker comprising a structure (Asp-Val-Cit) in which an aspartic acid residue is linked to an N-terminal of Val (Non-Patent Literature 4);
3) a linker comprising a structure represented by NH-C(R)-CO-Val-Cit (where R represents a side chain having a hydrophilic group such as PEG) (Patent Literatures 7 to 9);
4) a linker comprising a structure represented by NH-C(R)-CO-Val-Cit (where R represents a side chain having a hydrophilic group such as a sulfonate group or a sugar) (Patent Literature 10); and
5) a linker comprising a structure represented by NH-C(R)-CO-Val-Cit (where R represents a side chain having cyclodextrin) (Patent Literature 11).

As other examples of the linker having a specific group introduced into a spacer present between an antibody and a VC structure, the following linkers 6) to 8) have been reported:
6) a linker comprising a structure represented by C(M)-CO-Val-Cit (where C in C(M) represents a carbon atom, M in C(M) represents a stability adjusting group comprising a side chain such as an aromatic ring group, and CO represents a carbonyl group that is linked to an amino group of a valine residue to form an amide bond) (Patent Literature 12);
7) a highly controlled special PEG linker comprising PEG in both a main chain and a side chain of a spacer present between an antibody and a VC structure (Non-Patent Literature 5); and
8) a linker comprising a structure represented by C(Rᵢ)-NH-CO-Rᵢᵢ-CO-Cit (where Rᵢ represents a thiophenyl group, and Rᵢᵢ represents a cyclobutyl ring) in which a structure of C(Rᵢ)-NH is linked to a position of a VC analog structure (CO-Rᵢᵢ-CO-Cit) on an antibody side by an amide bond (Patent Literature 13).

Meanwhile, Non-Patent Literature 6 describes that the higher hydrophobicity of an ADC, the faster a plasma clearance, and that the hydrophobicity of an ADC can be evaluated by hydrophobic interaction chromatography (HIC)-HPLC.

### PRIOR ART LITERATURES

### PATENT LITERATURES

Patent Literature 1: WO 2018/199337 A
Patent Literature 2: WO 2019/240288 A
Patent Literature 3: WO 2019/240287 A
Patent Literature 4: WO 2020/090979 A
Patent Literature 5: US 2019/0365915 A
Patent Literature 6: WO 2018/218004 A
Patent Literature 7: WO 2019/094395 A
Patent Literature 8: US 2016/0310612 A
Patent Literature 9: WO 2020/252043 A
Patent Literature 10: WO 2020/236841 A
Patent Literature 11: WO 2018/213077 A
Patent Literature 12: JP 2016-050204 B2
Patent Literature 13: WO 2016/090038 A

### NON-PATENT LITERATURES

Non-Patent Literature 1: Dorywalska et al., Bioconjugate Chem., 2015, 26 (4), 650-659
Non-Patent Literature 2: Dorywalska et al., Mol Cancer Ther., 2016, 15 (5), 958-70
Non-Patent Literature 3: Poudel et al., ACS Med Chem Lett., 2020, 11 (11), 2190-2194
Non-Patent Literature 4: Ratnayake et al., Bioconjug Chem., 2019, 30 (1), 200-209
Non-Patent Literature 5: Walker et al., Bioconjug Chem. 2019, 30 (11), 2982-2988
Non-Patent Literature 6: Lyon et al., Nat Biotechnol., 2015, 33 (7), 733-5

### SUMMARY OF THE INVENTION

### PROBLEM TO BE SOLVED BY THE INVENTION

The object of the present invention is to provide a conjugate of an antibody and a functional substance or a salt thereof excellent in desired properties while controlling a binding ratio between the antibody and the functional substance within a desired range.

### MEANS FOR SOLVING PROBLEM

As a result of intensive studies, the present inventors have found that by selecting a lysine residue in a heavy chain in an immunoglobulin unit as a modification position of an antibody, and using a linker comprising a specific structural unit [that is, CO-(N-R₁)n-X-CONH-CH₂(-R_{A})-CONH-CH₂(-R_{B})-CO described later] for linking the immunoglobulin unit to a functional substance, a conjugate represented by formula (I) or a salt thereof excellent in desired properties can be easily produced while an average ratio of binding between the immunoglobulin unit and the functional substance (functional substance/immunoglobulin unit) is highly controlled within a desired range (1.5 to 2.5). For example, such a conjugate or a salt thereof can have one or more desired properties selected from the group consisting of excellent clearance (that is, a long residence time in the body), a low aggregation ratio, high cleavability by cathepsin B, and high stability in mouse plasma.

The present inventors have also found that among conjugates represented by formula (I), conjugates represented by formulae (I-1) to (I-3) can be excellent in the above desired properties, thereby completing the present invention.

For example, while the conjugates represented by formulae (I-1) and (I-2) each have a structural unit in which a tertiary amide-type structure having a group comprising a hydrophilic group is linked to a VC structure in a linker for linking an antibody and a functional substance to each other, the above prior art neither teaches nor suggests the conjugate of the present invention comprising a linker having such a structural unit.

In addition, while the conjugate represented by formula (I-3) has a structural unit in which a γ-glutamic acid residue is linked to a VC structure in a linker for linking an antibody and a functional substance to each other, the above prior art neither teaches nor suggests the conjugate comprising a linker having such a structural unit. Indeed, Patent Literatures 6 to 11 each disclose a linker comprising a structural unit in which at least one α-amino acid residue (that is, an α-type structure) is linked to a VC structure, but neither teaches nor suggests a linker comprising a structural unit in which a γ-glutamic acid residue is linked to a VC structure. Patent Literatures 12 and 13 and Non-Patent Literature 5 neither teach nor suggest a linker comprising a structural unit in which a γ-glutamic acid residue is linked to a VC structure.

Specifically, the present invention is as follows.
[1] A conjugate of an antibody and a functional substance or a salt thereof, which comprises a structural unit represented by formula (I),wherein at least one hydrophilic group is present in the structural unit.
[2] The conjugate or a salt thereof according to [1], wherein the immunoglobulin unit is a human immunoglobulin unit.
[3] The conjugate or a salt thereof of [2], wherein the human immunoglobulin unit is a human IgG antibody.
[4] The conjugate or a salt thereof according to any of [1] to [3], wherein the lysine residue is present at position 246/248, position 288/290, or position 317 in accordance with Eu numbering.
[5] The conjugate or a salt thereof according to any of [1] to [4], wherein r is 1.9 to 2.1.
[6] The conjugate or a salt thereof according to any of [1] to [5], wherein the hydrophilic group is one or more groups selected from the group consisting of a carboxylate group, a sulfonate group, a hydroxy group, a polyethylene glycol group, a polysarcosine group, and a sugar portion.
[7] The conjugate or a salt thereof according to any of [1] to [6], wherein L₁ represents a divalent group represented by formula (i).
[8] The conjugate or a salt thereof according to [7], wherein L₃ and L₄ each independently represent -(C(R)₂)ₘ-.
[9] The conjugate or a salt thereof according to [7] or [8], wherein Y is a divalent group represented by any of the following structural formulae: 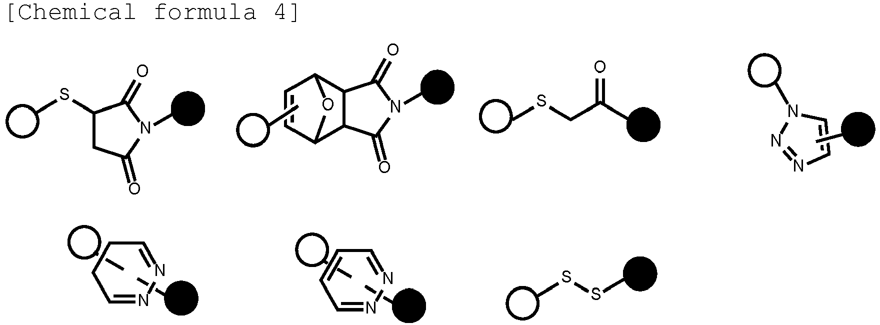
   wherein a white circle and a black circle each represent a bond,
   when the bond of the white circle is bonded to L₃, the bond of the black circle is bonded to L₄, and
   when the bond of the white circle is bonded to L₄, the bond of the black circle is bonded to L₃.
[10] The conjugate or a salt thereof according to any of [1] to [9], wherein the structural unit represented by formula (I) is a structural unit represented by formula (I-1), (I-2), (I-3), or (I-4).
[11] The conjugate or a salt thereof according to [10], wherein the structural unit represented by formula (I) is a structural unit represented by formula (I-1), (I-2), or (I-3) .
[12] The conjugate or a salt thereof according to [10], wherein the structural unit represented by formula (I-1), (I-2), (I-3), or (I-4) is a structural unit represented by formula (I-1a'), (I-1b'), (I-1c'), (I-2'), (I-3a'), (I-3b'), (1-4'), (I-4b'), or (I-4c').
[13] The conjugate or a salt thereof according to [12], wherein the structural unit represented by formula (I-1a'), (I-1b'), (I-1c'), (I-2'), (I-3a'), (I-3b'), or (I-4') is a structural unit represented by formula (I-1a'-1), (I-1a'-2), (I-1b'-1), (I-1c'-1), (I-2'-1), (I-2'-2), (I-3a'-1), (I-3a'-2), (I-3b'-1), (I-4a'-1) , (I-4b'-1), (I-4c'-1), or (1-4c'-2).
[14] The conjugate or a salt thereof according to any of [1] to [13], wherein L₂ is a divalent group represented by the following structural formula: 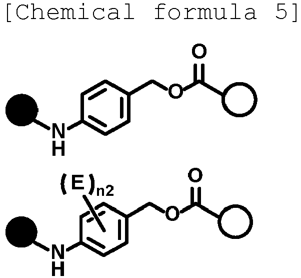
   wherein a black circle and a white circle each represent a bond,
   the bond of the black circle is bonded to a carbonyl group adjacent to L₂,
   the bond of the white circle is bonded to D,
   E represents an electron-withdrawing group, and
   n2 is an integer of 1 to 4.
[15] A compound or a salt thereof represented by formula (II-1), (II-2), (II-3) , (II-4b'), or (II-4c').
[16] The compound or a salt thereof according to [15], wherein the bioorthogonal functional group is a maleimide residue, a thiol residue, a furan residue, a halocarbonyl residue, an alkene residue, an alkyne residue, an azide residue, or a tetrazine residue.
[17] The compound or a salt thereof according to [15] or [16], wherein the compound represented by formula (II-1), (II-2), or (II-3) is represented by formula (II-1a'), (II-1b'), (II-1c'), (II-2'), (II-3a'), or (II-3b').
[18] The compound or a salt thereof according to any of [15] to [17], wherein the compound represented by formula (II-1a'), (II-1b'), (II-1c'), (11-2'), (II-3a'), (II-3b'), (II-4b'), or (II-4c') is represented by formula (II-1a'-1), (II-1a'-2), (II-1b'-1), (II-1c'-1), (II-2'-1), (II-2'-2), (II-3a'-1), (II-3a'-2) , (II-3b'-1), (II-4b'-1) , (II-4c'-1) or (II-4c'-2).
[19] The compound or a salt thereof according to any of [15] to [18], wherein L₂ is a divalent group represented by the following structural formula: 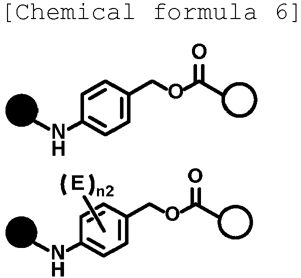
   wherein, a black circle and a white circle each represent a bond,
   the bond of the black circle is bonded to a carbonyl group adjacent to L₂, and
   the bond of the white circle is bonded to D,
   E represents an electron-withdrawing group, and
   n2 is an integer of 1 to 4.
[20] A reagent for derivatizing an antibody, the reagent comprising the compound or a salt thereof according to any of [15] to [19].

### EFFECT OF THE INVENTION

The conjugate of the present invention or a salt thereof can be excellent in desired properties while highly controlling an average ratio of binding between an immunoglobulin unit and a functional substance (functional substance/immunoglobulin unit) to a desired range (1.5 to 2.5) .

The compound of the present invention or a salt thereof, and a reagent of the present invention are useful as synthetic intermediates in production of the conjugate.

### EMBODIMENTS FOR CARRYING OUT THE INVENTION

### 1.Definitions of general terms

In the present invention, the term "antibody" is as follows. The term "immunoglobulin unit" corresponds to a divalent monomer unit that is a basic constituent element of such an antibody, and is a unit comprising two heavy chains and two light chains. Therefore, definitions, examples, and preferred examples of the origin, type (polyclonal or monoclonal, isotype, and full-length antibody or antibody fragment), antigen, position of a lysine residue, and regioselectivity of the immunoglobulin unit are similar to those of the antibody described below.

The origin of the antibody is not particularly limited, and for example, the antibody may be derived from an animal such as a mammal or a bird (e.g., a domestic fowl). The immunoglobulin unit is preferably derived from a mammal. Examples of such a mammal include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). Primates and rodents are preferred, and humans are more preferred.

The type of antibody may be a polyclonal antibody or a monoclonal antibody. The antibody may be a divalent antibody (e.g., IgG, IgD, or IgE) or a tetravalent or higher antibody (e.g., IgA antibody or IgM antibody). The antibody is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, Fc region proteins, and Fc-fusion proteins. Examples of the isotype of the monoclonal antibody include IgG (e.g., IgG1, IgG2, IgG3, and IgG4), IgM, IgA, IgD, IgE, and IgY. In the present invention, as the monoclonal antibody, a full-length antibody or an antibody fragment comprising a variable region and a CH1 domain and a CH2 domain can be used, but a full-length antibody is preferred. The antibody is preferably a human IgG monoclonal antibody, and more preferably a human IgG full-length monoclonal antibody.

As an antigen of the antibody, any antigen can be used. Examples of such an antigen include a protein [which comprises an oligopeptide and a polypeptide, and may be a protein modified with a biomolecule such as a sugar (e.g., a glycoprotein)], a sugar chain, a nucleic acid, and a small compound. The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, and Siglec-15

### (8) Blood Diseases

vWF, Factor IXa, Factor X, IFNγ, C5, BMP-6, Ferroportin, and TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

Specific examples of the monoclonal antibody include specific chimeric antibodies (e.g., rituximab, basiliximab, infliximab, cetuximab, siltuximab, dinutuximab, and altertoxaximab), specific humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and specific human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The positions of amino acid residues in the antibody and the position of a constant region of a heavy chain (e.g., CH2 domain) are in accordance with EU numbering (see, http://www.imgt.org/IMGTScientificChart/Numbering/Hu_IGHGnb er. html). For example, when human IgG is a target, a lysine residue at position 246 corresponds to an amino acid residue at position 16 of a human IgG CH2 region, a lysine residue at position 248 corresponds to an amino acid residue at position 18 of a human IgG CH2 region, a lysine residue at position 288 corresponds to an amino acid residue at position 58 of a human IgG CH2 region, a lysine residue at position 290 corresponds to an amino acid residue at position 60 of a human IgG CH2 region, and a lysine residue at position 317 corresponds to an amino acid residue at position 87 of a human IgG CH2 region. The notation at position 246/248 indicates that a lysine residue at position 246 or position 248 is a target. The notation at position 288/290 indicates that a lysine residue at position 288 or position 290 is a target.

According to the present invention, a specific lysine residue (e.g., a lysine residue at position 246/248, position 288/290, or position 317) in a heavy chain in an immunoglobulin unit constituting an antibody can be regioselectively modified (see, e.g., WO 2018/199337 A, WO 2019/240288 A, WO 2019/240287 A, and WO 2020/090979 A). In the present specification, "regioselective" or "regioselectivity" refers to a state in which even though a specific amino acid residue is not present locally in a specific region in the antibody, a certain structural unit capable of binding to the specific amino acid residue in the antibody is present locally in a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues of the same type as the specific amino acid residues in the target region. Such regioselectivity may be 50% or more, preferably 60% or more, more preferably 70% or more, even more preferably 80% or more, and particularly preferably 90% or more, 95% or more, 96% or more, 97% or more, 98% or more, 99% or more, 99.5% or more, or 1000.

In the present invention, as long as a specific lysine residue in a heavy chain in an antibody is regioselectively modified, a specific lysine residue at another position may be further regioselectively modified. For example, a method for regioselectively modifying a specific amino acid residue at a predetermined position in an antibody is described in WO 2018/199337 A, WO 2019/240288 A, WO 2019/240287 A, and WO 2020/090979 A. As such a specific amino acid residue, an amino acid residue (e.g., a lysine residue, an aspartic acid residue, a glutamic acid residue, an asparagine residue, a glutamine residue, a threonine residue, a serine residue, a tyrosine residue, or a cysteine residue) having a side chain that is easily modified (e.g., an amino group, a carboxy group, an amide group, a hydroxy group, or a thiol group) can be used. However, a lysine residue having a side chain comprising an amino group, a tyrosine residue having a side chain comprising a hydroxy group, a serine residue, a threonine residue, or a cysteine residue having a side chain comprising a thiol group may be preferred, and a lysine residue may be more preferred (that is, out of a lysine residue at position 246/248, a lysine residue at position 288/290, and a lysine residue at position 317, two lysine residues may be double modified regioselectively, or three lysine residues may be triple modified regioselectively).

### (Halogen atom)

Examples of the halogen atom include a fluorine atom, a chlorine atom, a bromine atom, and an iodine atom.

### (Monovalent group)

Examples of the monovalent group include a monovalent hydrocarbon group and a monovalent heterocyclic group.

The monovalent group may have one or more (e.g., 1 to 10, preferably 1 to 8, more preferably 1 to 6, even more preferably 1 to 5, particularly preferably 1 to 3) substituents described later.

### (Monovalent hydrocarbon group and terms related thereto)

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. The alkyl, alkenyl, and alkynyl may be linear or branched.

The alkyl is preferably C₁₋₁₂ alkyl, more preferably C₁₋₆ alkyl, and even more preferably C₁₋₄ alkyl. When the alkyl has a substituent, the number of carbon atoms does not comprise the number of carbon atoms of the substituent. Examples of the C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C₂₋₁₂ alkenyl, more preferably C₂₋₆ alkenyl, and even more preferably C₂₋₄ alkenyl. When the alkenyl has a substituent, the number of carbon atoms does not comprise the number of carbon atoms of the substituent. Examples of the C₂₋₁₂ alkenyl include vinyl, propenyl, and n-butenyl.

The alkynyl is preferably C₂₋₁₂ alkynyl, more preferably C₂₋₆ alkynyl, and even more preferably C₂₋₄ alkynyl. When the alkynyl has a substituent, the number of carbon atoms does not comprise the number of carbon atoms of the substituent. Examples of the C₂₋₁₂ alkynyl include ethynyl, propynyl, and n-butynyl.

The monovalent chain hydrocarbon group is preferably alkyl.

The monovalent alicyclic hydrocarbon group means a hydrocarbon group comprising only an alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

The cycloalkyl is preferably C₃₋₁₂ cycloalkyl, more preferably C₃₋₆ cycloalkyl, and even more preferably C₅₋₆ cycloalkyl. When the cycloalkyl has a substituent, the number of carbon atoms does not comprise the number of carbon atoms of the substituent. Examples of the C₃₋₁₂ cycloalkyl include cyclopropyl, cyclobutyl, cyclopentyl, and cyclohexyl.

The cycloalkenyl is preferably C₃₋₁₂ cycloalkenyl, more preferably C₃₋₆ cycloalkenyl, and even more preferably C₅₋₆ cycloalkenyl. When the cycloalkenyl has a substituent, the number of carbon atoms does not comprise the number of carbon atoms of the substituent. Examples of the C₃₋₁₂ cycloalkenyl include cyclopropenyl, cyclobutenyl, cyclopentenyl, and cyclohexenyl.

The cycloalkynyl is preferably C₃₋₁₂ cycloalkynyl, more preferably C₃₋₆ cycloalkynyl, and even more preferably C₅₋₆ cycloalkynyl. When the cycloalkynyl has a substituent, the number of carbon atoms does not comprise the number of carbon atoms of the substituent. Examples of the C₃₋₁₂ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C₆₋₁₂ aryl, more preferably C₆₋₁₀ aryl, and even more preferably C₆ aryl. When the monovalent aromatic hydrocarbon group has a substituent, the number of carbon atoms does not comprise the number of carbon atoms of the substituent. Examples of the C₆₋₁₂ aryl include phenyl and naphthyl.

The monovalent aromatic hydrocarbon group is preferably phenyl.

Among these groups, the monovalent hydrocarbon group is preferably alkyl, cycloalkyl, or aryl.

### (Monovalent heterocyclic group and terms related thereto)

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom comprised in the heterocyclic group.

The monovalent aromatic heterocyclic group is preferably a C₁₋₁₅ aromatic heterocyclic group, more preferably a C₁₋₉ aromatic heterocyclic group, and even more preferably a C₁₋₆ aromatic heterocyclic group. When the monovalent aromatic heterocyclic group has a substituent, the number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and even more preferably a C₂₋₆ nonaromatic heterocyclic group. When the monovalent nonaromatic heterocyclic group has a substituent, the number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these groups, the monovalent heterocyclic group is preferably a five-membered or six-membered heterocyclic group.

### (Divalent group)

The divalent group is a divalent linear hydrocarbon group, a divalent cyclic hydrocarbon group, a divalent heterocyclic group, one group selected from the group consisting of -C(=O)-, -C(=S)-, -NR₇-, -C(=O)-NR₇-, -NR₇-C(=O)-, -C(=S)-NR₇-, -NR₇-C(=S)-, -O-, -S-, -(O-R₈)ₘ-, and - (S-R₈)ₘ₁-, or a group having a main chain structure comprising two or more (e.g., 2 to 10, preferably 2 to 8, more preferably 2 to 6, even more preferably 2 to 5, particularly preferably 2 or 3) of these groups. R₇ represents a hydrogen atom or a substituent described later. R₈ represents a divalent linear hydrocarbon group, a divalent cyclic hydrocarbon group, or a divalent heterocyclic group. m1 is an integer of 1 to 10, preferably an integer of 1 to 8, more preferably an integer of 1 to 6, even more preferably an integer of 1 to 5, and particularly preferably an integer of 1 to 3.

The divalent linear hydrocarbon group is a linear alkylene, a linear alkenylene, or a linear alkynylene.

The linear alkylene is a C₁₋₆ linear alkylene, and is preferably a C₁₋₄ linear alkylene. Examples of the linear alkylene include methylene, ethylene, n-propylene, n-butylene, n-pentylene, and n-hexylene.

The linear alkenylene is a C₂₋₆ linear alkenylene, and is preferably a C₂₋₄ linear alkenylene. Examples of the linear alkenylene include ethylenylene, n-propynylene, n-butenylene, n-pentenylene, and n-hexenylene.

The linear alkynylene is a C₂₋₆ linear alkynylene, and is preferably a C₂₋₄ linear alkynylene. Examples of the linear alkynylene include ethynylene, n-propynylene, n-butynylene, n-pentynylene, and n-hexynylene.

The divalent linear hydrocarbon group is preferably a linear alkylene.

The divalent cyclic hydrocarbon group is an arylene or a divalent nonaromatic cyclic hydrocarbon group.

The arylene is preferably a C₆₋₁₄ arylene, more preferably a C₆₋₁₀ arylene, and particularly preferably a C₆ arylene. Examples of the arylene include phenylene, naphthylene, and anthracenylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, and particularly preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C₃₋₁₅ divalent aromatic heterocyclic group, more preferably a C₃₋₉ divalent aromatic heterocyclic group, and particularly preferably a C₃₋₆ divalent aromatic heterocyclic group. Examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C₃₋₁₅ nonaromatic heterocyclic group, more preferably a C₃₋₉ nonaromatic heterocyclic group, and particularly preferably a C₃₋₆ nonaromatic heterocyclic group. Examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, pyrrolinediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent heterocyclic group is preferably a divalent aromatic heterocyclic group.

The divalent group is preferably a divalent group having a main chain structure comprising one group selected from the group consisting of alkylene, arylene, -C(=O)-, - NR₇-, -C(=O)-NR₇-, -NR₇-C(=O)-, -O-, and -(O-R₈)ₘ-, or
a divalent group having a main chain structure comprising two or more groups selected from the group consisting of alkylene, arylene, -C(=O)-, -NR₇-, -C(=O)-NR₇-, -NR₇-C(=O)-, -O-, and -(O-R₈)ₘ₁-,
R₇ is a hydrogen atom or an alkyl,
R₈ is an alkylene or an arylene, and
m1 may be an integer of 1 to 5 (that is, 1, 2, 3, 4, or 5) .

The alkylene, the arylene, and the alkyl are similar to those described above.

The main chain structure in the divalent group may have one or more (e.g., 1 to 10, preferably 1 to 8, more preferably 1 to 6, even more preferably 1 to 5, particularly preferably 1 to 3) substituents described later.

### (Substituent)

Examples of the substituent include:
(i) a halogen atom;
(ii) a monovalent hydrocarbon group;
(iii) a monovalent heterocyclic group;
(iv) an aralkyl;
(v) Rₐ-O-, Rₐ-C(=O)-, Rₐ-O-C(=O)-, or Rₐ-C(=O)-O-, (where Rₐ represents a hydrogen atom or a monovalent hydrocarbon group);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a monovalent hydrocarbon group); and
(vii) a nitro group, a sulfate group, a sulfonate group, a cyano group, and a carboxyl group.

Definitions, examples, and preferred examples of the halogen atom, the monovalent hydrocarbon group, and the monovalent heterocyclic group in the substituent are similar to those described above.

The aralkyl refers to arylalkyl. Definitions, examples, and preferred examples of the aryl and the alkyl in the arylalkyl are as described above. The aralkyl is preferably C₃₋₁₅ aralkyl. Examples of such an aralkyl include benzoyl, phenethyl, naphthylmethyl, and naphthylethyl.

The substituent may be preferably:
(i) a halogen atom;
(ii) a C₁₋₁₂ alkyl, a C₁₋₁₂ phenyl, or a C₁₋₁₂ naphthyl;
(iii) a C₃₋₁₅ aralkyl;
(iv) a 5- or 6-membered heterocycle;
(v) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(vi) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(vii) the same groups as listed in the above (vii).

The substituent may be more preferably:
(i) a halogen atom;
(ii) a C₁₋₁₂ alkyl;
(iii) Rₐ-O-, Rₐ-C(=O)-, Rₐ-O-C(=O)-, or Rₐ-C(=O)-O-, (where Rₐ represents a hydrogen atom or a C₁₋₁₂ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₁₂ alkyl); or
(v) the same groups as listed in the above (vii).

The substituent may be even more preferably:
(i) a halogen atom;
(ii) a C₁₋₆ alkyl;
(iii) Rₐ-O-, Rₐ-C(=O)-, Rₐ-O-C(=O)-, or Rₐ-C(=O)-O-, (where Rₐ represents a hydrogen atom or a C₁₋₆ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₆ alkyl); or
(v) the same groups as listed in the above (vii).

The substituent may be particularly preferably:
(i) a halogen atom;
(ii) a C₁₋₄ alkyl;
(iii) Rₐ-O-, Rₐ-C(=O)-, Rₐ-O-C(=O)-, or Rₐ-C(=O)-O-, (where Rₐ represents a hydrogen atom or a C₁₋₄ alkyl);
(iv) NR_{b}R_{c}-, NR_{b}R_{c}-C(=O)-, NR_{b}R_{c}-C(=O)-O-, or R_{b}-C(=O)-NR_{c}-, (where R_{b} and R_{c} are the same as or different from each other, and each represent a hydrogen atom or a C₁₋₄ alkyl); or
(v) the same groups as listed in the above (vii).

### (Hydrophilic group)

The hydrophilic group is a group that can make a structural unit represented by formula (I) or a formula of a subordinate concept thereof more hydrophilic. By having a hydrophilic group at a predetermined site in the structural unit, the conjugate can be further stabilized in mouse plasma. Examples of such a hydrophilic group include a carboxylate group, a sulfonate group, a hydroxy group, a polyethylene glycol group, a polysarcosine group, and a sugar portion. The conjugate may comprise one or more (e.g., 1, 2, 3, 4, or 5) hydrophilic groups.

The polyethylene glycol group is a divalent group represented by -(CH₂-CH₂-O-)ₖ₁-. When the conjugate has a polyethylene glycol group, the conjugate may have a monovalent group in which one bond of the polyethylene glycol group is bonded to a hydrogen atom or a monovalent group (e.g., a monovalent hydrocarbon group). k1 may be, for example, an integer of 3 or more, preferably an integer of 4 or more, more preferably an integer of 5 or more, and even more preferably an integer of 6 or more. k1 may also be an integer of 20 or less, preferably an integer of 15 or less, more preferably an integer of 12 or less, and even more preferably an integer of 10 or less. More specifically, k1 may be an integer of 3 to 20, preferably an integer of 4 to 15, more preferably an integer of 5 to 12, and even more preferably an integer of 6 to 10

The polysarcosine group is a divalent group represented by -(NCH₃-CH₂-CO-)ₖ₂-. The polysarcosine group can be used as an alternative to PEG. k2 may be, for example, an integer of 3 or more, preferably an integer of 4 or more, more preferably an integer of 5 or more, and even more preferably an integer of 6 or more. k2 may also be an integer of 20 or less, preferably an integer of 15 or less, more preferably an integer of 12 or less, and even more preferably an integer of 10 or less. More specifically, k2 may be an integer of 3 to 20, preferably an integer of 4 to 15, more preferably an integer of 5 to 12, and even more preferably an integer of 6 to 10.

The sugar portion is a monosaccharide, an oligosaccharide (e.g., a disaccharide, a trisaccharide, a tetrasaccharide, or a pentasaccharide), or a polysaccharide. The sugar portion can comprise an aldose or a ketose, or a combination thereof. The sugar portion may be a monosaccharide such as ribose, deoxyribose, xylose, arabinose, glucose, mannose, galactose, fructose, or an amino sugar (e.g., glucosamine), or an oligosaccharide or a polysaccharide comprising such a monosaccharide.

In a specific embodiment, the sugar portion may be a low molecular weight hydrophilic group. The low molecular weight hydrophilic group refers to a hydrophilic group having a molecular weight of 1500 or less. The molecular weight of the low molecular weight hydrophilic group may be 1,200 or lower, 1,000 or lower, 800 or lower, 700 or lower, 600 or lower, 500 or lower, 400 or lower, 300 or lower, 200 or lower, or 100 or lower. Examples of the low molecular weight hydrophilic group include a carboxylate group, a sulfonate group, a hydroxy group, and a polyethylene glycol group, a polysarcosine group, and a sugar portion (e.g., a monosaccharide or an oligosaccharide) satisfying the above molecular weight.

### (Bioorthogonal functional group)

The bioorthogonal functional group refers to a group that does not react with biological components (e.g., amino acids, proteins, nucleic acids, lipids, sugars, and phosphoric acids) or has a low reaction rate to the biological components but selectively reacts with components other than the biological components. The bioorthogonal functional group is well known in the technical field concerned (see, e.g., Sharpless K. B. et al., Angew. Chem. Int. Ed. 40, 2004 (2015); Bertozzi C. R. et al., Science 291,2357 (2001); Bertozzi C. R. et al., Nature Chemical Biology 1,13 (2005)).

In the present invention, as the bioorthogonal functional group, a bioorthogonal functional group to a protein is used. This is because a thiol group-introduced antibody to be derivatized with a reagent of the present invention is a protein. The bioorthogonal functional group to a protein is a group that does not react with side chains of 20 types of natural amino acid residues forming proteins, or reacts with a target functional group although having a low reaction rate to the side chain. The 20 types of natural amino acids forming proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Among these 20 types of natural amino acids, glycine, which has no side chain (that is, which has a hydrogen atom as a side chain), and alanine, isoleucine, leucine, phenylalanine, and valine, which each have a hydrocarbon group as a side chain (that is, which each comprise no hetero atom selected from the group consisting of a sulfur atom, a nitrogen atom, and an oxygen atom in a side chain thereof) are inactive to normal reactions. Consequently, the bioorthogonal functional group to a protein is a group that does not react with, in addition to the side chains of these amino acids having side chains inactive to normal reactions, side chains of asparagine, glutamine, methionine, proline, serine, threonine, tryptophan, tyrosine, aspartic acid, glutamic acid, arginine, histidine, and lysin, or reacts with a target functional group although having a low reaction rate.

Examples of such a bioorthogonal functional group include an azide residue, an aldehyde residue, a thiol residue, an alkene residue (in other words, it is only required to have a vinylene (ethenylene) portion, which is a minimum unit having a carbon-carbon double bond. Hereinafter the same), an alkyne residue (in other words, it is only required to have an ethynylene portion, which is a minimum unit having a carbon-carbon triple bond. Hereinafter the same), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (e.g., a carbonyl residue having a fluorine atom, a chlorine atom, a bromine atom, or an iodine atom at an α-position. Hereinafter the same), an isonitrile residue, a sydnone residue, and a selenium residue.

More specifically, the bioorthogonal functional group may correspond to any one chemical structure selected from the group consisting of the following: 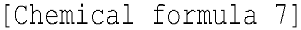 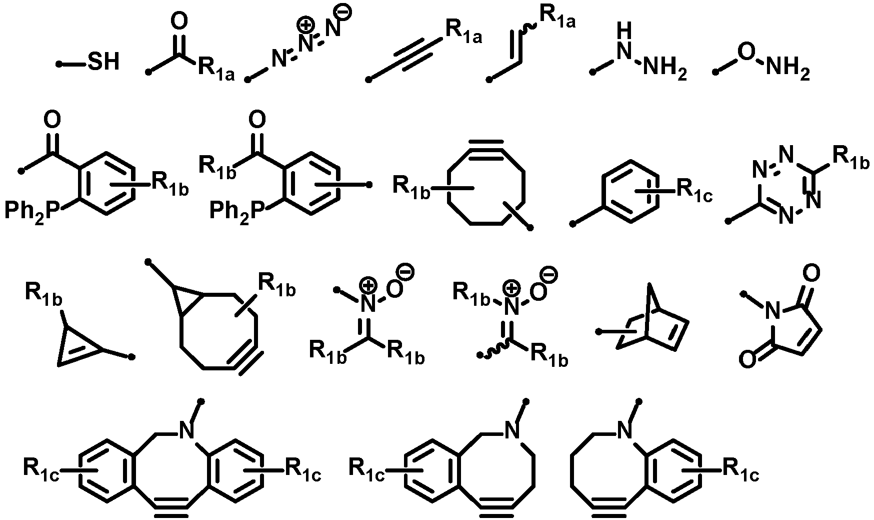 wherein
R₁ₐ, one or a plurality of R_{1b}s, and one or a plurality of R_{1c}s are the same as or different from each other, and each represent any one of the substituents described above or an electron-withdrawing group, and
- is a bond.

Examples of the electron-withdrawing group include a halogen atom, an alkyl substituted with a halogen atom (e.g., trifluoromethyl), a boronic acid residue, mesyl, tosyl, triflate, nitro, cyano, a phenyl group, a keto group (e.g., acyl), and an alkyloxy group, and a halogen atom, a boronic acid residue, mesyl, tosyl, and triflate are preferred.

The bioorthogonal functional group may be protected. The optionally protected bioorthogonal functional group refers to an unprotected bioorthogonal functional group or a protected bioorthogonal functional group. The unprotected bioorthogonal functional group corresponds to the bioorthogonal functional group described above. The protected bioorthogonal functional group is a group that generates a bioorthogonal functional group by cleavage of a protective group. The protective group can be cleaved by a specific treatment under a condition (a mild condition) incapable of causing denaturation or decomposition of proteins (e.g., cleavage of an amide bond). Examples of such a specific treatment include (a) a treatment with one or more substances selected from the group consisting of an acidic substance, a basic substance, a reducing agent, an oxidizing agent, and an enzyme, (b) a treatment with a physical and chemical stimulus selected from the group consisting of light, and (c) leaving a cleavable linker as it is when the cleavable linker comprises a self-degradable cleavable portion. Such a protective group and a cleavage condition therefor are common technical knowledge in the field concerned (e.g., G. Leriche, L. Chisholm, A. Wagner, Bioorganic & Medicinal Chemistry. 20,571 (2012); Feng P. et al., Jounal of American Chemical Society. 132,1500 (2010).; Bessodes M. et al., Journal of Controlled Release, 99,423 (2004).; DeSimone, J.M., Journal of American Chemical Society. 132,17928 (2010); Thompson, D.H., Journal of Controlled Release, 91,187 (2003); and Schoenmarks, R.G., Journal of Controlled Release, 95,291 (2004)).

Examples of the protected bioorthogonal functional group include a disulfide residue, an ester residue, an acetal residue, a ketal residue, an imine residue, and a vicinaldiol residue.

More specifically, the protected bioorthogonal functional group may correspond to any one chemical structure selected from the group consisting of the following: 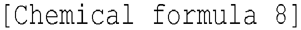 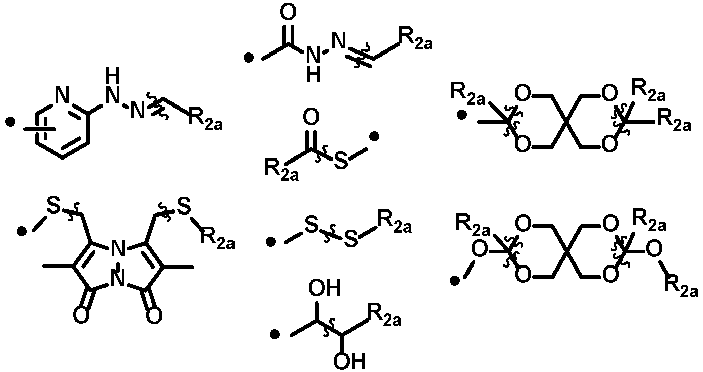
wherein the wavy line orthogonal to the bond indicates a cleavage site,
one or a plurality of R₂ₐs are the same as or different from each other, and each represent a hydrogen atom or a group selected from the group consisting of the substituents described above, and
   - is a bond.

The optionally protected bioorthogonal functional group is preferably an unprotected bioorthogonal functional group.

### (Functional substance)

The functional substance is not limited to a particular substance as long as it is a substance imparting any function to the antibody; examples thereof include drugs, labelling substances, and stabilizers; preferred are drugs and labelling substances. The functional substance may be a single functional substance or a substance in which two or more functional substances are linked with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). The drug may be a prophylactic or therapeutic agent for a disease, or a relief agent for side effects.

More specifically, the drug may be an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specific examples of the drug include auristatin (MMAE, MMAF), maytansine (DM1, DM4), PBD (pyrrolobenzodiazepine), IGN, camptothecin analogs, calicheamicin, duocarmycin, eribulin, anthracycline, dmDNA31, and tubricin.

The labelling substance is a substance that makes detection of a target (e.g., a tissue, a cell, or a substance) possible. Examples of the labelling substance include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl) Duthenium, and luminol), and radioisotopes (e.g., those described above) or substances comprising them.

The stabilizer is a substance that makes stabilization of an antibody possible. Examples of the stabilizer include diols, glycerin, nonionic surfactants, anionic surfactants, natural surfactants, saccharides, and polyols.

The functional substance may also be a peptide, a protein, a nucleic acid, a low molecular weight organic compound, a sugar chain, a lipid, a high molecular polymer, a metal (e.g., gold), or a chelator. Examples of the peptide include a cell membrane permeable peptide, a blood-brain barrier permeable peptide, and a peptide medicament. Examples of the protein include enzymes, cytokines, fragment antibodies, lectins, interferons, serum albumin, and antibodies. Examples of the nucleic acid include DNA, RNA, and artificial nucleic acid. Examples of the nucleic acid also include RNA interference inducible nucleic acids (e.g., siRNA), aptamers, and antisense. Examples of the low molecular weight organic compound include proteolysis targeting chimeras, dyes, and photodegradable compounds.

### (Salt)

In the present invention, examples of the term "salt" include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 2. Conjugate or salt thereof

The present invention provides a conjugate of an antibody and a functional substance or a salt thereof which comprises a structural unit represented by the following formula (I): 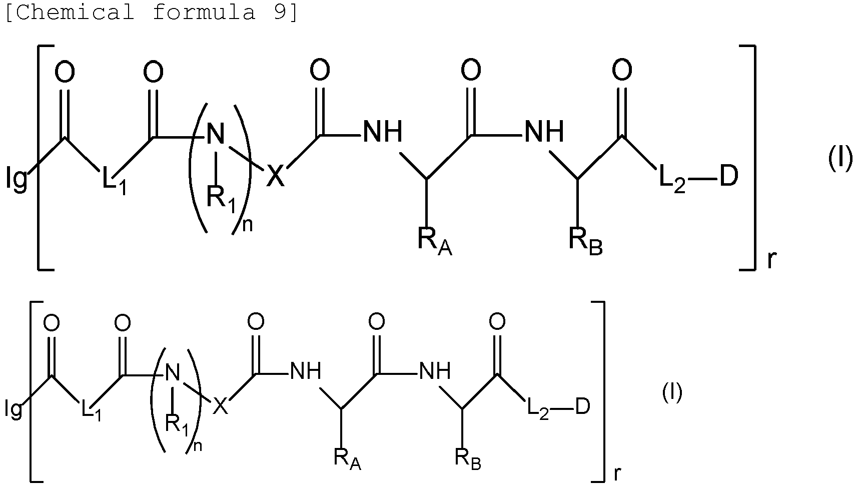 wherein
Ig represents an immunoglobulin unit comprising two heavy chains and two light chains, and regioselectively forms an amide bond with a carbonyl group adjacent to Ig via an amino group in side chains of lysine residues in the two heavy chains,
L₁ and L₂ each represent a divalent group,
R₁ represents a monovalent group optionally comprising a hydrophilic group,
X represents a divalent group optionally having a substituent, wherein the divalent group has 1 to 3 carbon atoms constituting a main chain portion linking two atoms present on both sides of X, and the substituent is a monovalent group optionally comprising a hydrophilic group,
D represents a functional substance,
R_{A} represents a side chain of a valine residue,
R_{B} represents a side chain of a citrulline residue or an alanine residue,
n is 0 or 1, wherein when n is 1, the substituent in X may form a ring optionally comprising a hydrophilic group together with R₁, and
an average ratio r of the amide bonds per two heavy chains is 1.5 to 2.5, and
wherein at least one hydrophilic group is present in the structural unit.

In formula (I) and other formulae presented in relation to the present invention, -(hyphen) indicates that two units (e.g., atoms or groups) present on both sides thereof are covalently bonded to each other.

The antibody includes the immunoglobulin unit. Examples of such an antibody include: an IgG antibody comprising two heavy chains and two light chains and comprising an immunoglobulin unit having a disulfide bond between the heavy chains and between the heavy chains and the light chains; an IgD antibody and an IgE antibody; an IgA antibody comprising four heavy chains and four light chains and comprising an immunoglobulin unit having a disulfide bond between the heavy chains and between the heavy chains and the light chains; and an IgM antibody comprising eight heavy chains and eight light chains and comprising an immunoglobulin unit having a disulfide bond between the heavy chains and between the heavy chains and the light chains, and an IgG antibody (e.g., IgG1, IgG2, IgG3, or IgG4) is preferred. The antibody is preferably a human IgG monoclonal antibody, and more preferably a human IgG full-length monoclonal antibody.

The divalent groups represented by L₁ and L₂ are the same as those described above.

The hydrophilic group and the monovalent group in R₁ are the same as those described above.

The divalent group in X is a divalent group in which the number of carbon atoms constituting a main chain portion linking two atoms present on both sides of X is 1 to 3. Such a main chain portion is constituted by a chain structure, a cyclic structure, or a structure comprising a combination thereof. When the main chain portion has a chain structure comprising no cyclic structure, the number of carbon atoms of the main chain portion can be determined by counting the number of carbon atoms in the chain structure. On the other hand, when the main chain portion has a structure comprising a cyclic structure, the number of predetermined carbon atoms constituting the cyclic structure can be determined by counting the number of carbon atoms in the main chain portion. Specifically, the number of carbon atoms of the main chain portion in the cyclic structure can be determined by counting the number of carbon atoms of the shortest route linking two bonds in the cyclic structure (see, e.g., the following thick routes (a) to (d)). When the main chain has a structure comprising a combination of a chain structure and a cyclic structure, the number of atoms of the main chain can be determined by adding the number of atoms in the chain structure not comprising the cyclic structure to the number of atoms of the shortest route linking two bonds in the cyclic structure. 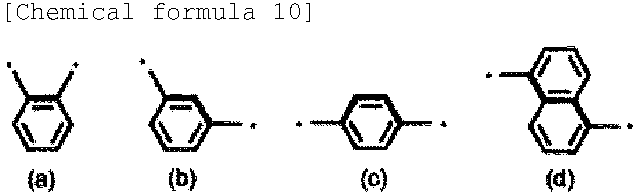
- is a bond.

In the case of (a), the shortest route is the thick route, and thus the number of atoms in the divalent cyclic structure counted as the number of carbon atoms of the main chain portion is two.

In the case of (b), the shortest route is the thick route, and thus the number of atoms in the divalent cyclic structure counted as the number of carbon atoms of the main chain portion is three.

In the case of (c), both routes are the shortest routes (equidistant), and thus the number of carbon atoms in the divalent cyclic structure counted as the number of carbon atoms in the main chain portion is four (therefore, in the present invention, such a structure is excluded from the divalent group in X).

In the case of (d), a route of a condensation site is the shortest routes, and thus the number of carbon atoms in the divalent cyclic structure counted as the number of atoms in the main chain is four (therefore, in the present invention, such a structure is excluded from the divalent group in X).

The divalent group in X can be selected from the divalent groups described above such that a main chain portion thereof satisfies the above conditions.

The divalent group in X may be preferably:
(1) a C₁₋₃ divalent linear hydrocarbon group;
(2) a divalent cyclic hydrocarbon group; or
(3) a divalent group in which a divalent cyclic hydrocarbon group and (one or two) C₁₋₂ divalent linear hydrocarbon groups are linked to each other.

The C₁₋₃ divalent linear hydrocarbon group is a C₁₋₃ linear alkylene (e.g., methylene, ethylene, or n-propylene), a C₂₋₃ linear alkenylene (e.g., ethenylene or propenylene), or a C₂₋₃ linear alkynylene (e.g., ethynylene or n-propynylene). The C₁₋₄ divalent linear hydrocarbon group is preferably a C₁₋₃ linear alkylene.

The divalent cyclic hydrocarbon group is an arylene or a divalent nonaromatic cyclic hydrocarbon group. By appropriately setting two bonds in such a divalent cyclic hydrocarbon group, the number of atoms constituting such a main chain as described above can be set to 3 to 5.

The arylene is preferably a C₆₋₁₀ arylene, and more preferably a C₆ arylene. Examples of the arylene include phenylene and naphthylene.

The divalent nonaromatic cyclic hydrocarbon group is preferably a C₃₋₁₂ monocyclic or polycyclic divalent nonaromatic cyclic hydrocarbon group, more preferably a C₄₋₁₀ monocyclic or polycyclic divalent nonaromatic cyclic hydrocarbon group, and particularly preferably a C₅₋₈ monocyclic divalent nonaromatic cyclic hydrocarbon group. Examples of the divalent nonaromatic cyclic hydrocarbon group include cyclopropylene, cyclobutylene, cyclopentylene, cyclohexylene, cycloheptylene, and cyclooctylene.

The divalent cyclic hydrocarbon group is preferably an arylene.

The C₁₋₂ divalent linear hydrocarbon group is a C₁₋₂ linear alkylene (e.g., methylene or ethylene), a C₂ linear alkenylene (ethenylene), or a C₂ linear alkynylene (ethynylene). The C₁₋₄ divalent linear hydrocarbon group is preferably a C₁₋₄ linear alkylene.

Among the above (1) to (3), the divalent group in X is preferably (1) or (2), and more preferably (1).

Regarding the substituent which the divalent group in X may have, the hydrophilic group and the monovalent group are the same as those described above.

The functional substance represented by D is the same as that described above.

R_{A} represents a side chain of a valine residue (that is, -CH(CH₃)₂).

R_{B} represents a side chain of a citrulline residue (that is, -CH₂CH₂CH₂NHCONH₂) or a side chain of an alanine residue (that is, -CH₃).

n is 0 or 1. When n is 0, there is no unit of N-R₁, and X is directly bonded to two carbonyl groups. When n is 1, the substituent in X may form a ring optionally comprising a hydrophilic group together with R₁. Such a ring comprises a nitrogen atom linked to R₁ as a ring-constituting atom. Such a ring is preferably a 5-membered ring or a 6-membered ring. Such a ring may also be a nonaromatic ring or an aromatic ring, and is preferably a nonaromatic ring (e.g., pyrrolidine or piperazine).

r represents an average ratio of the amide bonds per two heavy chains and is 1.5 to 2.5. Such an average ratio may be preferably 1.6 or more, more preferably 1.7 or more, even more preferably 1.8 or more, and particularly preferably 1.9 or more. Such an average ratio may also be preferably 2.4 or less, more preferably 2.3 or less, even more preferably 2.2 or less, and particularly preferably 2.1 or less. More specifically, such an average ratio may be preferably 1.6 to 2.4, more preferably 1.7 to 2.3, even more preferably 1.8 to 2.2, and particularly preferably 1.9 to 2.1.

In the structural unit represented by formula (I), at least one (e.g., 1, 2, or 3) hydrophilic group is present. Therefore, at least one of the "monovalent group optionally comprising a hydrophilic group" represented by R₁, the "monovalent group optionally comprising a hydrophilic group" for the "substituent" in the "divalent group optionally having a substituent" represented by X, and the "ring optionally comprising a hydrophilic group" when the substituent in X together with R₁ forms a ring comprises a hydrophilic group.

In a specific embodiment, L₁ may represent a divalent group represented by the following formula (i).

-L₄-Y-L₃- (i)

wherein
L₃ and L₄ each independently comprise a divalent group selected from the group consisting of -(C(R)₂)ₘ-, -(O-C(R)₂-C(R)₂)ₘ-, and -(C(R)₂-C(R)₂-O)ₘ-, and a combination thereof,
Rs each independently represent a hydrogen atom, a C₁₋₆ alkyl, a C₂₋₆ alkenyl, or a C₂₋₆ alkynyl,
m is an integer of 0 to 20, and
Y represents a divalent group generated by a reaction of two bioorthogonal functional groups capable of reacting with each other.

The C₁₋₆ alkyl, the C₂₋₆ alkenyl, and the C₂₋₆ alkynyl represented by R are the same as those described above.

m is an integer of 0 to 20. m may be preferably an integer of 1 or more, more preferably an integer of 2 or more, an integer of 3 or more, an integer of 4 or more, or an integer of 5 or more. m may also be preferably an integer of 15 or less, more preferably an integer of 12 or less, an integer of 10 or less, or an integer of 9 or less. ms in the divalent groups represented by L₃ and L₄ can be set to different integers.

In a specific embodiment, L₃ and L₄ may each independently comprise a divalent group represented by - (C(R)₂)ₘ-.

Y represents a divalent group generated by a reaction of two bioorthogonal functional groups capable of reacting with each other. Since a combination of two bioorthogonal functional groups capable of reacting with each other is well known, a person skilled in the art can appropriately select such a combination and appropriately set a divalent group generated by a reaction of the two bioorthogonal functional groups capable of reacting with each other. Examples of the combination of bioorthogonal functional groups capable of reacting with each other include a combination of a thiol residue and a maleimide residue, a combination of a furan residue and a maleimide residue, a combination of a thiol residue and a halocarbonyl residue (a halogen is replaced with a thiol by a substitution reaction), a combination of an alkyne residue (preferably, a ring group having a triple bond between carbon atoms, which may have such a substituent as described above) and an azide residue, a combination of a tetrazine residue and an alkene residue, a combination of a tetrazine residue and an alkyne residue, and a combination of a thiol residue and another thiol residue (disulfide bond). Therefore, Y may be a group generated by a reaction of a thiol residue and a maleimide residue, a group generated by a reaction of a furan residue and a maleimide residue, a group generated by a reaction of a thiol residue and a halocarbonyl residue, a group generated by a reaction of an alkyne residue and an azide residue, a group generated by a reaction of a tetrazine residue and an alkene residue, or a disulfide group generated by a combination of a thiol residue and another thiol residue.

In a specific embodiment, Y may be a divalent group represented by any one of the following structural formulae: 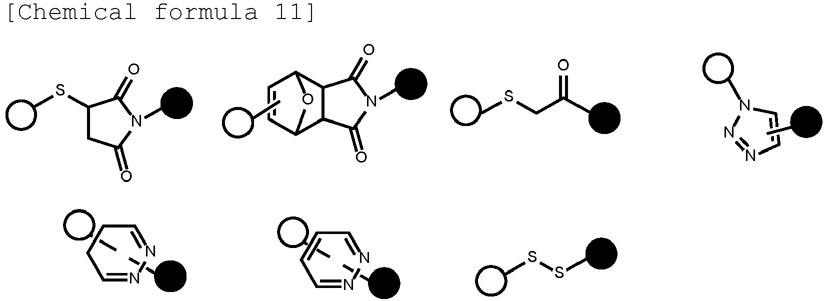
wherein the white circle and the black circle each represent a bond,
when the bond of the white circle is bonded to L₃, the bond of the black circle is bonded to L₄, and
when the bond of the white circle is bonded to L₄, the bond of the black circle is bonded to L₃.

In a preferred embodiment, the structural unit represented by the above formula (I) may be a structural unit represented by the following formula (I-1), (I-2), (I-3), or (1-4). Such a structural unit can be excellent in performance as a conjugate among the structural units represented by the above formula (I) (see, Examples). 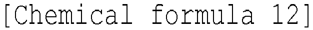 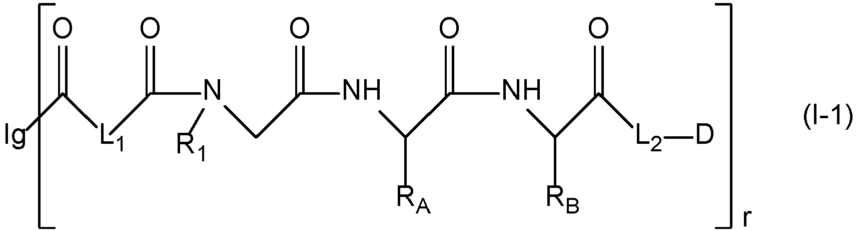 wherein
Ig, L₁, L₂, D, R_{A}, R_{B}, and r are the same as those in formula (I), and
R₁ represents a monovalent group comprising a hydrophilic group. 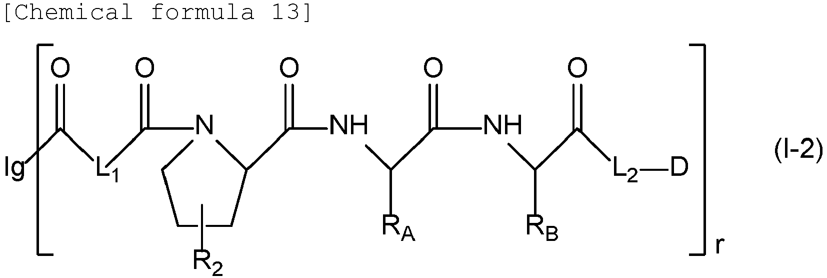
wherein
Ig, L₁, L₂, D, R_{A}, R_{B}, and r are the same as those in formula (I), and
R₂ represents a monovalent group comprising a hydrophilic group. 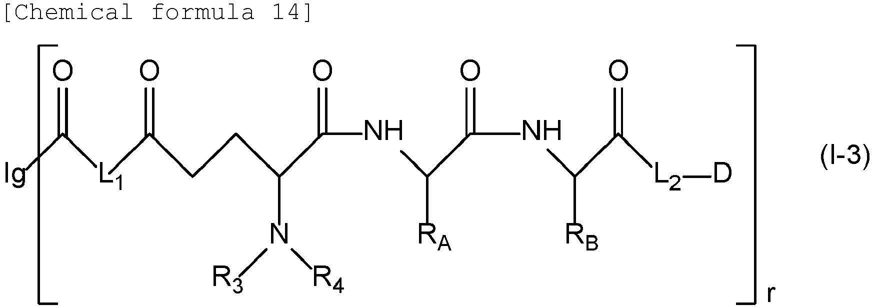
wherein
Ig, L₁, L₂, D, R_{A}, R_{B}, and r are the same as those in formula (I),
R₃ represents a hydrogen atom, a C₁₋₆ alkyl, or a monovalent group comprising a hydrophilic group, and
R₄ represents a monovalent group comprising a hydrophilic group. 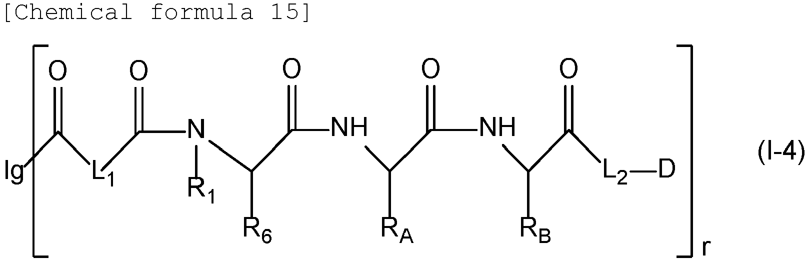
wherein
Ig, L₁, L₂, D, R_{A}, R_{B}, and r are the same as those in formula (I),
R₁ represents a hydrogen atom or a C₁₋₆ alkyl, and
R₆ represents a monovalent group comprising a hydrophilic group.

In the above formula (I-1), (I-2), (I-3), or (I-4), the monovalent group comprising a hydrophilic group and the C₁₋₆ alkyl are the same as those described above.

In a preferred embodiment, the structural unit represented by the above formula (I) may be a structural unit represented by the above formula (I-1), (I-2), or (I-3). Such a structural unit can be excellent in performance as a conjugate among the structural units represented by the above formula (I) (see, EXAMPLES).

In one embodiment, in the above formula (I-3), R₃ may represent a hydrogen atom or a C₁₋₆ alkyl, and R₄ may represent a monovalent group comprising a hydrophilic group.

In another embodiment, in the above formula (I-3), R₃ and R₄ each may independently represent a monovalent group comprising a hydrophilic group.

In a more preferred embodiment, the structural unit represented by the above formula (I-1), (I-2), (I-3), or (I-4) may be a structural unit represented by the following formula (I-1a'), (I-1b'), (I-1c'), (I-2'), (I-3a'), (I-3b'), (I-4a'), (I-4b'), or (I-4c'). Such a structural unit can be excellent in performance as a conjugate among the structural units represented by the above formula (I) (see, EXAMPLES). 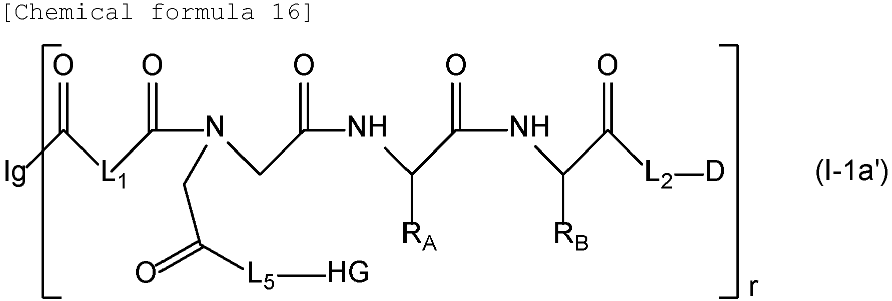 wherein
Ig, L₁, L₂, D, R_{A}, R_{B}, and r are the same as those in formula (I),
L₅ represents a bond or a divalent group, and
HG represents a hydrophilic group. 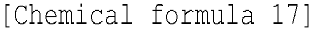 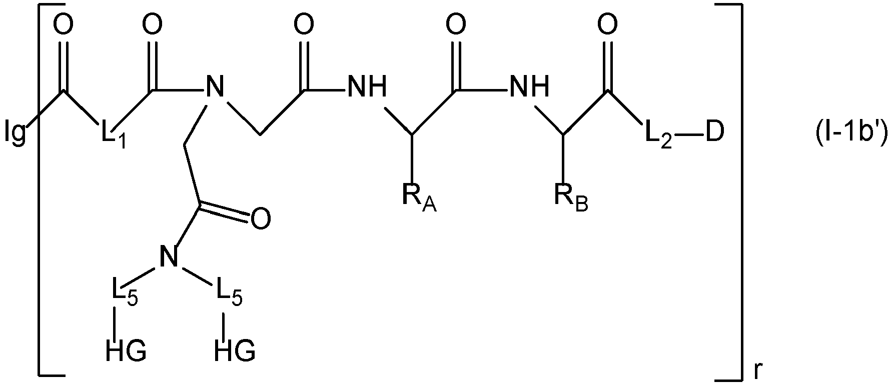
wherein
Ig, L₁, L₂, D, R_{A}, R_{B}, and r are the same as those in formula (I),
two L₅ each independently represent a bond or a divalent group, and
two HG each independently represent a hydrophilic group. 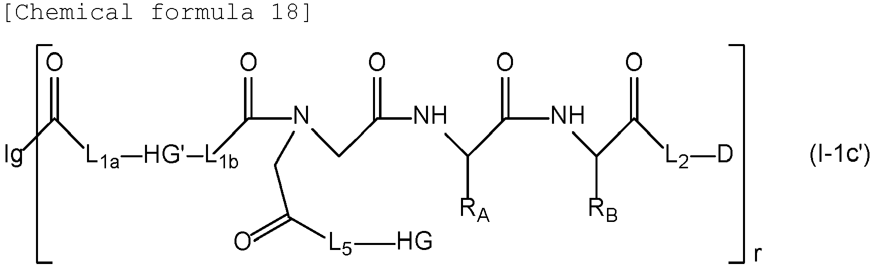
wherein
Ig, L₂, D, R_{A}, R_{B}, and r are the same as those in formula (I),
L₅ represents a bond or a divalent group,
HG represents a hydrophilic group,
L₁ₐ and L_{1b} each independently represent a bond or a divalent group, and
HG' represents a divalent hydrophilic group. 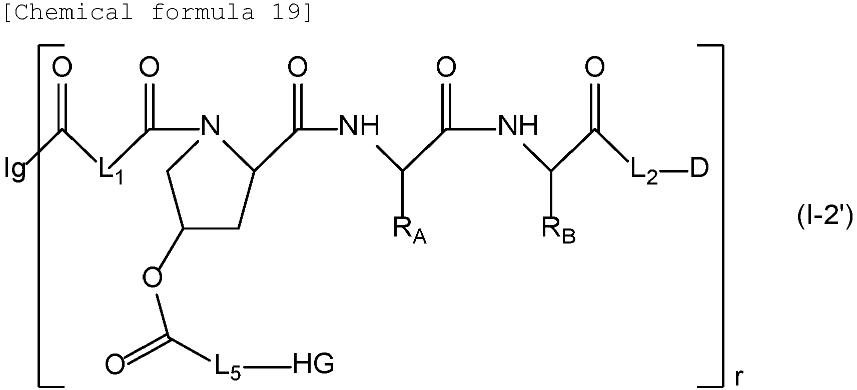
wherein
Ig, L₁, L₂, D, R_{A}, R_{B}, and r are the same as those in formula (I),
L₅ represents a bond or a divalent group, and
HG represents a hydrophilic group. 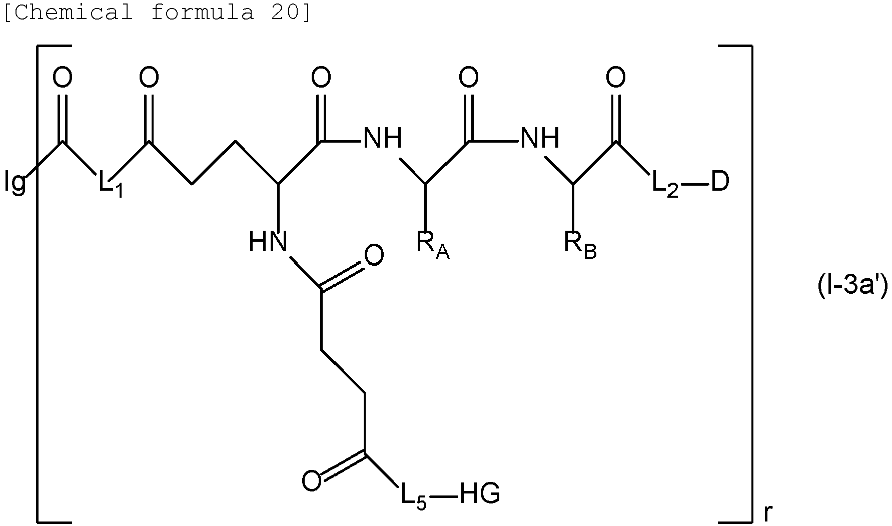
wherein
Ig, L₁, L₂, D, R_{A}, R_{B}, and r are the same as those in formula (I),
L₅ represents a bond or a divalent group, and
HG represents a hydrophilic group. 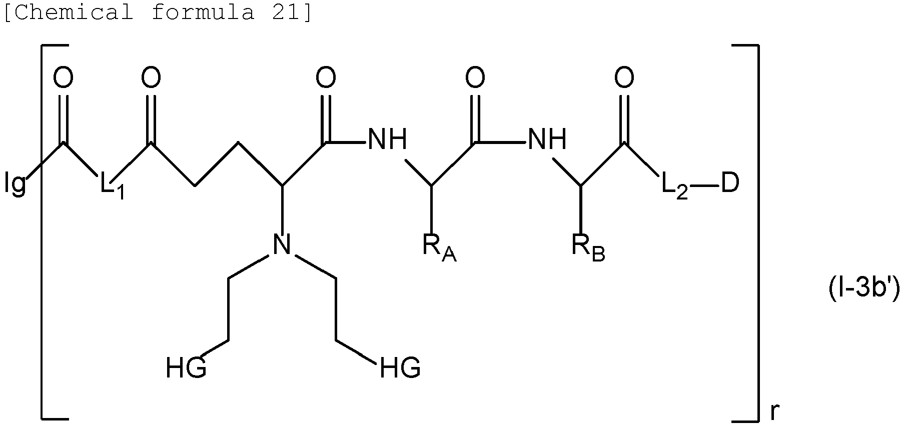
wherein
Ig, L₁, L₂, D, R_{A}, R_{B}, and r are the same as those in formula (I), and
HG represents a hydrophilic group. 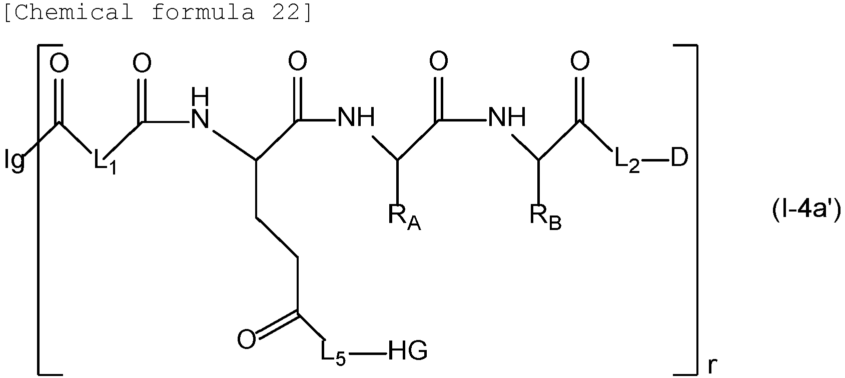
wherein
Ig, L₁, L₂, D, R_{A}, R_{B}, and r are the same as those in formula (I),
L₅ represents a bond or a divalent group, and
HG represents a hydrophilic group. 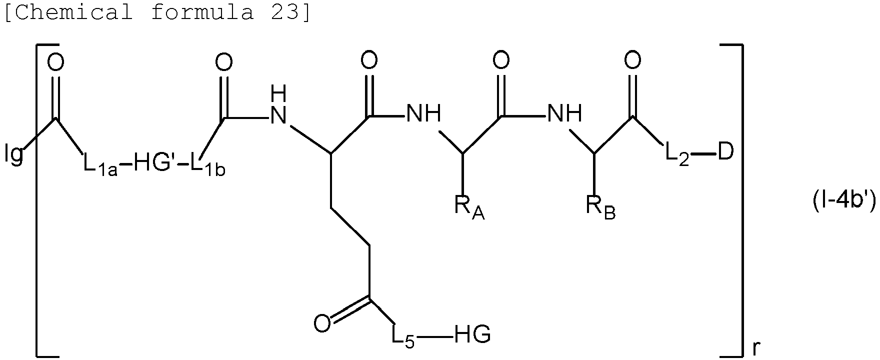
wherein
Ig, L₂, D, R_{A}, R_{B}, and r are the same as those in formula (I),
L₅ represents a bond or a divalent group,
HG represents a hydrophilic group,
L₁ₐ and L_{1b} each independently represent a bond or a divalent group,
HG' represents a divalent hydrophilic group. 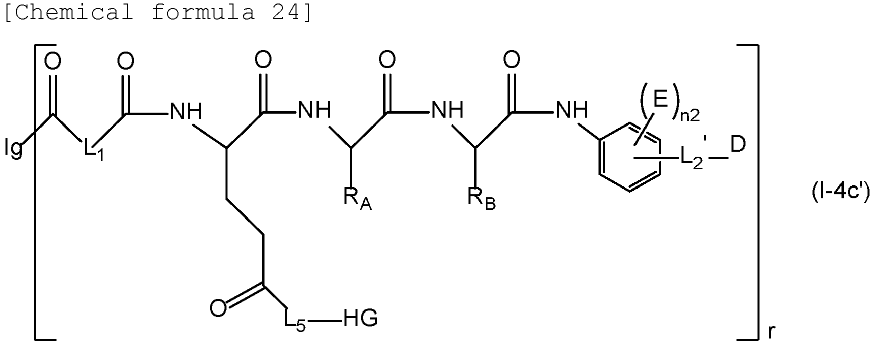
wherein
Ig, L₁, D, R_{A}, R_{B}, and r are the same as those in formula (I),
L₅ represents a bond or a divalent group,
HG represents a hydrophilic group,
L₂' represents a bond or a divalent group,
E represents an electron-withdrawing group, and
n2 is an integer of 1 to 4.

In formulae (I-1a') to (I-4c'), definitions, examples, and preferred examples of the divalent group and the hydrophilic group are the same as those described above. Examples of the divalent hydrophilic group may include a polyethylene glycol group, a polysarcosine group, and a sugar portion, as the hydrophilic group described above, and preferably a polyethylene glycol group and a polysarcosine group, more preferably a polyethylene glycol group. The electron-withdrawing group is same as those described above. The electron-withdrawing group could bind to phenyl ring at ortho-, meta-, or para-position, preferably ortho- or para-position, more preferably ortho- or para-position, relative to an adjacent amino group. n2 may be preferably 1 or 2. Definition, examples and preferable examples of the divalent hydrophilic group, the electron-withdrawing group and its binding position, and n2 also apply to the other figures.

In a preferred embodiment, the structural unit represented by "-L₅-HG" may be selected from the group consisting of the following:
(a)

   -OH
(b)

   -NH(CH₂CH₂O)₈CH₃
(c)

   -NHCH₂CH₂COOH

In a specific embodiment, the structural unit represented by formula (I-1a') may be the following structural unit: 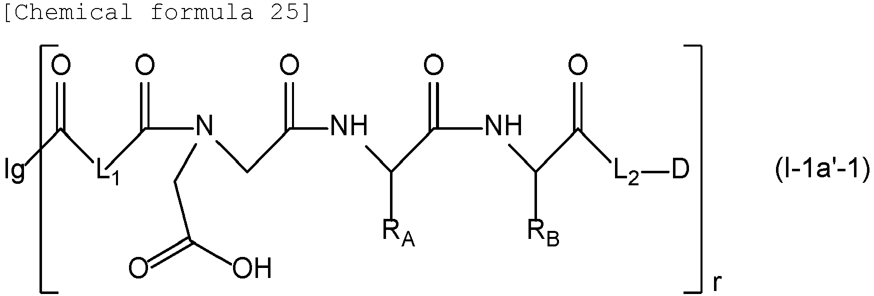 wherein
Ig, L₁, L₂, D, R_{A}, R_{B}, and r are the same as those in formula (I). 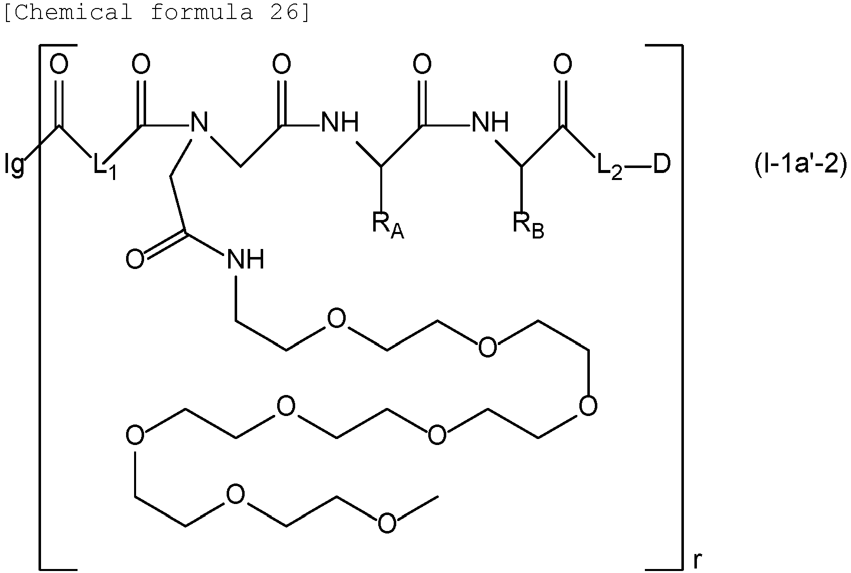 wherein
Ig, L₁, L₂, D, R_{A}, R_{B}, and r are the same as those in formula (I).

In another specific embodiment, the structural unit represented by formula (I-1b') may be the following structural unit: 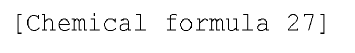 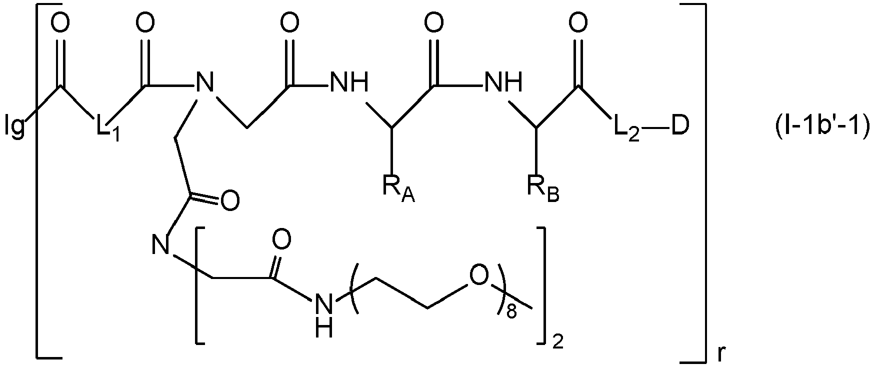 wherein
Ig, L₁, L₂, D, R_{A}, R_{B}, and r are the same as those in formula (I). 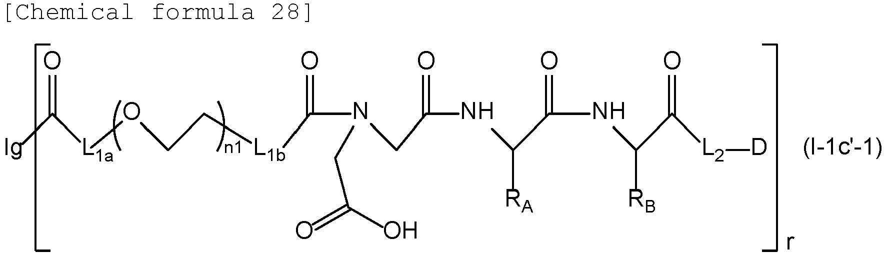 wherein
Ig, L₂, D, R_{A}, R_{B}, and r are the same as those in formula (I)
L₁ₐ and L_{1b} each independently represent a bond or a divalent group, and
n1 is an integer of 3 to 20.

In formula (I-1c'-1), definitions, examples, and preferred examples of the divalent group are the same as those described above. n1 may be an integer of 3 or more, preferably an integer of 4 or more, more preferably an integer of 5 or more, even more preferably an integer of 6 or more. n1 may also be an integer of 20 or less, preferably an integer of 15 or less, more preferably an integer of 12 or less, or even more preferably an integer of 10 or less. More specifically, n1 may be an integer of 3 to 20, preferably an integer of 4 to 15, more preferably an integer of 5 to 12, and even more preferably an integer of 6 to 10. Definition, examples and preferable examples of n1 also apply to the other figures.

In a still another specific embodiment, the structural unit represented by formula (I-2') may be the following structural unit: 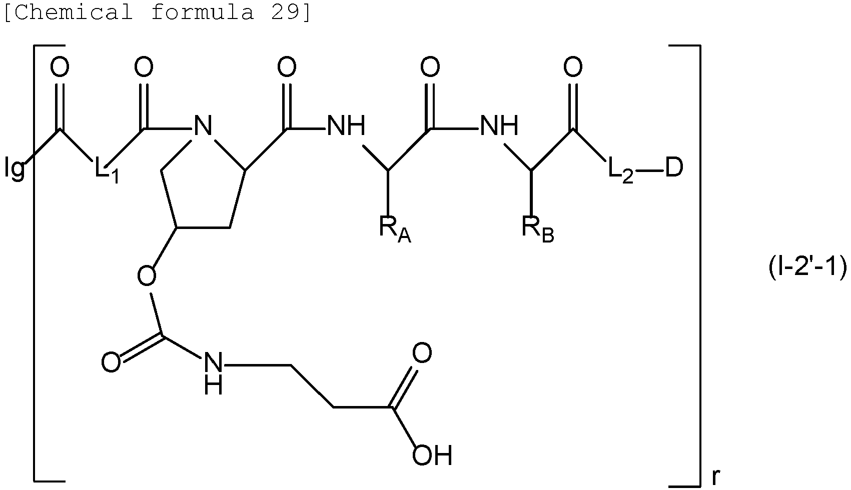 wherein
Ig, L₁, L₂, D, R_{A}, R_{B}, and r are the same as those in formula (I).

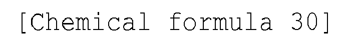

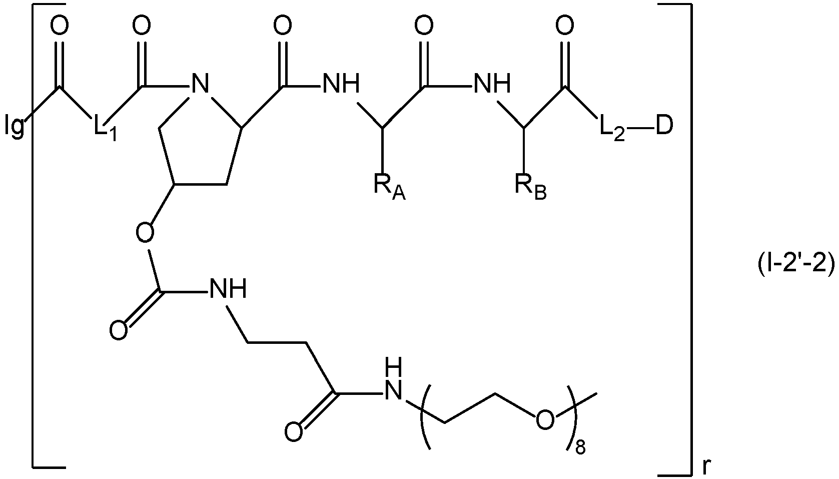

wherein
Ig, L₁, L₂, D, R_{A}, R_{B}, and r are the same as those in formula (I).

In a still another specific embodiment, the structural unit represented by formula (I-3a') may be the following structural unit: 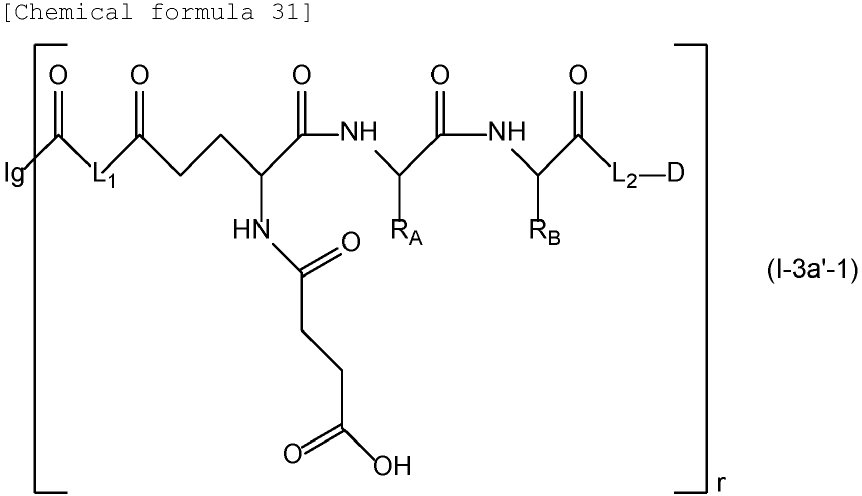 wherein
Ig, L₁, L₂, D, R_{A}, R_{B}, and r are the same as those in formula (I). 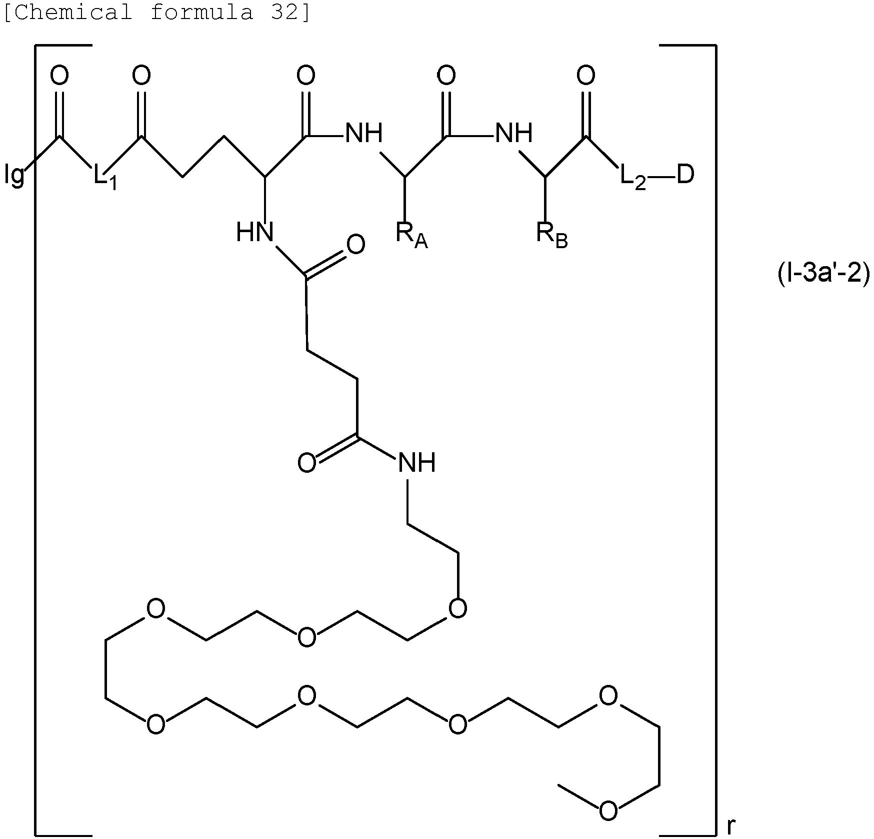 wherein
Ig, L₁, L₂, D, R_{A}, R_{B}, and r are the same as those in formula (I).

In a still another specific embodiment, the structural unit represented by formula (I-3b') may be the following structural unit: 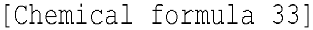 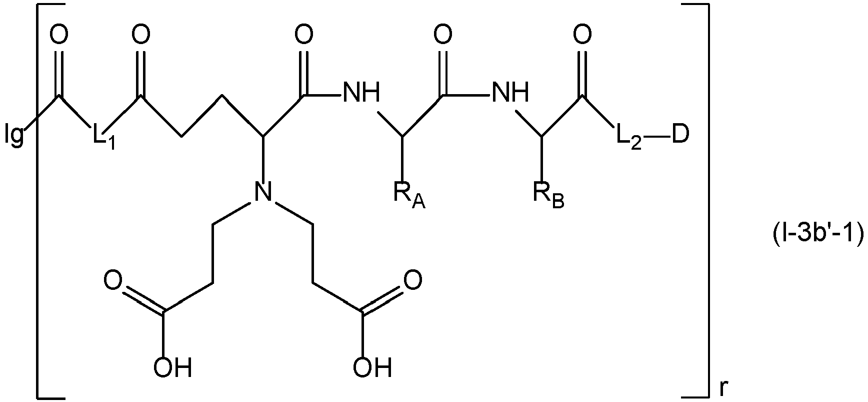 wherein
Ig, L₁, L₂, D, R_{A}, R_{B}, and r are the same as those in formula (I).

In a still another specific embodiment, the structural unit represented by formula (I-4a') may be the following structural unit: 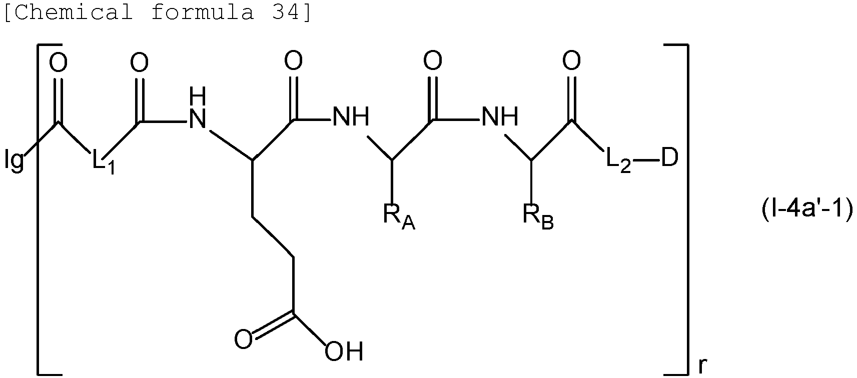 wherein
Ig, L₁, L₂, D, R_{A}, R_{B}, and r are the same as those in formula (I).

In a still another specific embodiment, the structural unit represented by formula (I-4b') may be the following structural unit: 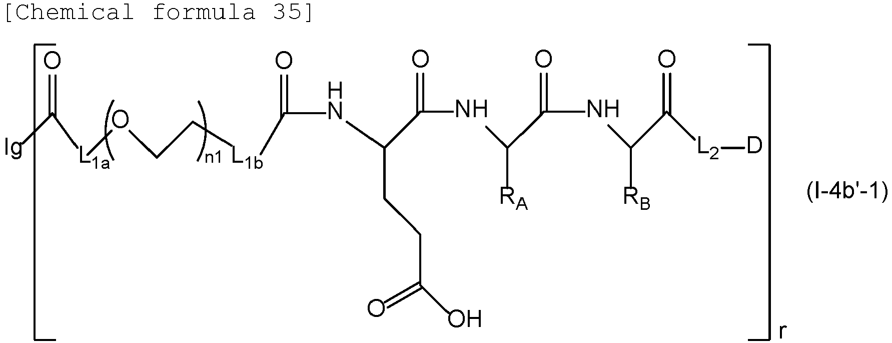 wherein
Ig, L₂, D, R_{A}, R_{B}, and r are the same as those in formula (I),
L₁ₐ and L_{1b} each independently represent a bond or a divalent group, and
n1 is an integer of 3 to 20..

In a still another specific embodiment, the structural unit represented by formula (I-4c') may be the following structural unit: 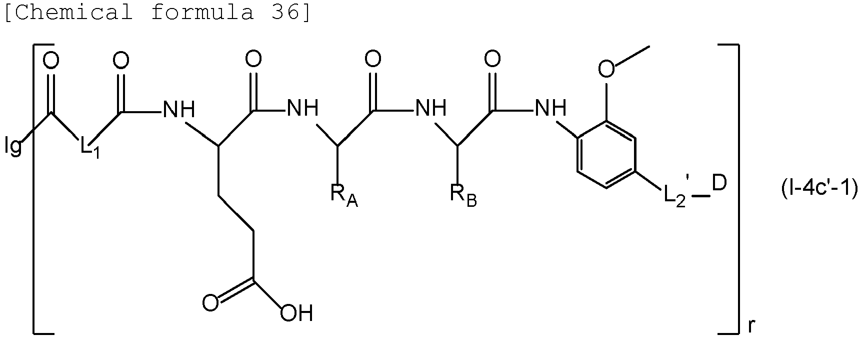 wherein
Ig, L₁, D, R_{A}, R_{B}, and r are the same as those in formula (I), and
L₂ represent a bond or a divalent group; or 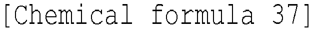 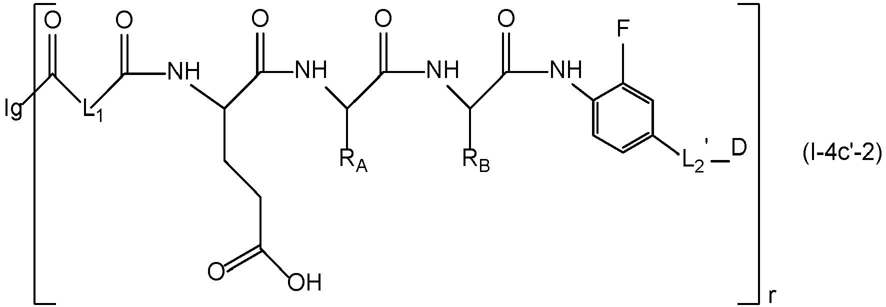
wherein
   Ig, L₁, D, R_{A}, R_{B}, and r are the same as those in formula (I), and
   L₂ represent a bond or a divalent group.

In a preferred embodiment, in the above formula, L₁ may be a divalent group represented by any one of the following structural formulae: 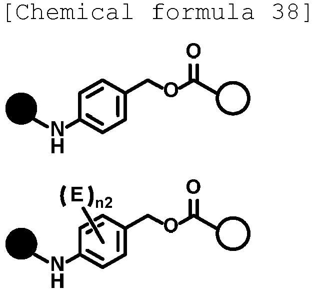
wherein a black circle and a white circle each represent a bond,
the bond of the black circle is bonded to a carbonyl group adjacent to L₂,
the bond of the white circle is bonded to D,
E represents an electron-withdrawing group, and
n2 is an integer of 1 to 4.

Definition, examples, and preferred examples of the electron-withdrawing group and its binding site, and n2 are the same as those described above.

In a more preferred embodiment, the structural unit represented by the above formula (I) may be a structural unit represented by the above formula (I-1a'), (I-1b'), (I-2'), (I-3a'), (I-3b'), (I-4b') or (I-4c') or a formula corresponding to a subordinate concept of these formulae. Such a structural unit can be excellent in performance as a conjugate among the structural units represented by the above formula (I) (see, EXAMPLES).

In a specific embodiment, the conjugate of the present invention or a salt thereof has a desired property of being less likely to aggregate and thus can be identified by an aggregation ratio. More specifically, the aggregation ratio of the conjugate of the present invention or a salt thereof may be 5% or less. This is because the present invention makes it easy to avoid aggregation of an antibody. The aggregation ratio is preferably 4.8% or less, more preferably 4.6% or less, even more preferably 4.4% or less, particularly preferably 4.2% or less, 4.0% or less, 3.8% or less, 3.6% or less, 3.4% or less, 3.2% or less, 3.0% or less, 2.8% or less, 2.6% or less, 2.4% or less, 2.2% or less, or 2.0% or less. The aggregation ratio of an antibody can be measured by size exclusion chromatography (SEC)-HPLC (see, EXAMPLES and Chemistry Select, 2020, 5, 8435-8439).

The conjugate of the present invention or a salt thereof can be produced by causing a compound represented by the following formula (II): 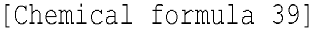 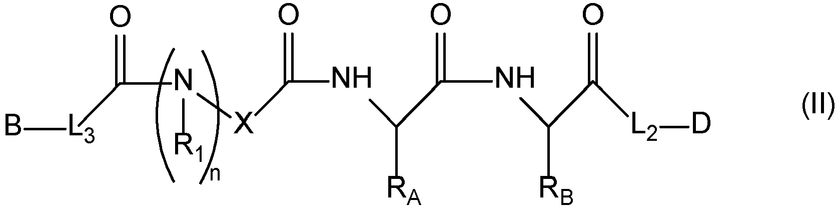 wherein
L₂ and L₃ each represent a divalent group,
R₁ represents a monovalent group optionally comprising a hydrophilic group,
X represents a divalent group optionally having a substituent, wherein the divalent group has 1 to 3 carbon atoms constituting a main chain portion linking two atoms present on both sides of X, and the substituent is a monovalent group optionally comprising a hydrophilic group,
B represents a bioorthogonal functional group,
D represents a functional substance,
R_{A} represents a side chain of a valine residue,
R_{B} represents a side chain of a citrulline residue or an alanine residue,
n is 0 or 1, wherein when n is 1, the substituent in X may form a ring optionally comprising a hydrophilic group together with R₁,
with a raw material antibody comprising an immunoglobulin unit comprising two heavy chains and two light chains (wherein the two heavy chains each comprise a lysine residue regioselectively modified with a bioorthogonal functional group). Definitions, examples, and preferred examples of L₂, L₃, R₁, X, D, R_{A}, R_{B}, and n in the above formula (II) are the same as those described above for the above formula (I).

A definition, examples, and preferred examples of the bioorthogonal functional group represented by B are the same as those described above.

In a specific embodiment, the bioorthogonal functional group represented by B may be a maleimide residue, a thiol residue, a furan residue, a halocarbonyl residue, an alkene residue, an alkyne residue, an azide residue, or a tetrazine residue. The bioorthogonal functional group represented by B can be selected so as to be capable of reacting with a bioorthogonal functional group (e.g., bioorthogonal functional group represented by B') in a raw material antibody described later.

In a preferred embodiment, the compound represented by the above formula (II) or a salt thereof may be a compound represented by the following formula (II-1), (II-2), (II-3), or (II-4), or a salt thereof. Such a compound or a salt thereof is useful as a synthetic intermediate in production of a conjugate that can be excellent in performance among the compounds represented by the above formula (II) and salts thereof (see, EXAMPLES). 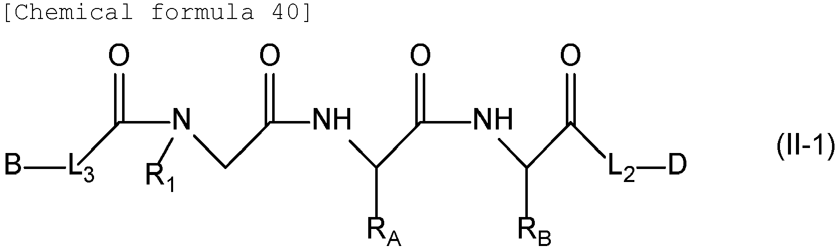 wherein
L₂ and L₃ each represent a divalent group,
R₁ represents a monovalent group optionally comprising a hydrophilic group,
D represents a functional substance,
R_{A} represents a side chain of a valine residue,
R_{B} represents a side chain of a citrulline residue or an alanine residue, and
B represents a bioorthogonal functional group. 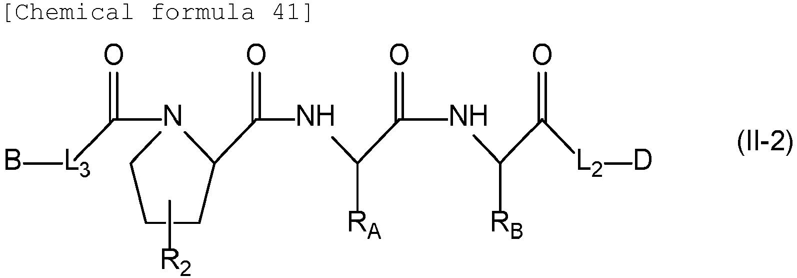
wherein
L₂, L₃, D, R_{A}, R_{B}, and B are the same as those in formula (II-1), and
R₂ represents a monovalent group optionally comprising a hydrophilic group. 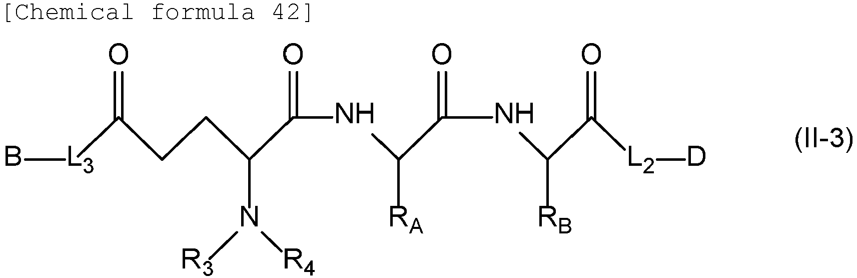
wherein
L₂, L₃, D, R_{A}, R_{B}, and B are the same as those in formula (II-1),
R₃ represents a hydrogen atom or a C₁-₆ alkyl, and
R₄ represents a monovalent group comprising a hydrophilic group. 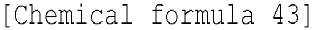 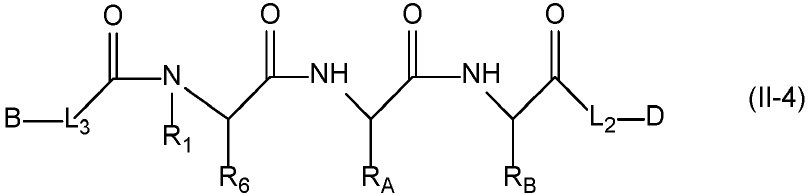
wherein
L₂, L₃, D, R_{A}, R_{B}, and B are the same as those in formula (II-1),
R₁ represents a hydrogen atom or a C₁-₆ alkyl, and
R₆ represents a monovalent group comprising a hydrophilic group.

In the above formula (II-1), (II-2), (II-3), or (II-4), the monovalent group comprising a hydrophilic group and the C₁₋₆ alkyl are the same as those described above.

In one embodiment, in the above formula (II-3), R₃ may represent a hydrogen atom or a C₁-₆ alkyl, and R₄ may represent a monovalent group comprising a hydrophilic group.

In another embodiment, in the above formula (I-3), R₃ and R₄ each may independently represent a monovalent group comprising a hydrophilic group.

In a preferred embodiment, the compound represented by the above formula (II) or a salt thereof may be a compound represented by the above formula (II-1), (II-2), or (II-3), or a salt thereof. Such a compound or a salt thereof is useful as a synthetic intermediate in production of a conjugate that can be excellent in performance among the compounds represented by the above formula (II) and salts thereof (see, EXAMPLES).

In a more preferred embodiment, the compound represented by the above formula (II-1), (II-2), (II-3), or (II-4), or a salt thereof may be a compound represented by the following formula (II-1a'), (II-1b'), (II-1c'), (II-2'), (II-3a'), (II-3b'), (II-4a'), (II-4b')or (II-4c'), or a salt thereof. Such a compound or a salt thereof is useful as a synthetic intermediate in production of a conjugate that can be excellent in performance among the compounds represented by the above formula (II) and salts thereof (see, EXAMPLES). 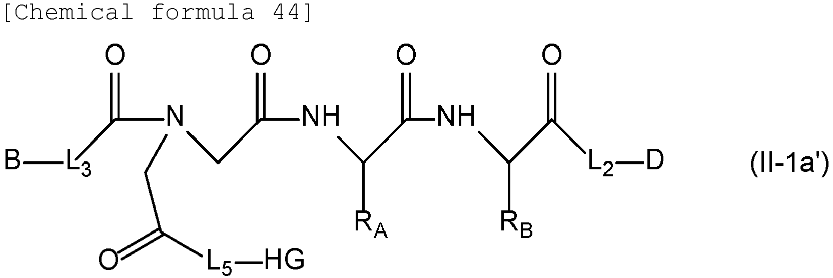 wherein
L₂, L₃, D, R_{A}, R_{B}, and B are the same as those in formula (II-1),
L₅ represents a bond or a divalent group, and
HG represents a hydrophilic group. 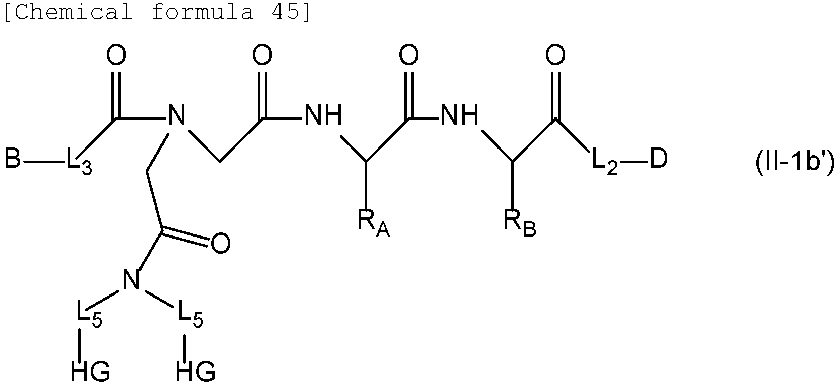
wherein
L₂, L₃, D, R_{A}, R_{B}, and B are the same as those in formula (II-1),
two L₅ each independently represent a bond or a divalent group, and
two HG each independently represent a hydrophilic group. 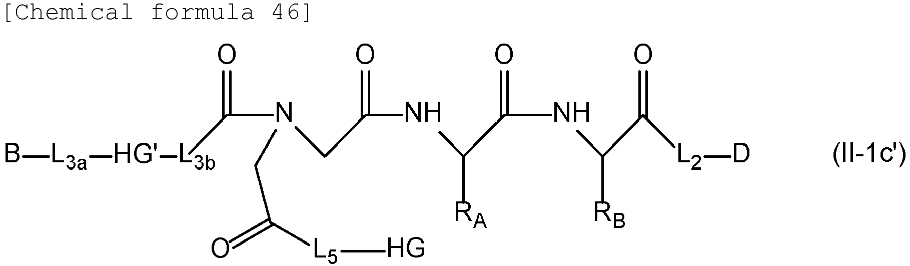
wherein
L₂, D, R_{A}, R_{B}, and B are the same as those in formula (II-1),
L₅ represents a bond or a divalent group,
HG represents a hydrophilic group,
L₃ₐ and L_{3b} each independently represent a bond or a divalent group, and
HG' each independently represent a divalent hydrophilic group. 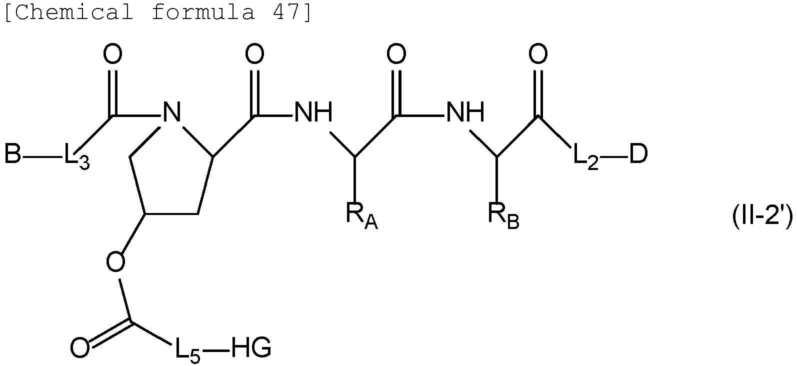
wherein
L₂, L₃, D, R_{A}, R_{B}, and B are the same as those in formula (II-1),
L₅ represents a bond or a divalent group, and
HG represents a hydrophilic group. 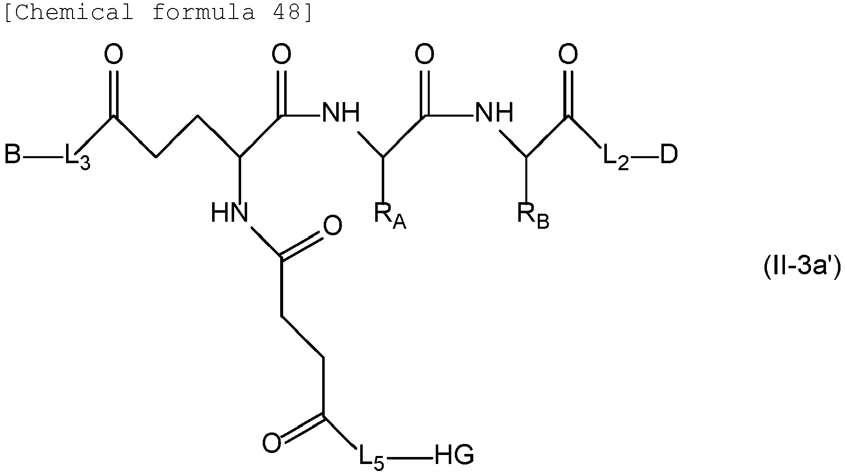
wherein
L₂, L₃, D, R_{A}, R_{B}, and B are the same as those in formula (II-1),
L₅ represents a bond or a divalent group, and
HG represents a hydrophilic group. 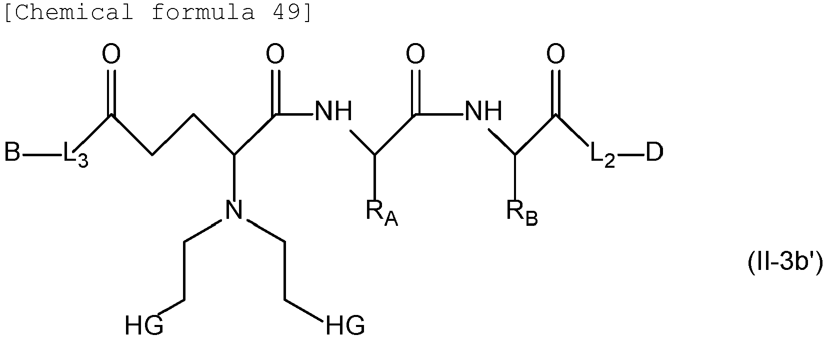
wherein
L₂, L₃, D, R_{A}, R_{B}, and B are the same as those in formula (II-1), and
HG represents a hydrophilic group. 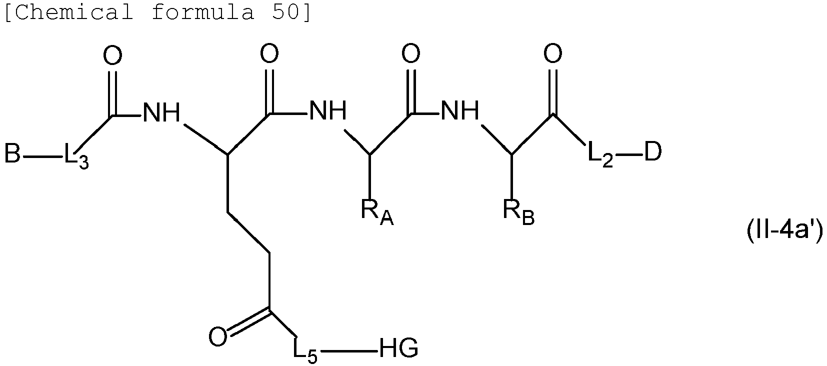
wherein
L₂, L₃, D, R_{A}, R_{B}, and B are the same as those in formula (II-1),
L₅ represents a bond or a divalent group, and
HG represents a hydrophilic group. 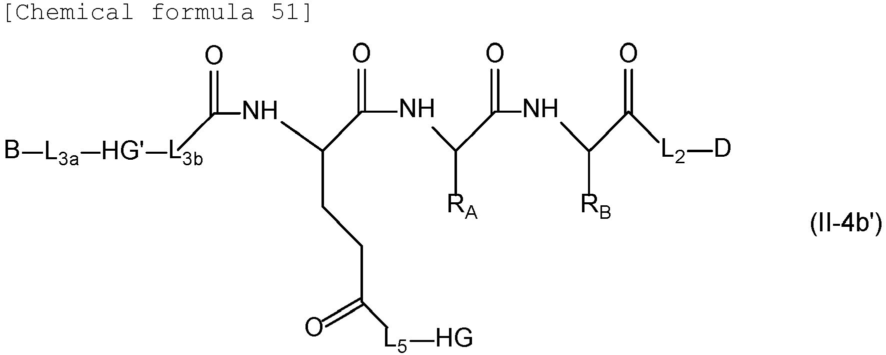
wherein
L₂, D, R_{A}, R_{B}, and B are the same as those in formula (II-1),
L₅ represents a bond or a divalent group,
HG represents a hydrophilic group,
L₃ₐ and L_{3b} each independently represent a bond or a divalent group, and
HG' represents a divalent hydrophilic group. 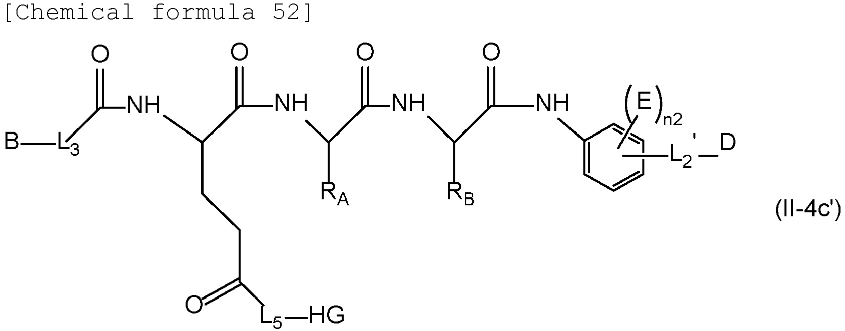
wherein
L₃, D, R_{A}, R_{B}, and B are the same as those in formula (II-1),
L₅ represents a bond or a divalent group,
HG represents a hydrophilic group,
L₂' represent a bond or a divalent group, and
E represents an electron-withdrawing group, and
n2 is an integer of 1 to 4.

In formulae (II-1a') to (II-4c'), definitions, examples, and preferred examples of structural units represented by the divalent group and the hydrophilic group are the same as those described above. Definition, examples, and preferred examples of the divalent hydrophilic group, the electron-withdrawing group and its binding site, and n2 are the same as those described above.

In a preferred embodiment, the structural unit represented by "-L₅-HG" may be selected from the group consisting of the following:
(a)

   -OH
(b)

   -NH(CH₂CH₂O)₈CH₃
(c)

   -NHCH₂CH₂COOH

In a specific embodiment, the compound represented by formula (II-1a') may be the following: 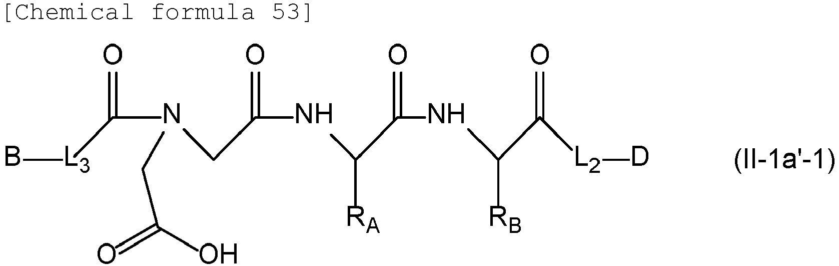 wherein
L₂, L₃, D, R_{A}, R_{B}, and B are the same as those in formula (II-1). 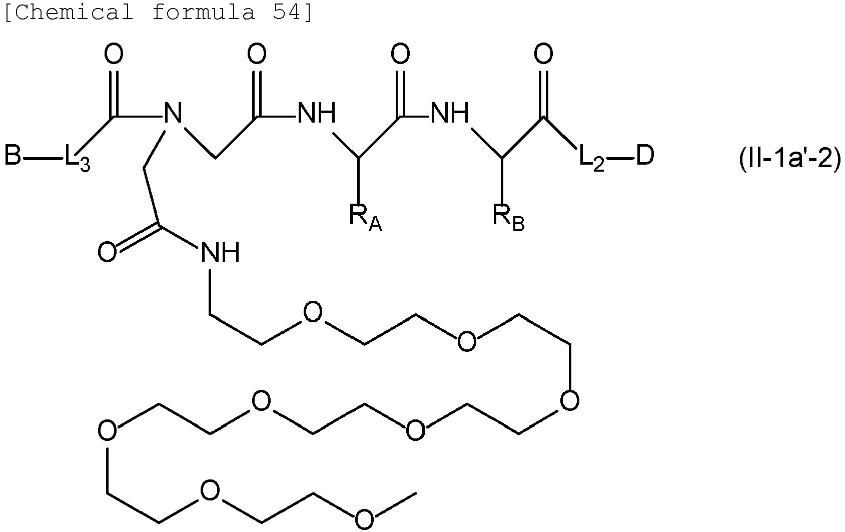 wherein
L₂, L₃, D, R_{A}, R_{B}, and B are the same as those in formula (II-1).

In another specific embodiment, the compound represented by formula (II-1b') may be the following: 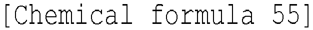 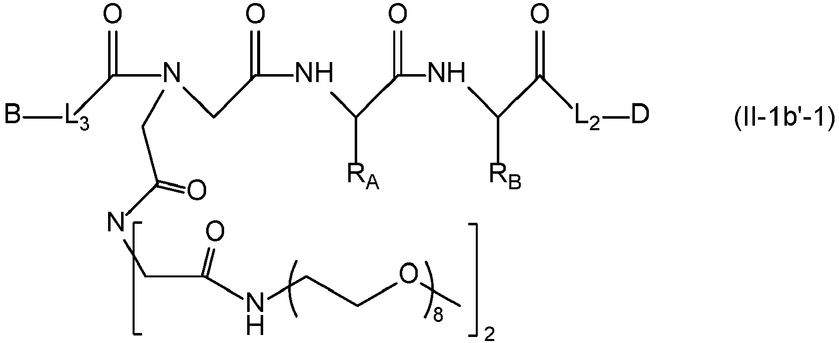 wherein
L₂, L₃, D, R_{A}, R_{B}, and B are the same as those in formula (II-1).

In another specific embodiment, the compound represented by formula (II-1c') may be the following: 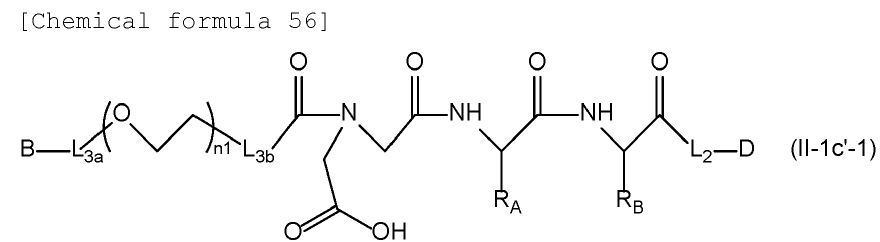 wherein
L₂, D, R_{A}, R_{B}, and B are the same as those in formula (II-1) ;
L₃ₐ and L_{3b} each independently represent a bond or a divalent group, and
n1 is a integer of 3 to 20.

Definition, examples, and preferred examples of the divalent group represented by L₃ₐ and L_{3b} and n1 are the same as those described above.

In another specific embodiment, the compound represented by formula (II-2') may be the following: 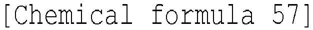 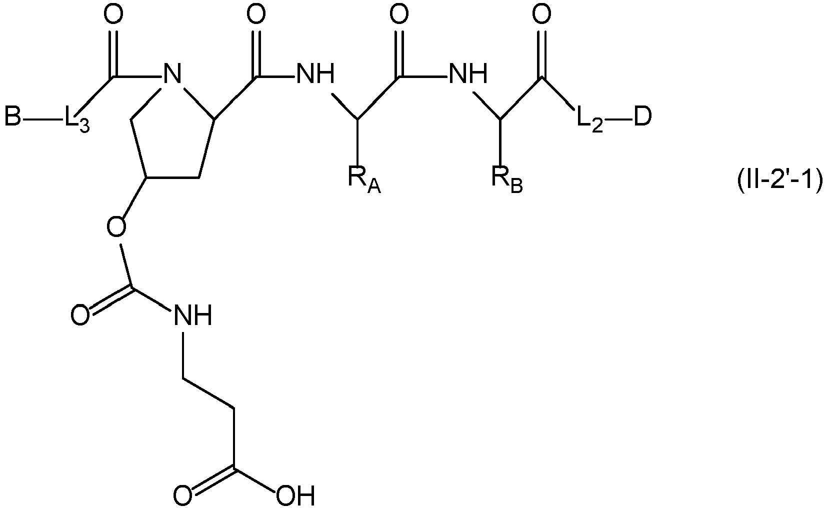 wherein
L₂, L₃, D, R_{A}, R_{B}, and B are the same as those in formula (II-1). 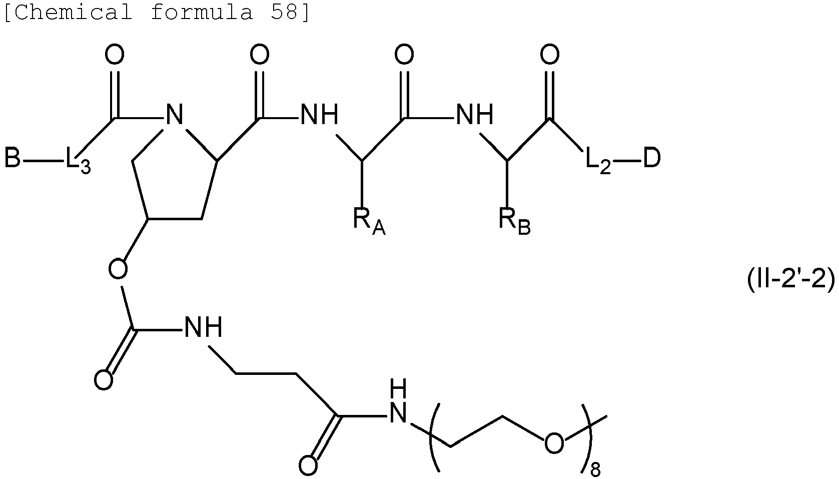 wherein
L₂, L₃, D, R_{A}, R_{B}, and B are the same as those in formula (II-1).

In still another specific embodiment, the compound represented by formula (II-3a') may be the following: 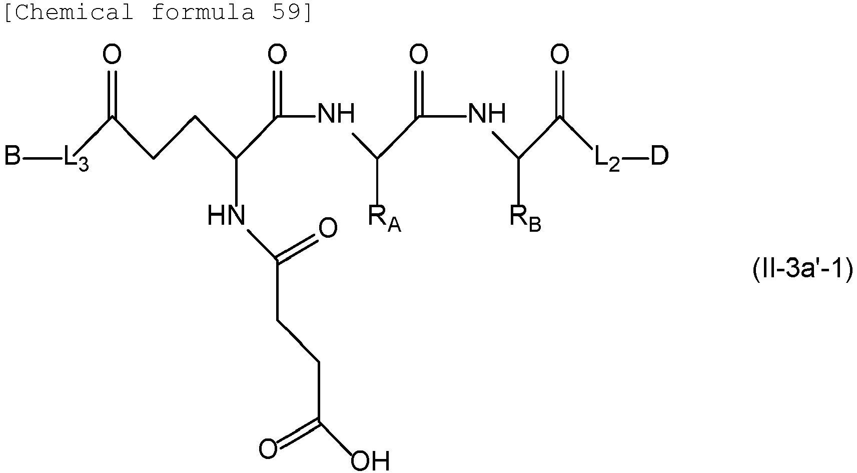 wherein
L₂, L₃, D, R_{A}, R_{B}, and B are the same as those in formula (II-1). 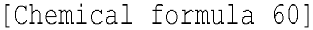 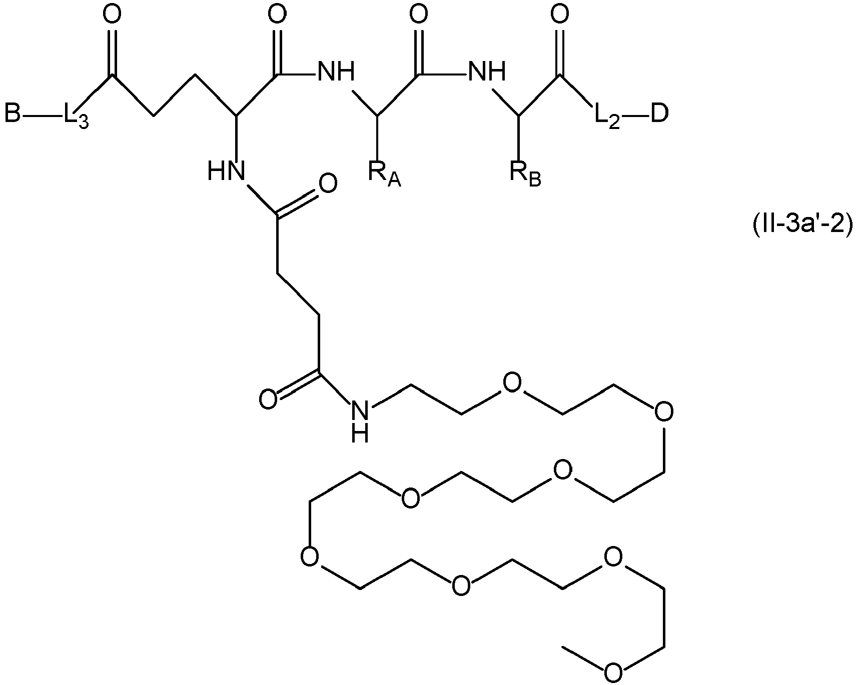 wherein
L₂, L₃, D, R_{A}, R_{B}, and B are the same as those in formula (II-1).

In still another specific embodiment, the compound represented by formula (II-3b') may be the following: 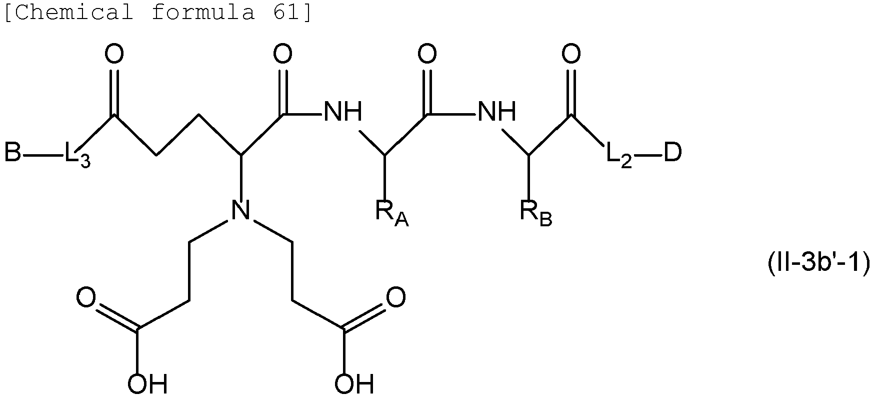 wherein
L₂, L₃, D, R_{A}, R_{B}, and B are the same as those in formula (II-1).

In a further still another specific embodiment, the compound represented by formula (II-4a') may be the following: 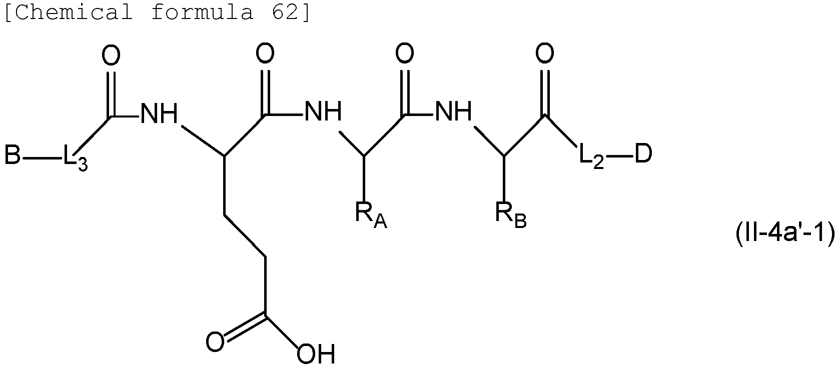 wherein
L₂, L₃, D, R_{A}, R_{B}, and B are the same as those in formula (II-1).

In a further still another specific embodiment, the compound represented by formula (II-4b') may be the following: 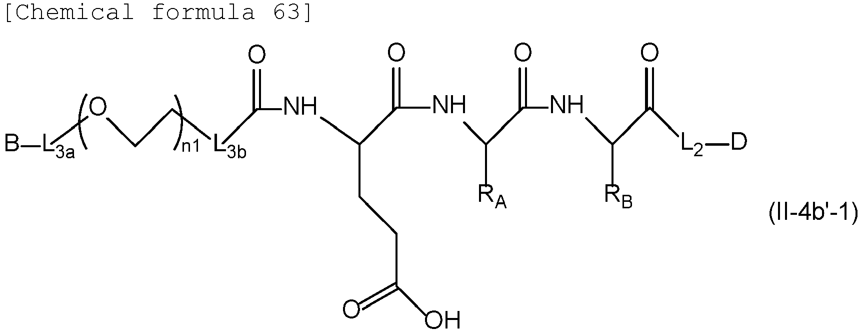 wherein
L₂, D, R_{A}, R_{B}, and B are the same as those in formula (II-1),
L₃ₐ and L_{3b} each independently represent a bond or a divalent group, and
n1 is a integer of 3 to 20.

In a further still another specific embodiment, the compound represented by formula (II-4c') may be the following: 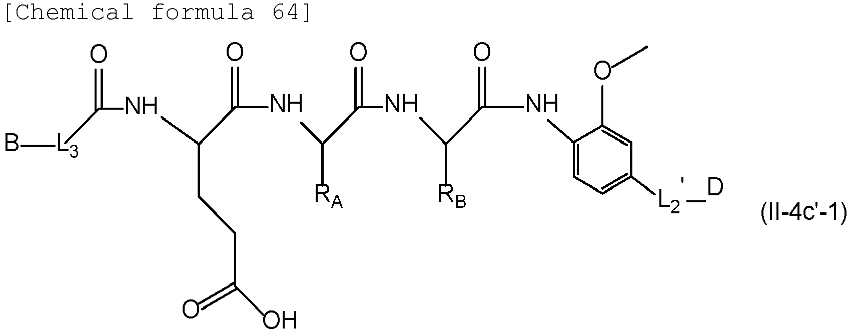 wherein
L₃, D, R_{A}, R_{B}, and B are the same as those in formula (II-1), and
L₂' represents a bond or a divalent group; or 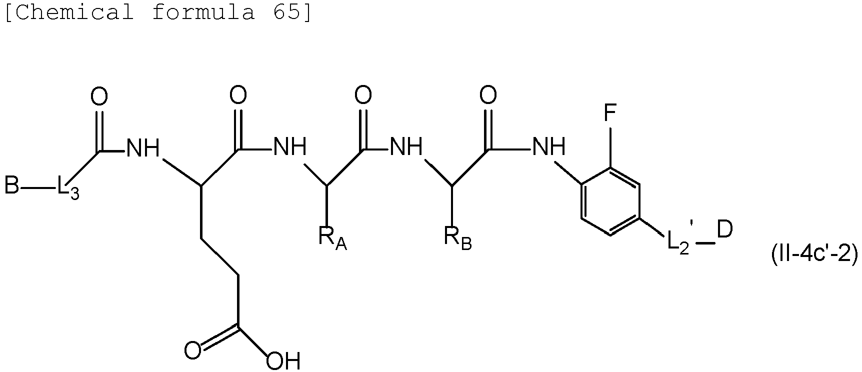
wherein
   L₃, D, R_{A}, R_{B}, and B are the same as those in formula (II-1), and
   L₂' represents a bond or a divalent group.

In a preferred embodiment, in the above formula, L₂ may be a divalent group represented by the following structural formula: 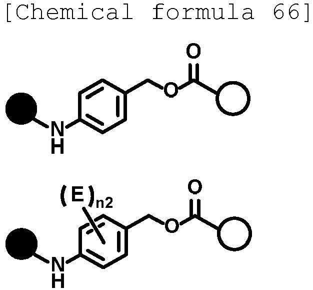
wherein a black circle and a white circle each represent a bond,
the bond of the black circle is bonded to a carbonyl group adjacent to L₂,
the bond of the white circle is bonded to D
E represents an electron-withdrawing group, and
n2 is an integer of 1 to 4.

Definition, examples, and preferred examples of the electron-withdrawing group and its binding site, and n2 are the same as those described above.

In a more preferred embodiment, the compound represented by the above formula (II) or a salt thereof may be a structural unit represented by the above formula (II-1a'), (II-1b'), (II-2'), (II-3a'), (II-3b'), (II-4b'), or (II-4c'), or a formula corresponding to a subordinate concept of these formulae. Such a compound or a salt thereof is useful as a synthetic intermediate in production of a conjugate that can be excellent in performance among the compounds represented by the above formula (II) and salts thereof (see, EXAMPLES).

The raw material antibody comprises a lysine residue regioselectively modified with a bioorthogonal functional group. The bioorthogonal functional group in the raw material antibody may be a maleimide residue, a thiol residue, a furan residue, a halocarbonyl residue, an alkene residue, an alkyne residue, an azide residue, or a tetrazine residue. The bioorthogonal functional group in the raw material antibody can be selected so as to be capable of reacting with a bioorthogonal functional group (e.g., bioorthogonal functional group represented by B) in the above-described compound or a salt thereof.

In a specific embodiment, the raw material antibody may comprise an immunoglobulin unit represented by the following formula (III): 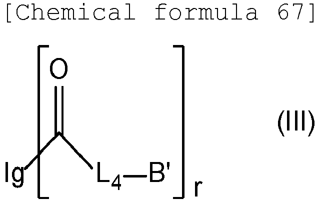 wherein
Ig represents an immunoglobulin unit comprising two heavy chains and two light chains, and regioselectively forms an amide bond with a carbonyl group adjacent to Ig via an amino group in side chains of lysine residues in the two heavy chains,
L₄ represents a divalent group selected from the group consisting of -(C(R)₂)ₘ-, -(O-C(R)₂-C(R)₂)ₘ-, and -(C(R)₂-C (R)₂-O)ₘ-,
Rs each independently represent a hydrogen atom, a C₁-₆ alkyl, a C₂₋₆ alkenyl, or a C₂₋₆ alkynyl,
m is an integer of 0 to 10,
B' represents a bioorthogonal functional group capable of reacting with a bioorthogonal functional group represented by B, and
an average ratio r of the amide bonds per two heavy chains is 1.5 to 2.5. Definitions, examples, and preferred examples of Ig, L₄, R, m, and r in the above formula (III) are the same as those described above.

The bioorthogonal functional group represented by B' is the same as the bioorthogonal functional group described above for the raw material antibody.

The reaction can be appropriately performed under a condition incapable of causing denaturation or decomposition (e.g., cleavage of an amide bond) of proteins (a mild condition). Such a reaction can be performed in an appropriate reaction system such as a buffer at room temperature (e.g., about 15°C to 30°C), for example. The pH of the buffer is e.g., 5 to 9, preferably 5.5 to 8.5, and more preferably 6.0 to 8.0. The buffer may comprise an appropriate catalyst. The reaction time is e.g., 1 minute to 20 hours, preferably 10 minutes to 15 hours, more preferably 20 minutes to 10 hours, and even more preferably 30 minutes to 8 hours. For the details of such a reaction, see, e.g., G. J. L. Bernardes et al., Chem. Rev., 115,2174 (2015); G. J. L. Bernardes et al., Chem. Asian. J., 4,630 (2009); B. G. Davies et al., Nat. Commun., 5,4740 (2014); A. Wagner et al., Bioconjugate. Chem., 25,825 (2014).

Production of the conjugate or a salt thereof can be confirmed by, for example, reverse phase HPLC under reducing conditions or mass spectrometry, depending on a specific raw material thereof and the molecular weight of a product. The conjugate or a salt thereof can be appropriately purified by any method such as chromatography (e.g., affinity chromatography).

The conjugate of the present invention or a salt thereof can be used as, for example, a medicament or a reagent (e.g., a diagnostic medicament or a research reagent).

The conjugate of the present invention or a salt thereof may be provided in a form of a pharmaceutical composition. Such a pharmaceutical composition may comprise a pharmaceutically allowable carrier in addition to the conjugate of the present invention or a salt thereof. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The conjugate of the present invention or a salt thereof may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set appropriately.

### 3. Compound or salt thereof, and reagent comprising same

The present invention also provides a compound represented by the above formula (II-1), (II-2), or (II-3), or a salt thereof. Details of these compounds or salts thereof are the same as those described above.

In a preferred embodiment, the compound represented by the above formula (II-1), (II-2), or (II-3), or a salt thereof may be a compound represented by the above formula (II-1a'), (II-1b'), (II-2'), (II-3a'), or (II-3b'), or a formula corresponding to a subordinate concept of these formulae or a salt thereof. Details of these compounds or salts thereof are the same as those described above.

The compound of the present invention or a salt thereof is useful as a synthetic intermediate in production of a conjugate that can be excellent in performance (see, EXAMPLES).

The present invention also provides a reagent for derivatizing an antibody, the reagent comprising a compound represented by the above formula (II-1), (II-2), (II-3), (II-4b') or (II-4c') above, or a salt thereof.

Details of a raw material antibody derivatized with the reagent of the present invention, or a salt thereof, and a conjugate obtained by a derivatization reaction of the raw material antibody or a salt thereof, or a salt thereof are as described above.

The reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Tris-hydrochloric acid buffer, a carbonic acid-bicarbonic acid buffer, an aqueous boric acid solution, a glycine-sodium hydroxide buffer, and a citric acid buffer); buffers are preferred. The pH of solutions is e.g., 5.0 to 9.0 and preferably 5.5 to 8.5. The reagent of the present invention can be provided in a liquid form or a powder form (e.g., freeze-dried powder).

### EXAMPLES

The present invention is explained by the following Examples in more detail, but the present invention is not limited to the following Examples.

### EXAMPLE 1: Synthesis of Linker-payload mimic

### (1-1) Synthesis of Linker-payload mimic (1)

Linker-payload mimic (1) was synthesized as follows.

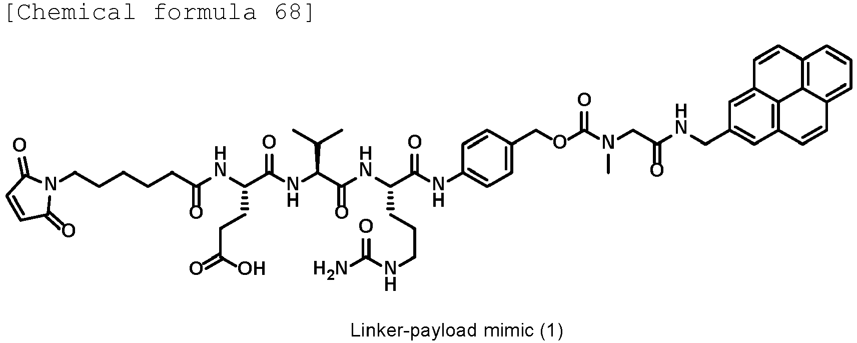

### (1-1-1) Synthesis of pyrene (2)

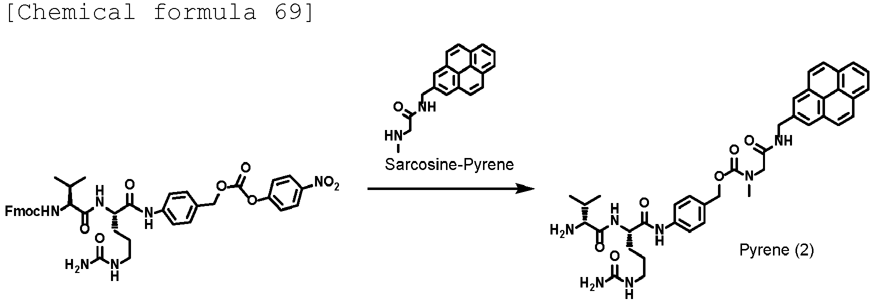

Fmoc-Val-Cit-PAB-PNP (CAS No: 863971-53-2, 121.2 mg, 0.15 mmol) was dissolved in N,N-dimethylformamide (5 mL), and well-known (described in WO 2018218004 A1) sarcosine-pyridine (59.2 mg, 0.196 mmol), N,N-diisopropylethylamine (39 µL, 0.227 mmol), and 4-dimethylaminopyridine (3.7 mg, 0.03 mmol) were added thereto. The mixture was stirred at room temperature for two hours, and then diethylamine (2 mL, 18.95 mmol) was added thereto, and the mixture was stirred at room temperature for 1.5 hours. The mixture was concentrated under reduced pressure, and then purified by reverse phase preparative chromatography. A fraction comprising a product was collected and concentrated under reduced pressure to remove acetonitrile. The residue was freeze-dried to obtain the pyrene (2) (73.7 mg, 0.104 mmol).
¹H NMR(400MHz, DMSO-d6)δ10.21(s, 1H), 8.68(s, 1H), 8.43-7.93(m, 12H), 7.60(t, J=7.1Hz, 2H), 7.31(m, 2H), 6.04(s, 1H), 5.48(s, 2H), 5.02(d, J=16.1Hz, 4H), 4.54(s, 1H), 3.96(s, 2H), 3.66(s, 2H), 2.92(d, J=6.1Hz, 3H), 2.08(q, J=6.6Hz, 1H), 1.80-1.56(m, 2H), 1.46(s, 2H), 1.21-1.13(m, 1H), 0.94(dt, J=6.8, 3.0Hz, 6H).
MS(ESI)m/z:708.80[M+H]⁺

### (1-1-2) Synthesis of pyrene (3)

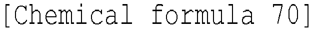

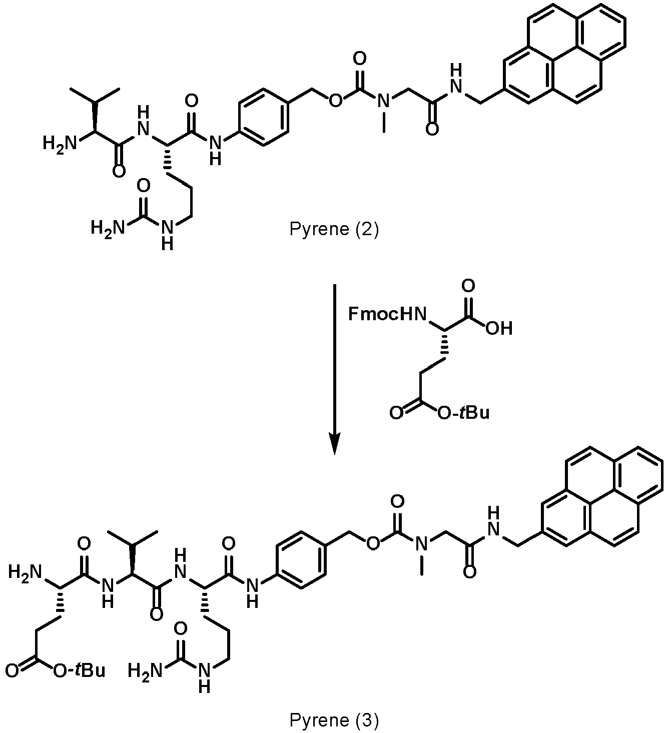

Fmoc-Glu (OtBu)-OH•H₂O (11.1 mg, 0.025 mmol) was dissolved in dimethylformamide (1 mL), and pyrene (2) (17.3 mg, 0.024 mmol), 1-hydroxy-7 azabenzotriazole (5.1 mg, 0.037 mmol), 1-(3-dimethylaminopropyl)-3-ethylcarbodiimide hydrochloride (7.3 mg, 0.038 mmol), and triethylamine (7.1 µL, 0.51 mmol) were added thereto. The mixture was stirred at room temperature for 2.5 hours, and then diethylamine (0.2 mL, 1.91 mmol) was added thereto. The mixture was stirred at room temperature for 1.5 hours. The mixture was concentrated under reduced pressure, and then purified by reverse phase preparative chromatography. A fraction comprising a product was collected and concentrated under reduced pressure to remove acetonitrile. The residue was freeze-dried to obtain pyrene (3) (15.6 mg, 0.017 mmol).
¹H NMR(400MHz, Chloroform-d)δ10.87(s, 1H), 8.68(m, 1H), 8.41-7.97(m, 13H), 7.60-7.58(m, 2H), 7.30(m, 2H), 6.00(t, J=6.2Hz, 1H), 5.45(s, 2H), 5.05-4.99(m, 4H), 4.46(m, 1H), 4.26(m, 1H), 3.96(s, 2H), 3.88(m, 1H), 3.07(m, 1H), 2.96(m, 1H), 2.93(d, J=5.6Hz, 3H), 2.68(t, J=1.8Hz, 1H), 2.34-2.30(m, 3H), 2.03(m, 1H), 1.93-1.90(m, 2H), 1.30(s, 9H), 1.15(s, 1H), 0.92-0.86(m, 6H).
MS(ESI)m/z:893.45[M+H]⁺

### (1-1-3) Synthesis of pyrene (4)

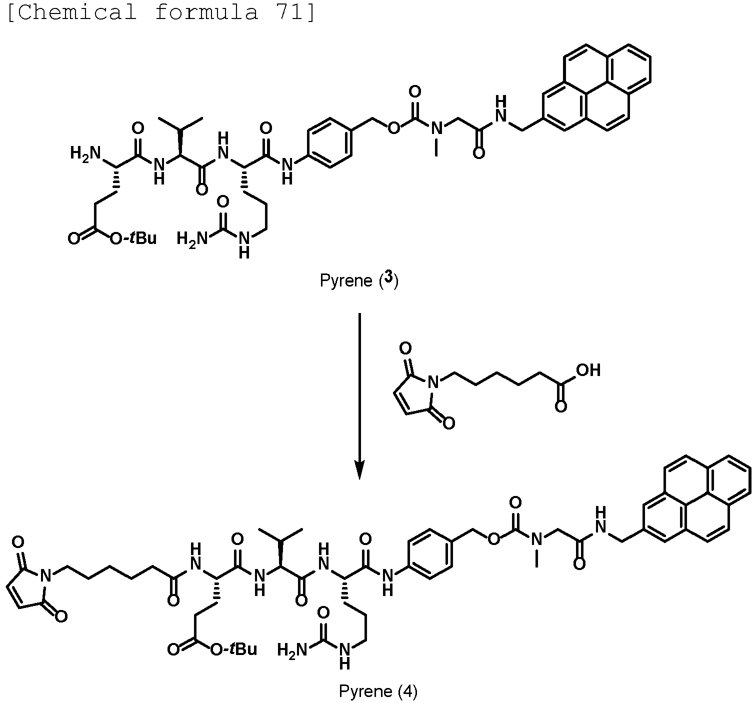

Pyrene (3) (15.6 mg, 0.017 mmol) was dissolved in dimethylformamide (1.5 mL), and 6-maleimidohexanoic acid (3.7 mg, 0.018 mmol), 1-hydroxy-7 azabenzotriazole (3.5 mg, 0.025 mmol), 1-(3-dimethylaminopropyl)-3-ethylcarbodiimide hydrochloride (4.8 mg, 0.025 mmol), and triethylamine (4.8 µL, 0.34 mmol) were added thereto. The mixture was stirred at room temperature for three hours, and then 6-maleimidohexanoic acid (1.8 mg, 0.009 mmol), 1-(3-dimethylaminopropyl)-3 ethylcarbodiimide hydrochloride (2.3 mg, 0.012 mmol), and triethylamine (2.4 µL, 0.17 mmol) were added thereto. The mixture was stirred at room temperature for 1.5 hours. Thereafter, 4-dimethylaminopyridine (0.5 mg, 0.004 mmol) was added thereto, and the mixture was further stirred for 2.5 hours. The mixture was concentrated under reduced pressure, and then purified by column chromatography (dichloromethane : methanol = 9 . 1). A fraction comprising a product was collected and concentrated under a reduced pressure to obtain the pyrene (4) (8.0 mg, 0.007 mmol).
MS(ESI)m/z:1086.60[M+H]⁺

### (1-1-4) Synthesis of Linker-payload mimic (1)

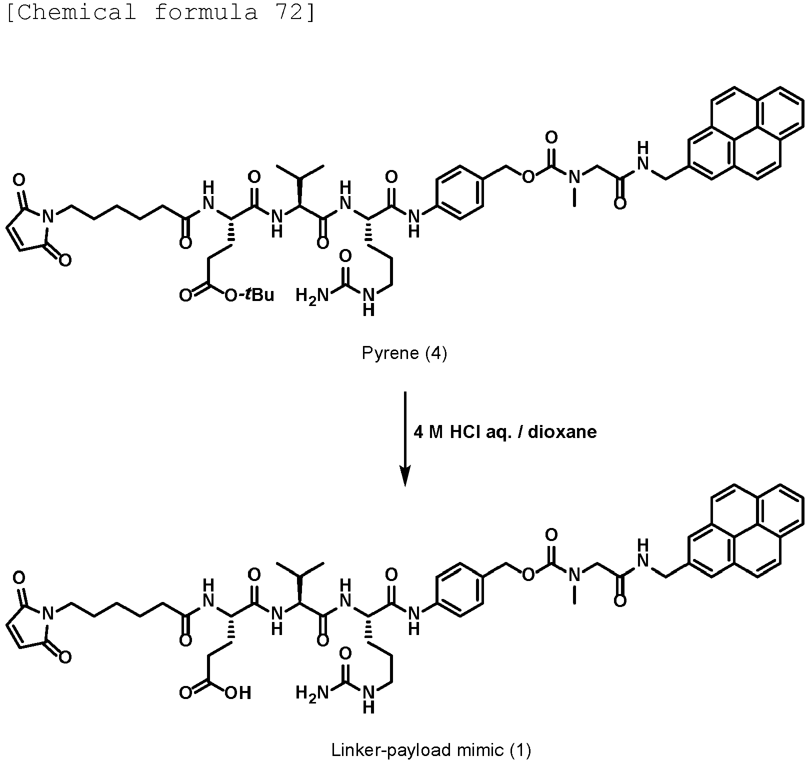

Pyrene (4) (9.7 mg, 0.0089 mmol) was dissolved in 1,4-dioxane (1 mL) and a hydrogen chloride/1,4-dioxane solution (1 mL), and the solution was stirred in an ice water bath for two hours. Dimethylformamide (1 mL) was added thereto to raise the temperature to room temperature, and then the mixture was concentrated under reduced pressure. Thereafter, purification was performed by reverse phase preparative chromatography. A fraction comprising a product was collected and concentrated under reduced pressure to remove acetonitrile. The residue was freeze-dried to obtain Linker-payload mimic (1) (2.0 mg, 0.002 mmol).
MS(ESI)m/z:1030.50[M+H]⁺

### (1-2) Synthesis of Linker-payload mimic (5) Linker-payload mimic (5) was synthesized as follows.

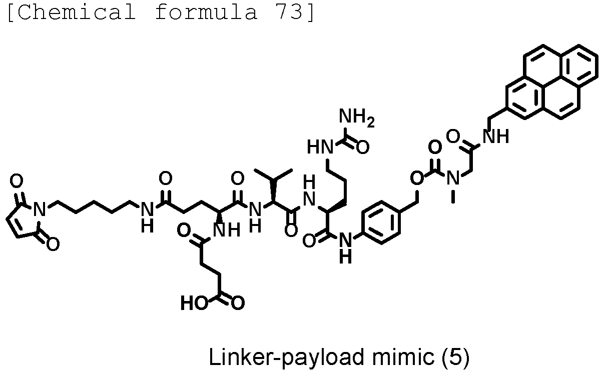

### (1-2-1) Synthesis of maleimide (6)

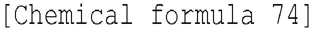

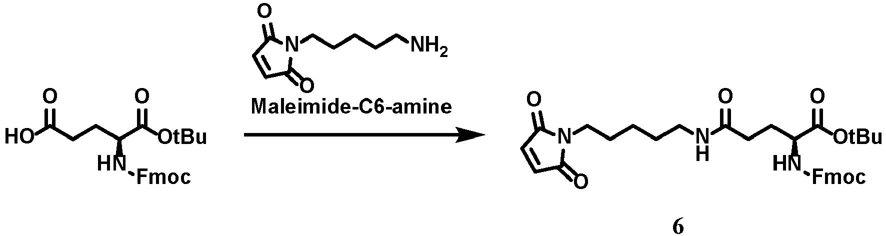

Fmoc-Glu-OtBu (213.0 mg, 0.50 mmol) was dissolved in dichloromethane (2 mL), and N-(5-aminopentyl) maleimide hydrochloride (108.7 mg, 0.48 mmol), 1-hydroxybenzotriazole (101.7 mg, 0.753 mmol), 1-ethyl-3-(3-dimethylaminopropyl) carbodiimide hydrochloride (143.6 mg, 0.749 mmol), triethylamine (155 µL, 1.12 mmol), and 4-dimethylaminopyridine (7.2 mg, 0.0589 mmol) were added thereto. The mixture was stirred at room temperature for two hours. The mixture was concentrated under reduced pressure, and then purified by column chromatography (hexane : ethyl acetate = 4 . 1). A fraction comprising a product was collected and concentrated under a reduced pressure to obtain the maleimide (6) (254.1 mg, 0.431 mmol) .
¹H NMR(400 MHz, Methanol-d4)δ7.82(d, J=7.6 Hz, 3H), 7.70(t, J=6.7 Hz, 2H), 7.41(t, J=7.5 Hz, 2H), 7.37-7.29(m, 2H), 6.79(s, 2H), 4.43(dd, J=10.4, 6.8 Hz, 1H), 4.34(dd, J=10.4, 7.0 Hz, 1H), 4.24(t, J=6.9 Hz, 1H), 4.18-4.00(m, 1H), 3.50(t, J=7.1 Hz, 2H), 3.16(td, J=6.9, 2.0 Hz, 2H), 2.28(t, J=7.5 Hz, 2H), 2.24-2.08(m, 1H), 1.99-1.83(m, 1H), 1.48(s, 13H), 1.28(dt, J=17.1, 7.1 Hz, 2H).
MS(ESI)m/z:590.25[M+H]⁺

### (1-2-2) Synthesis of maleimide (7)

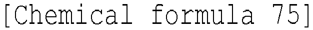

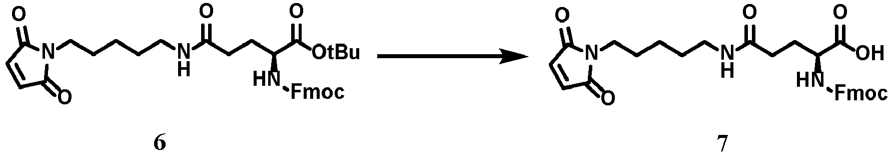

Maleimide (6) (253.1 mg, 0.41 mmol) was dissolved in dichloromethane (2 mL), and trifluoroacetic acid (2 mL) was added thereto. The mixture was stirred at room temperature for two hours. The mixture was concentrated under reduced pressure, and then 1 M hydrochloric acid (2 mL) was added thereto. The mixture was stirred at room temperature for three minutes, concentrated under reduced pressure, and then freeze-dried to obtain the maleimide (7) (244.6 mg, 0.458 mmol).
¹H NMR(400 MHz, Methanol-d4)δ7.82(d, J=7.5 Hz, 2H), 7.70(dt, J=9.3, 4.7 Hz, 2H), 7.41(t, J=7.5 Hz, 2H), 7.33(t, J=7.4 Hz, 2H), 6.79(s, 2H), 4.49-4.30(m, 2H), 4.30-4.11(m, 2H), 3.50(t, J=7.0 Hz, 2H), 3.16(t, J=7.2 Hz, 2H), 2.36-2.09(m, 3H), 2.09-1.86(m, 1H), 1.56(dt, J=25.5, 7.5 Hz, 5H), 1.39-1.23(m, 2H).
MS(ESI)m/z:534.20[M+H]⁺

### (1-2-3) Synthesis of pyrene (8)

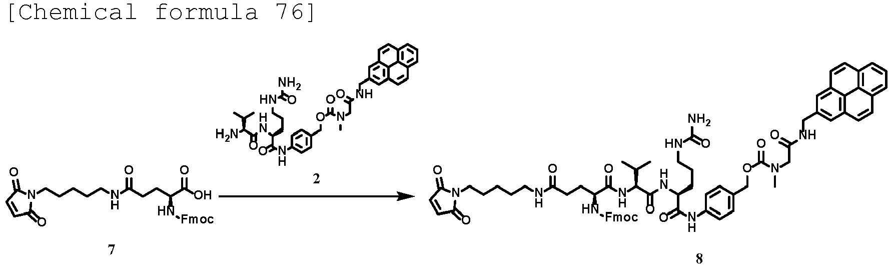

Maleimide (7) (31.5 mg, 0.052 mmol) was dissolved in N,N-dimethylformamide (2 mL), and pyrene (2) (35.5 mg, 0.50 mmol) synthesized in Example 1-1-1, 1-hydroxybenzotriazole (10.1 mg, 0.0747 mmol), 1-ethyl-3-(3-dimethylaminopropyl) carbodiimide hydrochloride (15.0 mg, 0.0782 mmol), triethylamine (14 µL, 0.10 mmol), and 4-dimethylaminopyridine (0.7 mg, 0.00573 mmol) were added thereto. The mixture was stirred at room temperature for two hours, and then purified by column chromatography (hexane : ethyl acetate = 15 : 85). A fraction comprising a product was collected and concentrated under a reduced pressure to obtain the pyrene (8) (7.4 mg, 6.05 nmol).
MS (ESI)m/z: 1223. 65 [M+H]⁺, 612.65 [M+2H]²⁺

### (1-2-4) Synthesis of Linker-payload mimic (5)

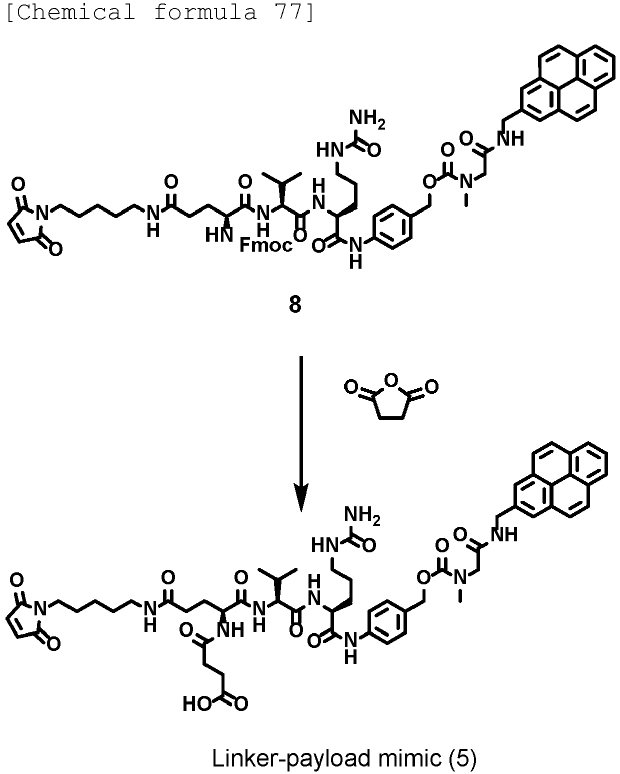

Pyrene (8) (6.3 mg, 0.0051 mmol) was dissolved in N,N-dimethylformamide (0.6 mL), and dicyclohexylamine (0.12 mL, 0.6 mmol) was added thereto. The mixture was stirred at room temperature for three hours, and then succinic anhydride (1.0 mg, 0.0095 mmol) was added thereto. The mixture was stirred at room temperature for 1.5 hours, and then purified by reverse phase preparative chromatography. A fraction comprising a product was collected and concentrated under reduced pressure to remove acetonitrile. The residue was freeze-dried to obtain Linker-payload mimic (5) (1.10 mg, 0.991 nmol).
¹H NMR(400MHz, DMSO-d6)δ10.01(s, 1H), 8.59(d, J=6.2Hz, 1H), 8.36-7.87(m, 10H), 7.67(t, J=8.7Hz, 2H), 7.22(dd, J=26.1, 8.1Hz, 3H), 6.91(s, 2H), 5.92(s, 1H), 4.94(d, J=18.4Hz, 4H), 4.33(d, J=7.5Hz, 1H), 3.88(s, 2H), 3.10(s, 3H), 3.05-2.78(m, 7H), 2.69(d, J=22.3 Hz, 1H), 2.38-2.22(m, 3H), 2.15-1.89(m, 4H), 1.81(d, J=14.3Hz, 1H), 1.72-1.50(m, 3H), 1.48-1.22(m, 6H), 1.14(dd, J=35.5, 8.5Hz, 4H), 0.92(s, 1H), 0.85-0.68(m, 6H).
MS(ESI)m/z:1101.55[M+H]⁺

### (1-3) Synthesis of Linker-payload mimic (9)

Linker-payload mimic (9) was synthesized as follows.

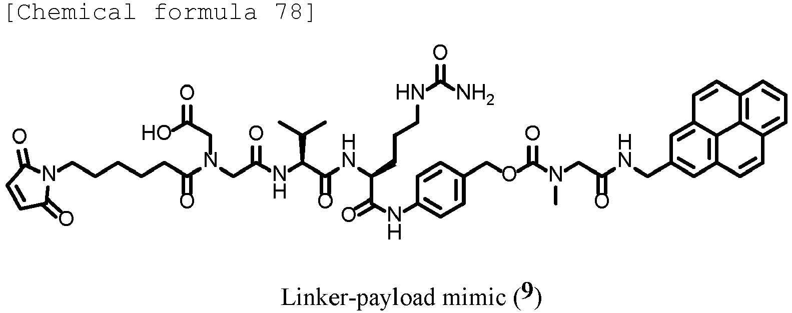

### (1-3-1) Synthesis of maleimide (10)

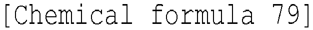

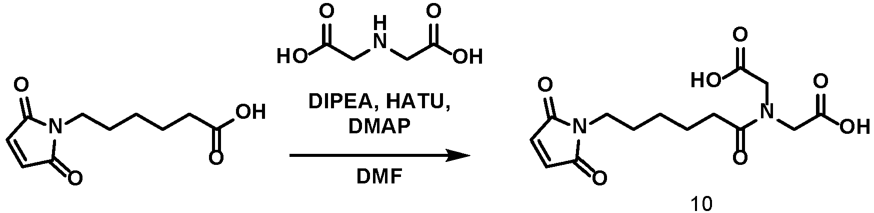

6-Maleimidohexanoic acid (300 mg, 1.420 mmol) was dissolved in dimethylformamide (2 mL), and 1-[bis(dimethylamino) methylene]-1H-1,2,3,-triazolo[4,5-b]pyridinium 3-oxide hexafluorophosphate (647.8 mg, 1.704 mmol), iminodiacetic acid (189.0 mg, 1.420 mmol), N,N-diisopropylethylamine (0.29 mL, 1.704 mmol), and 4-dimethylaminopyridine (34.7 mg, 0.284 mmol) were added thereto. The mixture was stirred for one hour. The mixture was concentrated under reduced pressure, and then purified by reverse phase preparative chromatography. A fraction comprising a product was collected and concentrated under reduced pressure to remove acetonitrile. The residue was freeze-dried to obtain maleimide (10) (65.9 mg, 0.202 mmol).
¹H NMR(400 MHz, DMSO-d6)δ6.79(s, 2H), 4.25(s, 2H), 4.11(s, 2H), 3.49(t, J=7.0Hz, 2H), 2.35(t, J=7.4Hz, 2H), 1.16(m, 4H), 1.33(m, 2H)
MS(ESI)m/z:327.00[M+H]⁺

### (1-3-2) Synthesis of Linker-payload mimic (9)

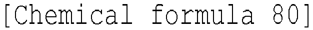

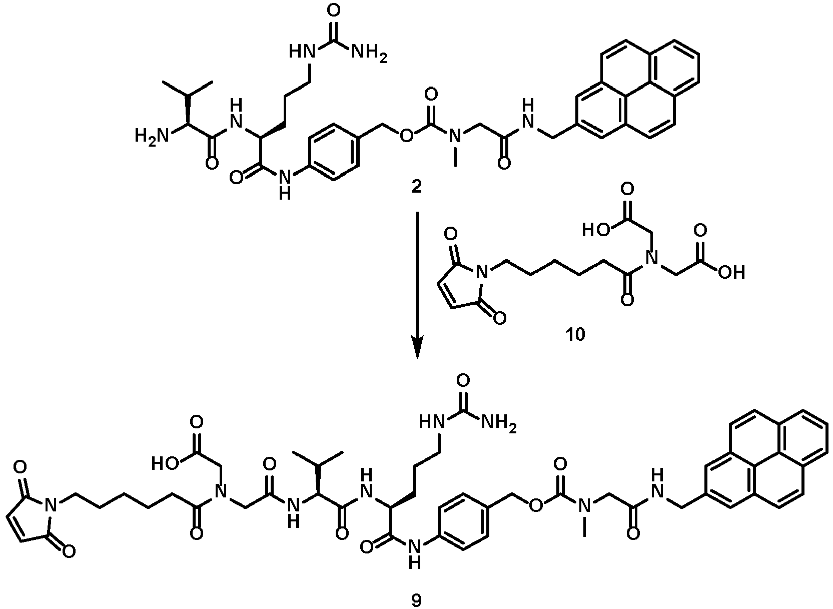

Maleimide (10) (46.2 mg, 0.142 mmol) was dissolved in dimethylformamide (0.3 mL), and 1-[bis (dimethylamino) methylene]-1H-1,2,3,-triazolo[4,5-b]pyridinium 3-oxide hexafluorophosphate (54.0 mg, 0.142 mmol) was added thereto. The mixture was stirred for one hour, and then a dimethylformamide solution (0.3 mL) of pyrene (2) (50.1 mg, 0.71 mmol) synthesized in Example 1-1-1 and N,N-diisopropylethylamine (0.025 mL, 0.142 mmol) was added thereto. The mixture was further stirred for three hours. After purification by reverse phase preparative chromatography, a fraction comprising a product was collected and concentrated under reduced pressure to remove acetonitrile. The residue was freeze-dried to obtain Linker-payload mimic (9) (24.4 mg, 0.024 mmol).
MS(ESI)m/z:1016.50[M+H]⁺

### EXAMPLE 2: Synthesis of Linker-Payload

### (2-1) Synthesis of Linker-payload (11)

Linker-payload (11) was synthesized according to Example 1-1 using known Val-Cit-PABA-MMAE (Organic & Biomolecular Chemistry, 2016, 14, 9501-9518). 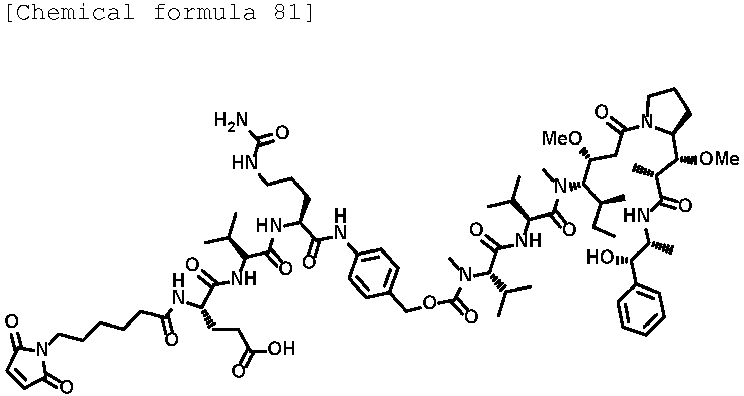 Linker-payload (11)

### MS(ESI)m/z:1445.8[M+H]⁺

### (2-2) Synthesis of Linker-payload (12)

Linker-payload (12) was synthesized as follows. 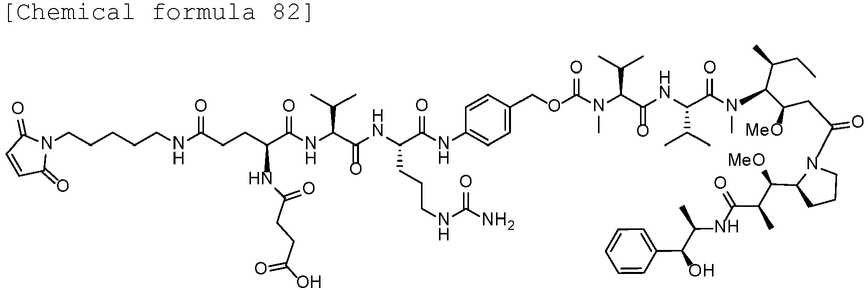 Linker-payload (12)

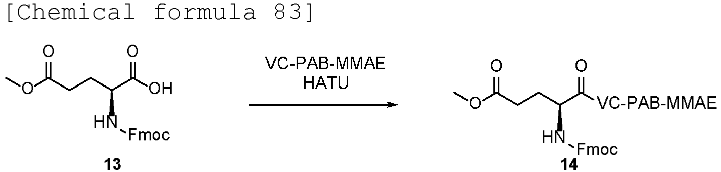

Fmoc-Glu (OMe)-H (56 mg, 0.15 mmol) was dissolved in dimethylformamide (1 mL), and 1-[bis(dimethylamino) methylene]-1H-1,2,3,-triazolo[4,5-b] pyridinium 3-oxide hexafluorophosphate (56 mg, 0.15 mmol) and N,N-diisopropylethylamine (0.046 mL, 0.27 mmol) were added thereto. The mixture was stirred at room temperature for one minute. To the reaction mixture, a DMF solution (1 mL) of known Val-Cit-PABA-MMAE (Organic & Biomolecular Chemistry, 2016, 14, 9501-9518) (150 mg, 0.13 mmol) was added, and the mixture was further stirred for 30 minutes. After completion of the reaction, the reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Compound 14 (180 mg, 0.12 mmol).

MS(ESI)m/z:1488.2[M+H]⁺

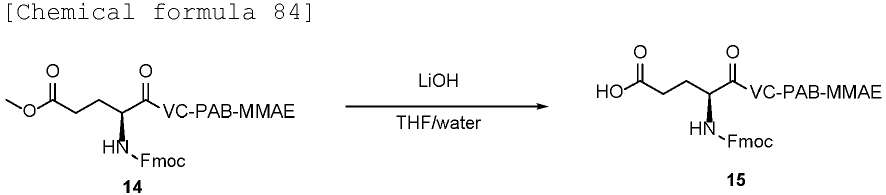

Lithium hydroxide (8.7 mg, 0.36 mmol) and Compound 14 (180 mg, 0.12 mmol) were dissolved in 1 : 1 = water : THF (8 mL), and the solution was stirred at room temperature for 15 minutes. After completion of the reaction, a 1 M aqueous hydrochloric acid solution was carefully added to the reaction solution to adjust the pH to 5 to 6. This was purified by reverse phase preparative chromatography. A fraction comprising a product was collected and concentrated under reduced pressure to remove acetonitrile. The residue was freeze-dried to obtain Compound 15 (100 mg, 0.068 mmol).
MS(ESI)m/z:1474.4[M+H]⁺

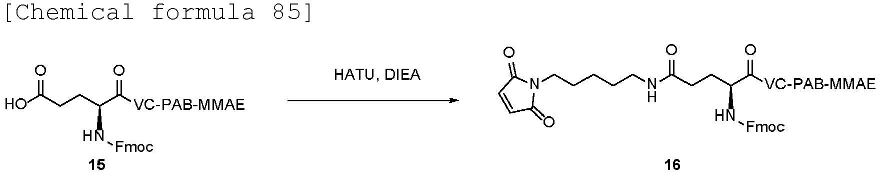

Compound 15 (100 mg, 0.068 mmol) was dissolved in dimethylformamide (2 mL), and 1-[bis(dimethylamino) methylene]-1H-1,2,3,-triazolo[4,5-b]pyridinium 3-oxide hexafluorophosphate (28 mg, 0.075 mmol) and N,N-diisopropylethylamine (0.035 mL, 0.20 mmol) were added thereto. The mixture was stirred at room temperature for one minute. To the reaction mixture, N-(5-aminopentyl) maleimide hydrochloride (30 mg, 0.10 mmol) was added, and the mixture was further stirred for five minutes. After completion of the reaction, the reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Compound 16 (83 mg, 0.051 mmol).
MS(ESI)m/z:1638.6[M+H]⁺

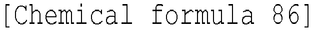

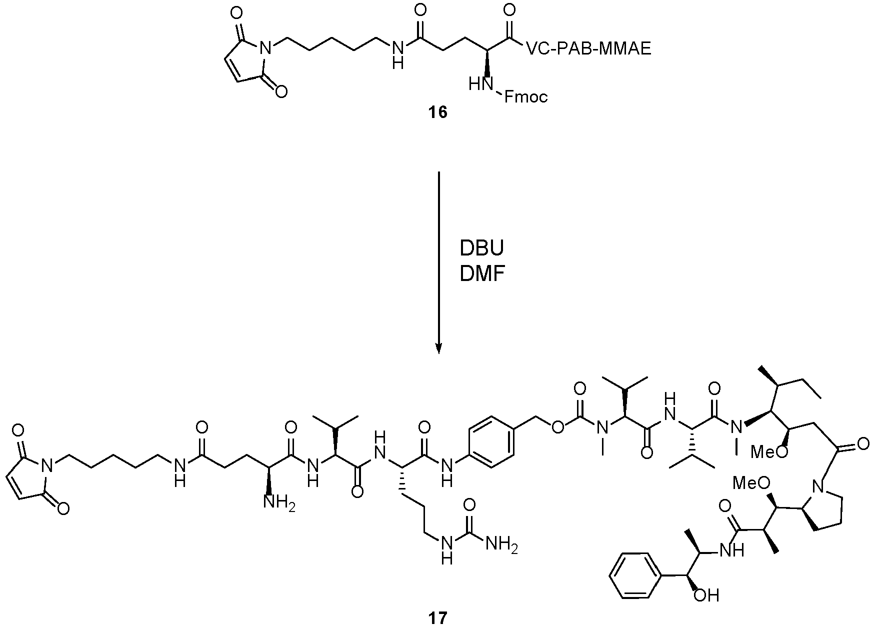

Compound 16 (21 mg, 0.013 mmol) was dissolved in dimethylformamide (1 mL), and diazabicycloundecene (0.0042 mL, 0.028 mmol) was added thereto. The mixture was stirred at room temperature for two minutes. After completion of the reaction, a 1 M aqueous hydrochloric acid solution was carefully added to the reaction solution to adjust the pH to 5 to 6. The reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Compound 17 (10 mg, 0.0071 mmol).
MS(ESI)m/z:1416.5[M+H]⁺

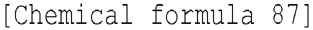

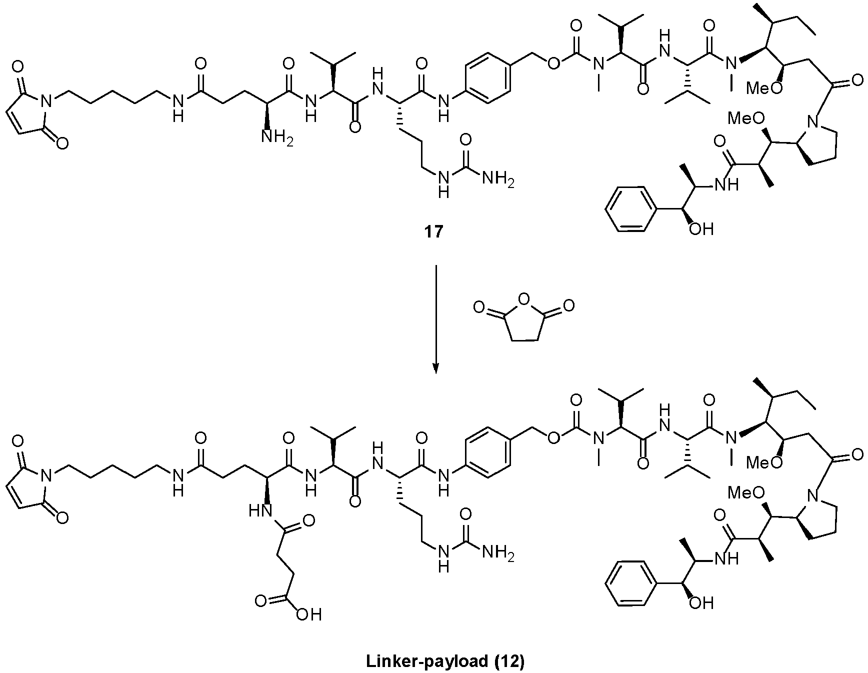

Compound (17) (21 mg, 0.015 mmol) was dissolved in N,N-dimethylformamide (2 mL) under an argon atmosphere, and 4-methylmorpholine (0.082 mL, 0.074 mmol) and succinic anhydride (7.4 mg, 0.074 mmol) were added thereto. The mixture was stirred at room temperature for one hour. Then, the reaction solution was diluted with a 1 : 1 = water: acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Linker-payload (12) (17.5 mg, 0.012 nmol).

¹H NMR(500 MHz, DMSO-d6)δ12.06(br.s, 1H), 9.94-10.05(m, 1H), 8.26-8.33(m, 0.5H), 8.13-8.20(m, 1H), 8.10(d, J=7.8Hz, 2H), 8.03-8.07(m, 0.5H), 7.86-7.90(m, 0.5H), 7.69-7.77(m, 2H), 7.60-7.64(m, 0.5H), 7.55-7.60(m, 2H), 7.29-7.35(m, 3H), 7.23-7.29(m, 3H), 7.17(s, 1H), 6.99(s, 2H), 5.94-6.02(m, 1H), 5.39-5.44(m, 2H), 5.34(d, J=4.9Hz, 1H), 4.94-5.12(m, 2H), 4.70-4.77(m, 1H), 4.58-4.68(m, 1H), 4.49-4.52(m, 1H), 4.40-4.45(m, 1H), 4.33-4.40(m, 1H), 4.21-4.29(m, 2H), 4.18-4.21(m, 1H), 3.92-4.02(m, 2H), 3.76-3.80(m, 1H), 3.53-3.61(m, 1H), 3.41-3.50(m, 1H), 3.36(t, J=7.1Hz, 2H), 3.29-3.31(m, 1H), 3.21-3.26(m, 4H), 3.20(s, 2H), 3.17(s, 2H), 3.11(s, 2H), 2.90-3.07(m, 5H), 2.82-2.89(m, 3H), 2.31-2.45(m, 5H), 2.22-2.30(m, 1H), 2.02-2.17(m, 3H), 1.97-2.01(m, 2H), 1.83-1.91(m, 1H), 1.75-1.82(m, 1H), 1.65-1.75(m, 2H), 1.50-1.62(m, 2H), 1.42-1.50(m, 4H), 1.26-1.39(m, 3H), 1.13-1.22(m, 2H), 0.96-1.06(m, 6H), 0.72-0.89(m, 24H)
MS(ESI)m/z:1516.4[M+H]⁺

### (2-3) Synthesis of Linker-payload (18)

Linker-Payload (18) was synthesized as follows.

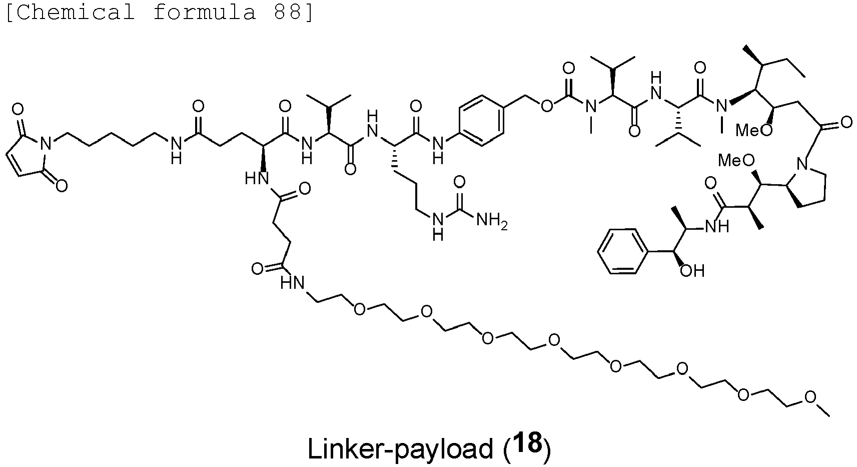

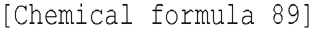

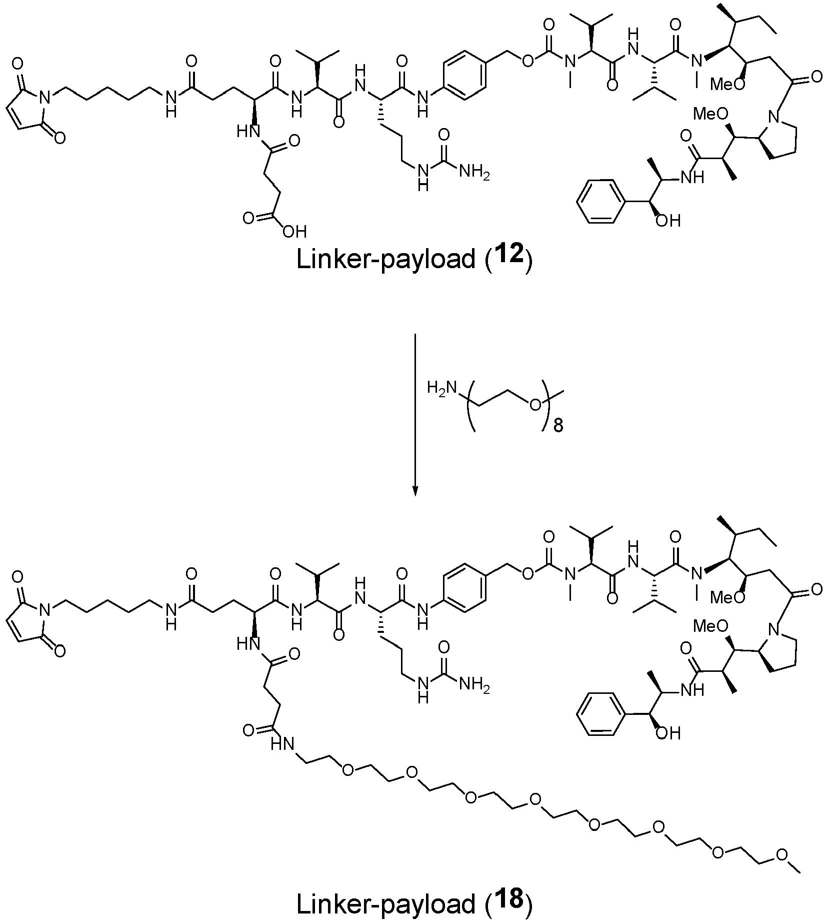

Linker-payload (12) (10 mg, 0.0066 mmol) was dissolved in dimethylformamide (1 mL), and 1-[bis(dimethylamino) methylene]-1H-1,2,3,-triazolo[4,5-b]pyridinium 3-oxide hexafluorophosphate (2.8 mg, 0.0073 mmol) and N,N-diisopropylethylamine (0.0023 mL, 0.013 mmol) were added thereto. The mixture was stirred at room temperature for one minute. To the reaction mixture, methoxy-PEG8-amine (3.8 mg, 0.0099 mmol) was added, and the mixture was further stirred for two minutes. After completion of the reaction, the reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Linker-payload (18) (12.1 mg, 0.064 mmol).
¹H NMR(500 MHz, DMSO-d6)δ9.89-10.00(m, 1H), 8.26-8.35(m, 1H), 8.10-8.15(m, 1H), 8.03-8.10(m, 1H), 7.92-7.96(m, 1H), 7.86-7.92(m, 0.5H), 7.71-7.78(m, 2H), 7.60-7.65(m, 0.5H), 7.53-7.60(m, 2H), 7.23-7.36(m, 6H), 7.13-7.20(m, 1H), 6.99(s, 2H), 5.93-6.00(m, 1H), 5.40-5.43(m, 2H), 5.32-5.36(m, 1H), 4.92-5.14(m, 2H), 4.69-4.78(m, 1H), 4.58-4.68(m, 1H), 4.45-4.51(m, 1H), 4.39-4.45(m, 1H), 4.31-4.39(m, 1H), 4.24-4.30(m, 1H), 4.19-4.24(m, 1H), 4.13-4.18(m, 1H), 3.91-4.05(m, 2H), 3.53-3.61(m, 1H), 3.46-3.53(m, 36H), 3.40-3.44(m, 3H), 3.33-3.40(m, 3H), 3.21-3.26(m, 6H), 3.14-3.21(m, 4H), 3.11(s, 1H), 2.91-3.06(m, 4H), 2.82-2.90(m, 2H), 2.22-2.44(m, 6H), 2.05-2.16(m, 3H), 1.94-2.04(m, 2H), 1.84-1.93(m, 1H), 1.75-1.83(m, 1H), 1.63-1.74(m, 2H), 1.50-1.62(m, 2H), 1.40-1.50(m, 4H), 1.31-1.40(m, 4H), 1.13-1.21(m, 4H), 0.95-1.07(m, 6H), 0.71-0.90(m, 24H)
MS(ESI)m/z:1882.6[M+H]⁺

### (2-4) Synthesis of Linker-payload (19)

Linker-Payload (19) was synthesized as follows.

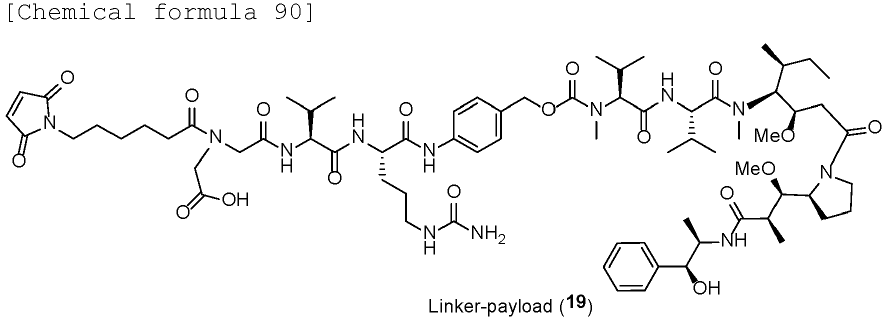

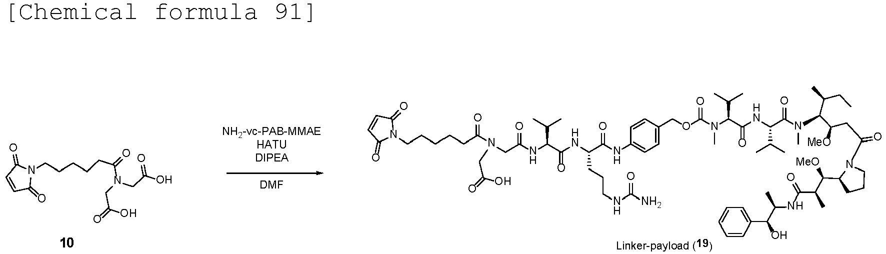

Maleimide (10) (8 mg, 0.0245 mmol) was dissolved in dimethylformamide (0.2 mL), and 1-[bis(dimethylamino) methylene]-1H-1,2,3,-triazolo[4,5-b]pyridinium 3-oxide hexafluorophosphate (4.7 mg, 0.0123 mmol) and N,N-diisopropylethylamine (0.0085 mL, 0.0491 mmol) were added thereto. The mixture was stirred at room temperature for five minutes. To the reaction mixture, known Val-Cit-PABA-MMAE (Organic & Biomolecular Chemistry, 2016, 14, 9501-9518) (13.8 mg, 0.0123 mmol) and N,N-diisopropylethylamine (0.0086 mL, 0.0491 mmol) were added, and the mixture was further stirred for two hours. After completion of the reaction, a 1 M aqueous hydrochloric acid solution was carefully added to the reaction solution to adjust the pH to 5 to 6. The reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Linker-payload (19) (12.1 mg, 0.064 mmol).
¹H NMR(500 MHz, DMSO-d6)δ9.98(brs, 0.63H), 9.77(brs, 0.43H), 8.40-8.27(m, 1H), 8.21(d, J=7.3Hz, 1H), 8.10-8.02(m, 1H), 7.88(d, J=8.8Hz, 1H), 7.63-7.57(m, 3H), 7.34-7.24(m, 6H), 7.21-7.13(m, 1H), 6.99(d, J=2.4Hz, 2H), 6.04-5.96(m, 1H), 5.75(s, 1H), 5.46-5.40(m, 3H), 5.33(d, J=4.9Hz, 1H), 5.13-4.93(m, 4H), 4.79-4.60(m, 3H), 4.53-4.35(m, 4H), 4.34-4.23(m, 3H), 4.21-3.88(m, 9H), 3.78(dd, J=9.5, 2.2Hz, 1H), 3.65-3.53(m, 3H), 3.51-3.43(m, 2H), 3.40-3.34(m, 2H), 3.28-3.16(m, 8H), 3.14-3.10(m, 2H), 3.08-2.81(m, 9H), 2.41(d, J=16.1Hz, 1H), 2.32-1.90(m, 11H), 1.87-1.64(m, 7H), 1.64-1.41(m, 11H), 1.40-1.13(m, 8H), 1.11-0.95(m, 6H), 0.95-0.65(m, 26H).
MS(ESI)m/z:1431.2[M+H]⁺

### (2-5) Synthesis of Linker-payload (20)

Linker-Payload (20) was synthesized as follows.

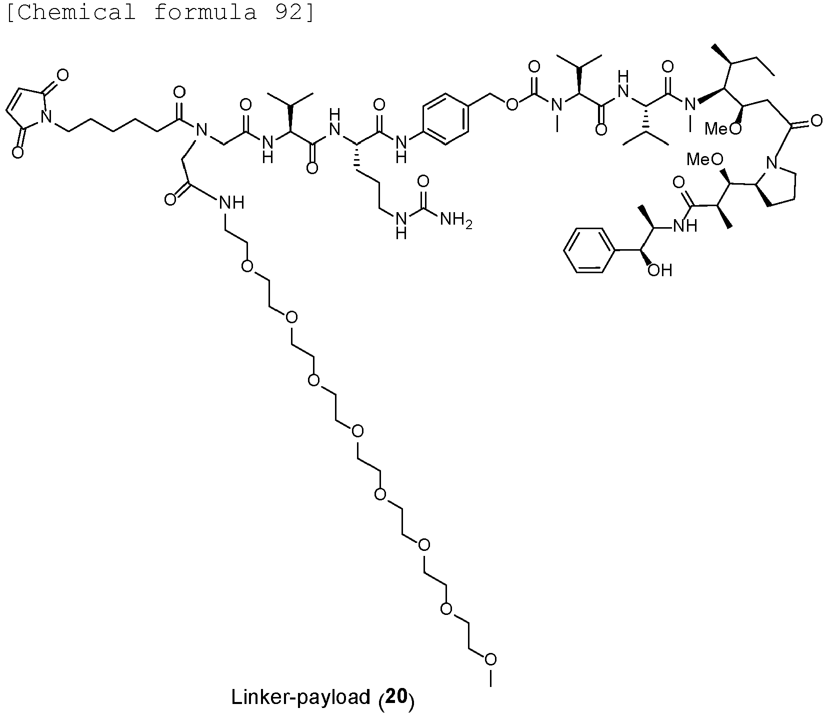

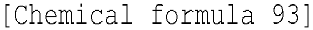

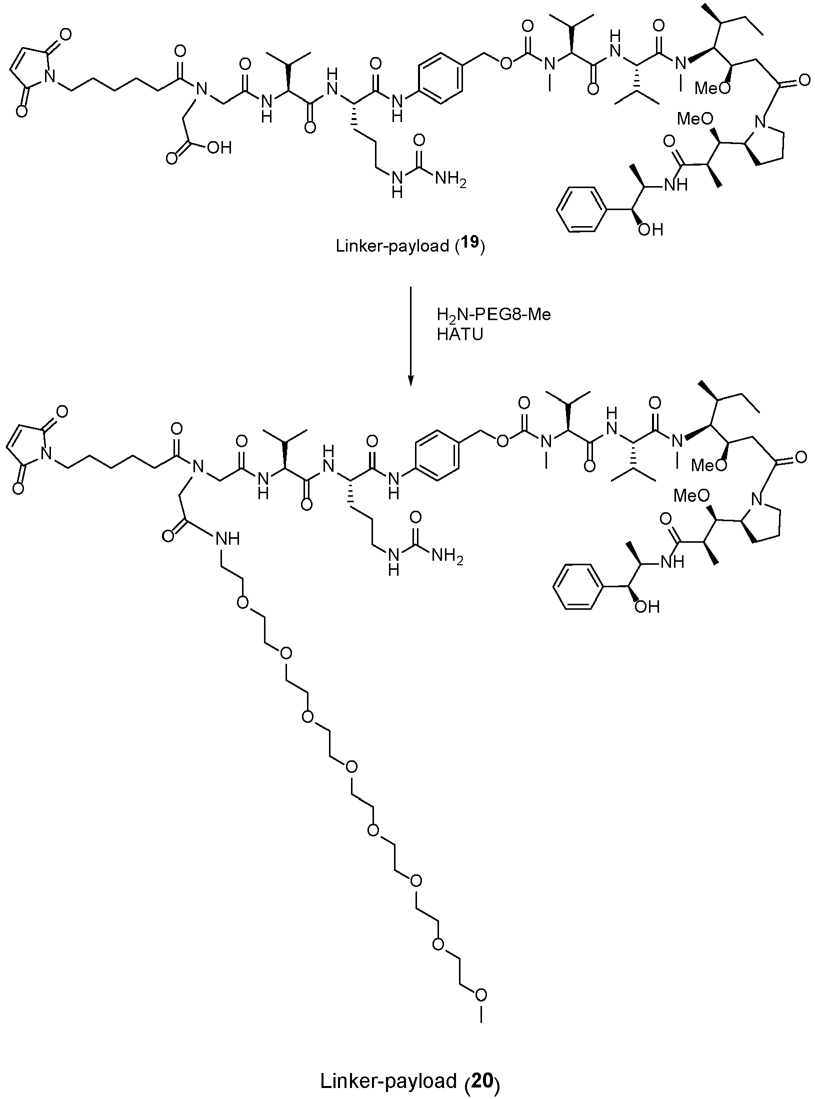

Linker-payload (19) (14.6 mg, 0.0102 mmol) was dissolved in dimethylformamide (0.12 mL), and 1-[bis(dimethylamino) methylene]-1H-1,2,3,-triazolo[4,5-b]pyridinium 3-oxide hexafluorophosphate (4.7 mg, 0.0122 mmol) and N,N-diisopropylethylamine (0.0035 mL, 0.0204 mmol) were added thereto. The mixture was stirred at room temperature for five minutes. To the reaction mixture, methoxy-PEG8-amine (4.7 mg, 0.0122 mmol) and N,N-diisopropylethylamine (0.0035 mL, 0.0204 mmol) were added, and the mixture was further stirred for 10 minutes. After completion of the reaction, a 1 M aqueous hydrochloric acid solution was carefully added to the reaction solution to adjust the pH to 5 to 6. The reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain Linker-payload (20) (12.9 mg, 0.072 mmol).
MS(ESI)m/z:1796.9[M+H]⁺

### (2-6) Synthesis of Linker-payload (21)

Linker-Payload (21) was synthesized as follows.

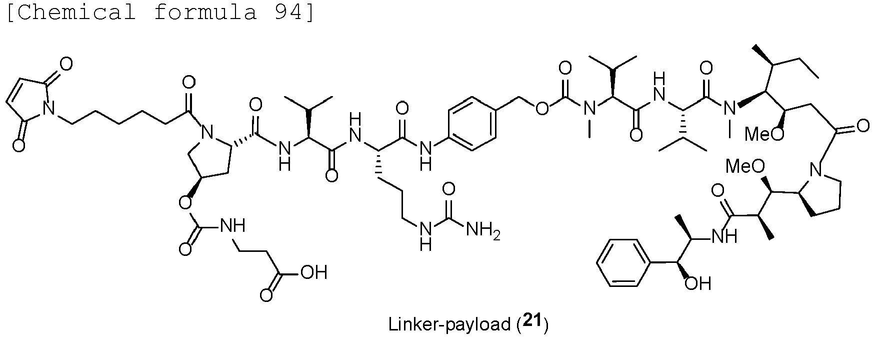

Linker-Payload (21) was synthesized according to the following scheme.

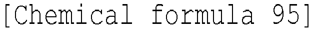

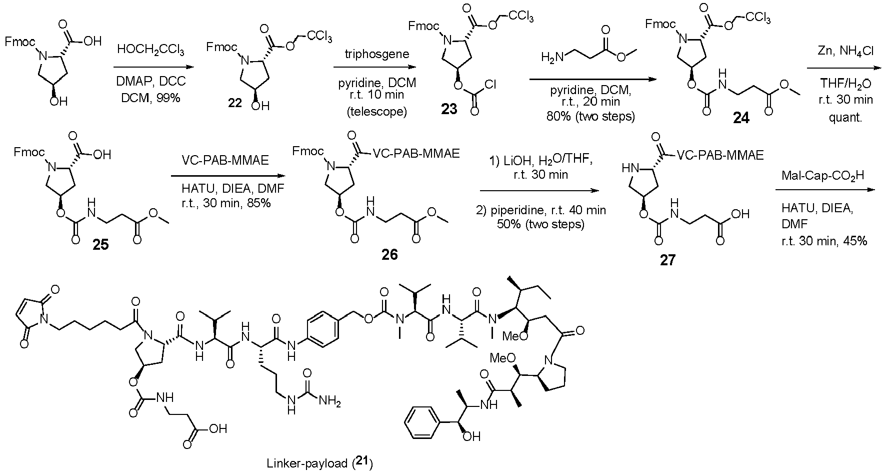

MS analysis results of Linker-Payload (21) were as follows.
MS(ESI)m/z:1544.8[M+H]⁺

### (2-7) Synthesis of Linker-payload (32)

Linker-payload (32) was synthesized as follows.

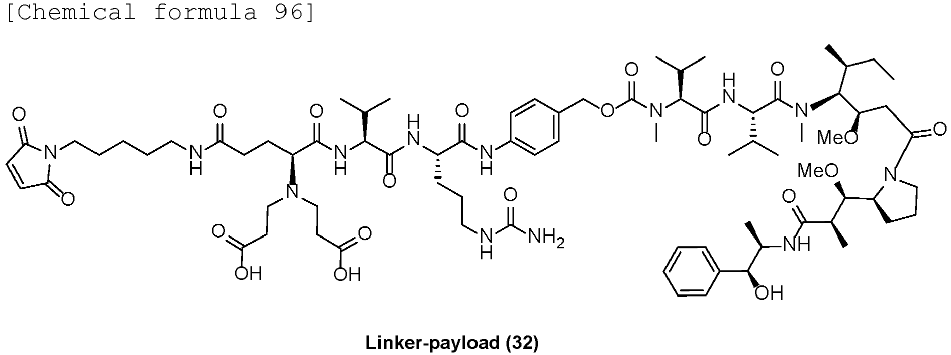

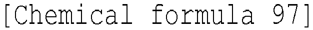

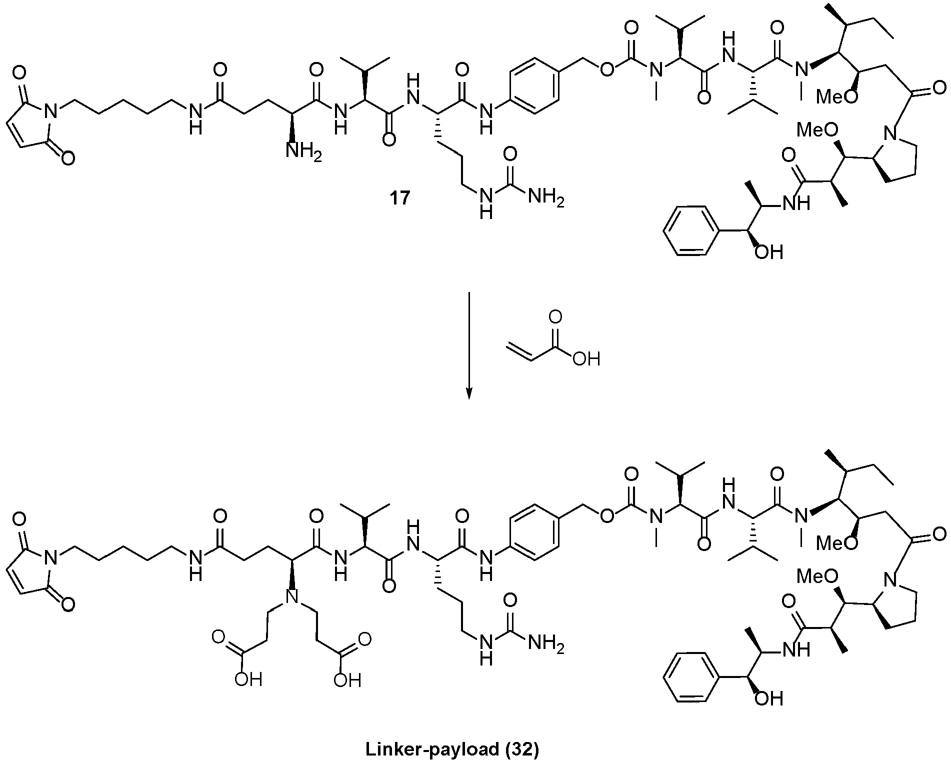

Compound (17) (15 mg, 0.011 mmol) was dissolved in aclyrate (1.5mL), and the mixture was stirred at 40°C for 24 hours. The reaction solution was diluted with a 1 : 1 = water : acetonitrile solution (comprising 0.05 v/v% formic acid), and purified by reverse phase preparative chromatography. A fraction comprising a product was collected and concentrated under reduced pressure to remove acetonitrile. The residue was freeze-dried to obtain the Linker-payload (32) (2.5 mg, 0.0016 mmol).
¹H NMR(500 MHz,DMSO-d6)δ10.58(brs,2H),9.59(brs,1H),8.99-9.07(m,1H),7.98-8.04(m,1H),7.85-7.97(m,2H),7.72-7.78(m,1H),7.59-7.68(m,2H),7.22-7.36(m,8H),7.11-7.20(m,1H),6.99(s,2H),6.80-6.93(m,1H), 5.49-5.89(m,4H),4.91-5.14(m,2H),4.69-4.78(m,1H),4.57-4.67(m,1H),4.35-4.53(m,3H),4.18-4.31(m,2H),3.89-4.08(m,2H),3.74-3.81(m,1H),3.51-3.66(m,1H),3.34-3.39(m,3H),3.25-3.11(m,10H),2.91-3.07(m,5H),2.84-2.90(m,3H),2.65-2.83(m,4H),2.37-2.43(m,5H),2.22-2.33(m,1H),2.09-2.20(m,4H),1.96-2.09(m,2H),1.83-1.96(m,2H),1.69-1.83(m,2H),1.59-1.68(m,1H),1.53-1.58(m,1H),1.42-1.52(m,4H),1.26-1.40(m,3H),1.14-1.25(m,2H),0.94-1.06(m,6H),0.71-0.89(m,24H)
MS(ESI)m/z:1560.3[M+H]⁺

### (2-8) Synthesis of Linker-payload (33)

Linker-payload (33) was synthesized as follows.

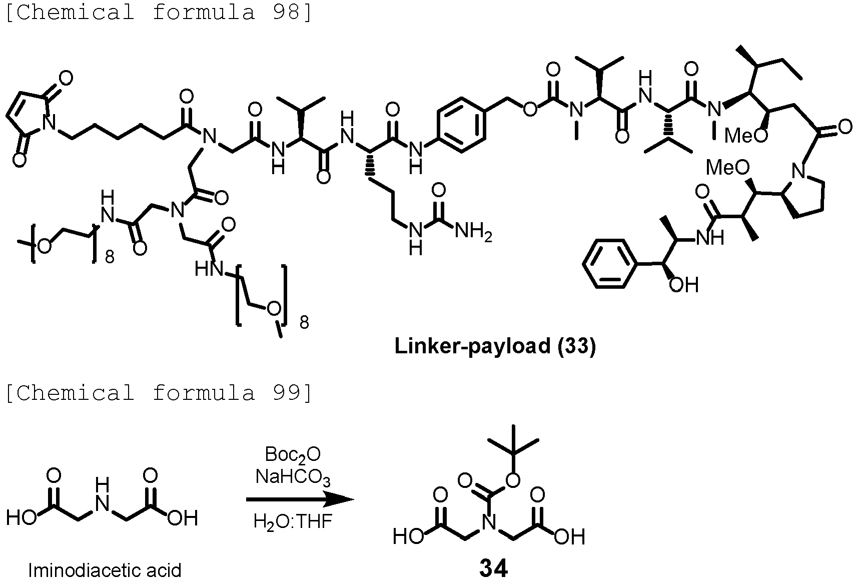

Iminodiacetic acid (212 mg, 1.59 mmol) and sodium bicarbonate (534 mg, 6.36 mmol) were dissolved in water (4mL). After foaming had ceased, THF (2mL) and Boc2O (416 mg, 1.91 mmol) were added thereto. The mixture was stirred at room temperature for 72 hours. The reaction solution was concentrated under reduced pressure to remove THF, and adjusted to about pH1 with 1N hydrochloric acid. The aqueous layer was extracted with ethylacetate (15 mL × 3), the organic layer was dried over magnesium sulfate, and then the filtered organic layer was concentrated under reduced pressure to obtain compound (34) (205 mg,0.877 mmol) .
MS(ESI)m/z:256.0[M+Na]⁺

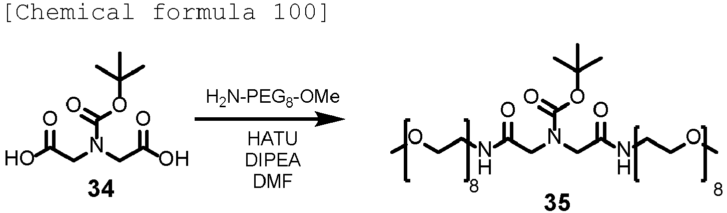

Compound (34) (14.6 mg, 0.0625 mmol), HATU (26.2mg, 0.0689mmol), DIPEA (21.8µL,0.125 mmol) were dissolved in DMF (1.5 ml). The mixture was stirred at room temperature for 5 minutes and obtain a reaction solution. A solution dissolving H2N-PEG8-OMe (52.7 mg, 0.138 mmol) and DIPEA (21.8 µL, 0.125 mmol) in DMF (1 mL) was added to the above reaction solution, followed by stirring at room temperature for 1 hour. Further, HATU (26.2 mg, 0.0689 mmol), and DIPEA (21.8 µL, 0.125 mmol) were added to the reaction solution, followed by stirring at room temperature for 2 hours. The reaction solution was concentrated under reduce pressure, and diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload the compound (35) (33.2mg, 0.0344 mmol) .
MS(ESI)m/z:964.4[M+H]⁺

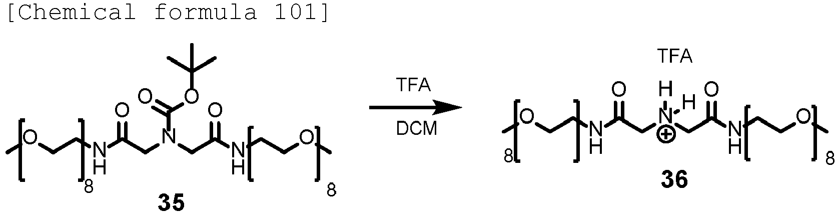

Compound (35) (33.2mg,0.0344 mmol) was dissolved in dichloromethane (2mL), then trifluoroacetic acid (1mL) was added thereto. The mixture were stirred at room temperature overnight and concentrated under reduced pressure to obtain Compound (36) (33.6 mg, quant).
MS(ESI)m/z:864.2[M+H]⁺

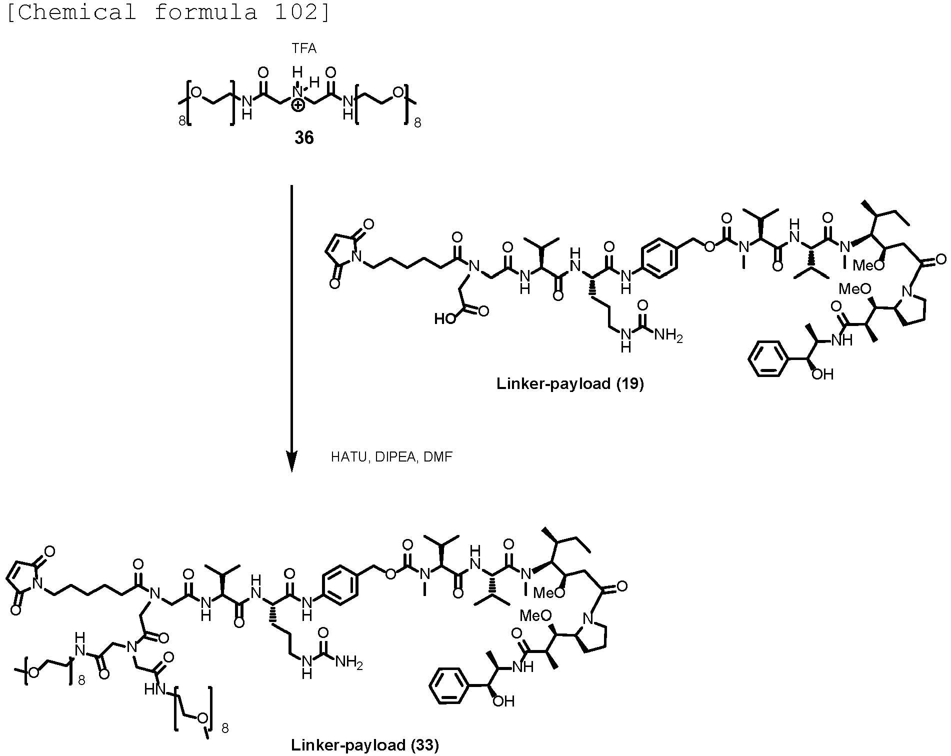

Linker-payload (19) (6.6 mg, 0.0046 mmol), HATU (4.4 mg, 0.0046 mmol), DIPEA (1.6 µL,0.0092 mmol) were dissolved in DMF (0.15 ml). The mixture was stirred at room temperature for 5 minutes. A solution dissolving Compound (36) (5.0 mg, 0.0058 mmol) and DIPEA (1.6 µL, 0.0058 mmol) in DMF(0.15 mL) was added to the above reaction solution, and the mixture was stirred at room temperature for 3 hours. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload(33)(2.0 mg, 0.00088 mmol) .
MS(ESI)m/z:2277.8[M+H]⁺

### (2-9) Synthesis of Linker-payload (37)

Linker-Payload(37) was synthesized as follows.

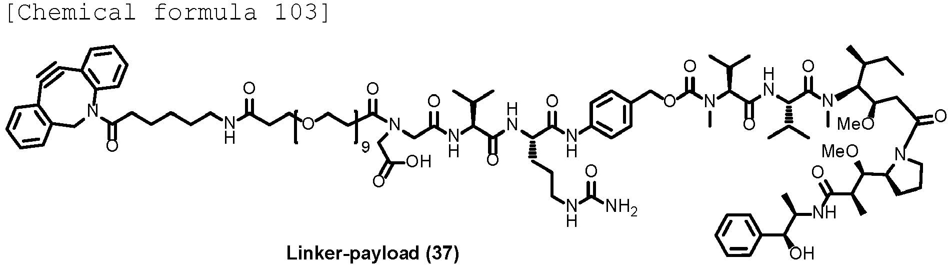

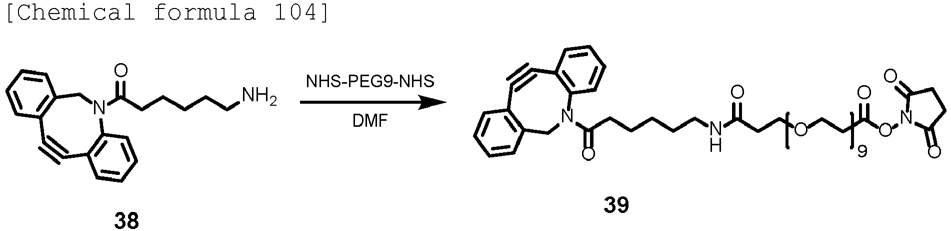

NHS-PEG9-NHS (164 mg, 0.231 mmol) was dissolved in DMF (1 mL) to obtain NHS-PEG9-NHS solution. In another flask, Compound (38) (35.5 mg, 0.112 mmol) was dissolved in DMF (1.5 mL) to obtain a solution. The solution was added to the NHS-PEG9-NHS solution described above to obtain the reaction solution. The reaction solution was stirred at room temperature for 30 min, diluted with water (8 mL), and purified by reverse phase preparative chromatography. A fraction comprising the product was collected and concentrated under reduced pressure to remove acetonitrile. The residue was freeze-dried to obtain Compound (39) (56.8 mg, 0.0623 mmol).
MS(ESI)m/z:912.2[M+H]⁺

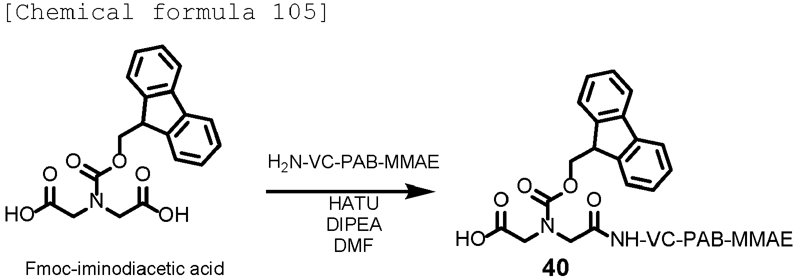

Fmoc-iminodiacetic acid (13.6 mg, 0.0382 mmol), HATU (14.5 mg, 0.0382 mmol), and DIPEA (10.0 µL, 0.057 mmol) were dissolved in DMF (0.75 ml) and stirred at room temperature for 5 minutes to obtain Fmoc-iminodiacetic acid solution. In another flask, H2N-VC-PAB-MMAE (42.9 mg,0.0382 mmol) and DIPEA (10.0 µL,0.057 mmol) were dissolved in DMF (0.75 mL) to obtain a solution. The solution was added to the Fmoc-iminodiacetic acid solution described above to obtain the reaction solution. The reaction solution was stirred at room temperature for 18 h, diluted with a 1 : 1 = water : acetonitrile solution, and purified by reverse phase preparative chromatography.
A fraction comprising the product was collected and concentrated under reduced pressure to remove acetonitrile. The residue was freeze-dried to obtain Compound (40) (21.2 mg, 0.0143 mmol).
MS(ESI)m/z:1460.4[M+H]⁺

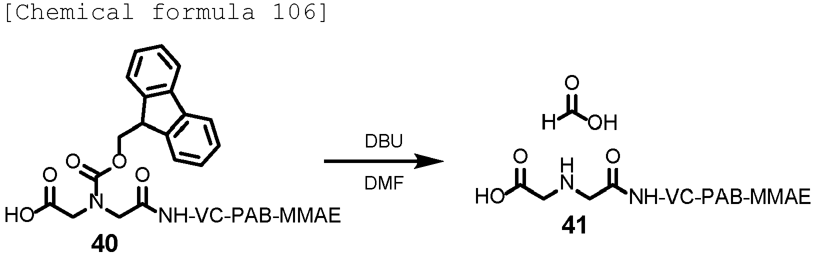

Compound (40)(100 mg, 0.0695 mmol) was dissolved in DMF (2.5 mL), and DBU (31 µL, 0.205 mmol) was added thereto. The mixture was stirred at room temperature for 5 minutes to obtain a reaction solution. The reaction solution was diluted with a 1 : 1 = water : acetonitrile solution (containing 0.05 % formic acid), and purified by reverse phase preparative chromatography. A fraction comprising the product was collected and concentrated under reduced pressure to remove acetonitrile. The residue was freeze-dried to obtain Compound (41) (86.6 mg, 0.0674 mmol) .
MS(ESI)m/z:1238.4[M+H]⁺

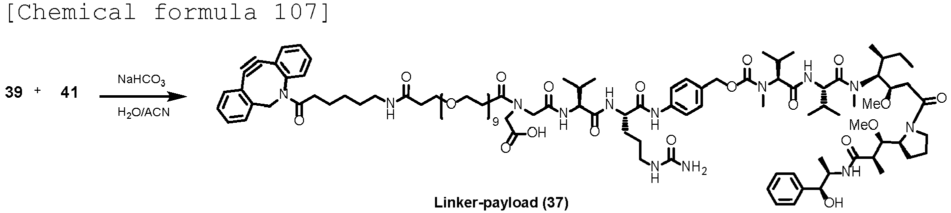

Compound (39) (22.9 mg, 0.0185 mmol) and Compound (41) (33.7 mg, 0.0178 mmol) were dissolved in 1 : 1 = water : acetonitrile solution (2 mL), and sodium bicarbonate (1.9 mg,0.0222 mmol) was added thereto. The mixture was stirred at room temperature for 23 hours to obtain a reaction solution. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload (37) (7.3 mg, 0.0036 mmol).
MS(ESI)m/z:2035.5[M+H]⁺

### (2-10) Synthesis of Linker-payload (42)

Linker-Payload (42) was synthesized as follows.

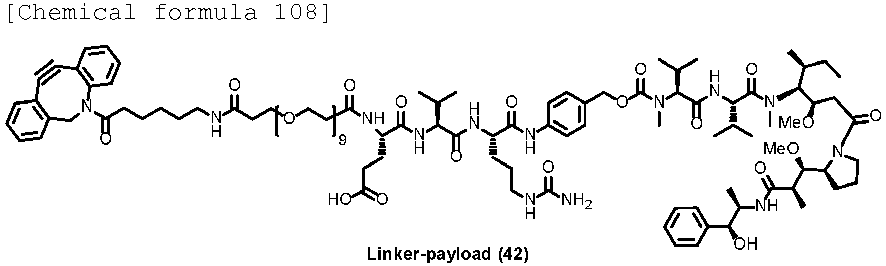

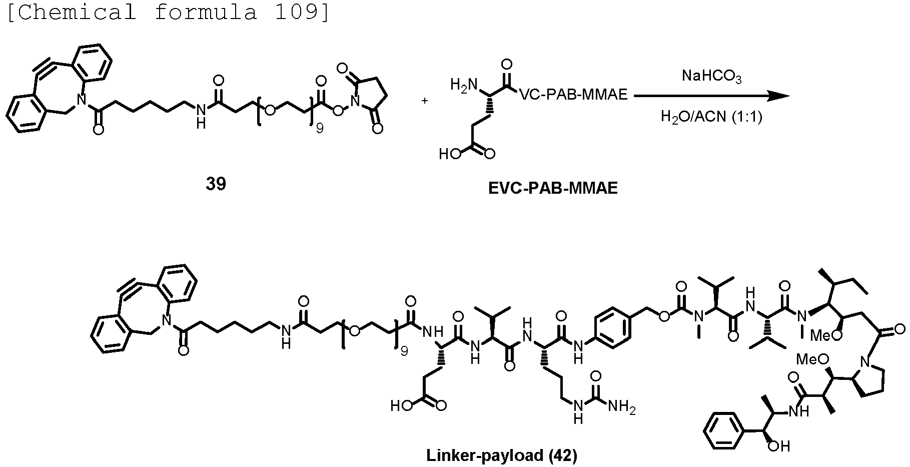

Compound (39) (23.4 mg,0.0257 mmol), and EVC-PAB-MMAE (21.4 mg, 0.0171 mmol) were dissolved in 1:1 = water : acetonitrile solution (3mL), and sodium bicarbonate (10 mg, 0.119 mmol) was added thereto to obtain a reaction solution. The reaction solution was stirred at room temperature for 1 hour, adjusted to about pH4 with 1N hydrochloric acid, diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload (42) (16.4 mg, 0.008 mmol).
MS(ESI)m/z:2049.8[M+H]⁺

### (2-11) Synthesis of Linker-payload (43)

Linker-Payload (43) was synthesized as follows.

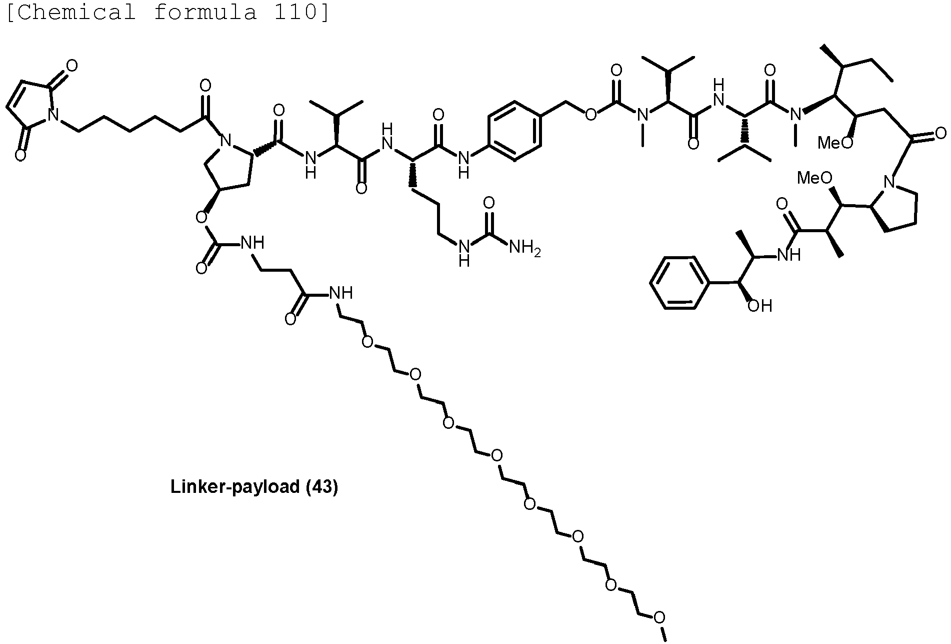

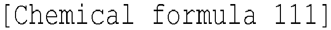

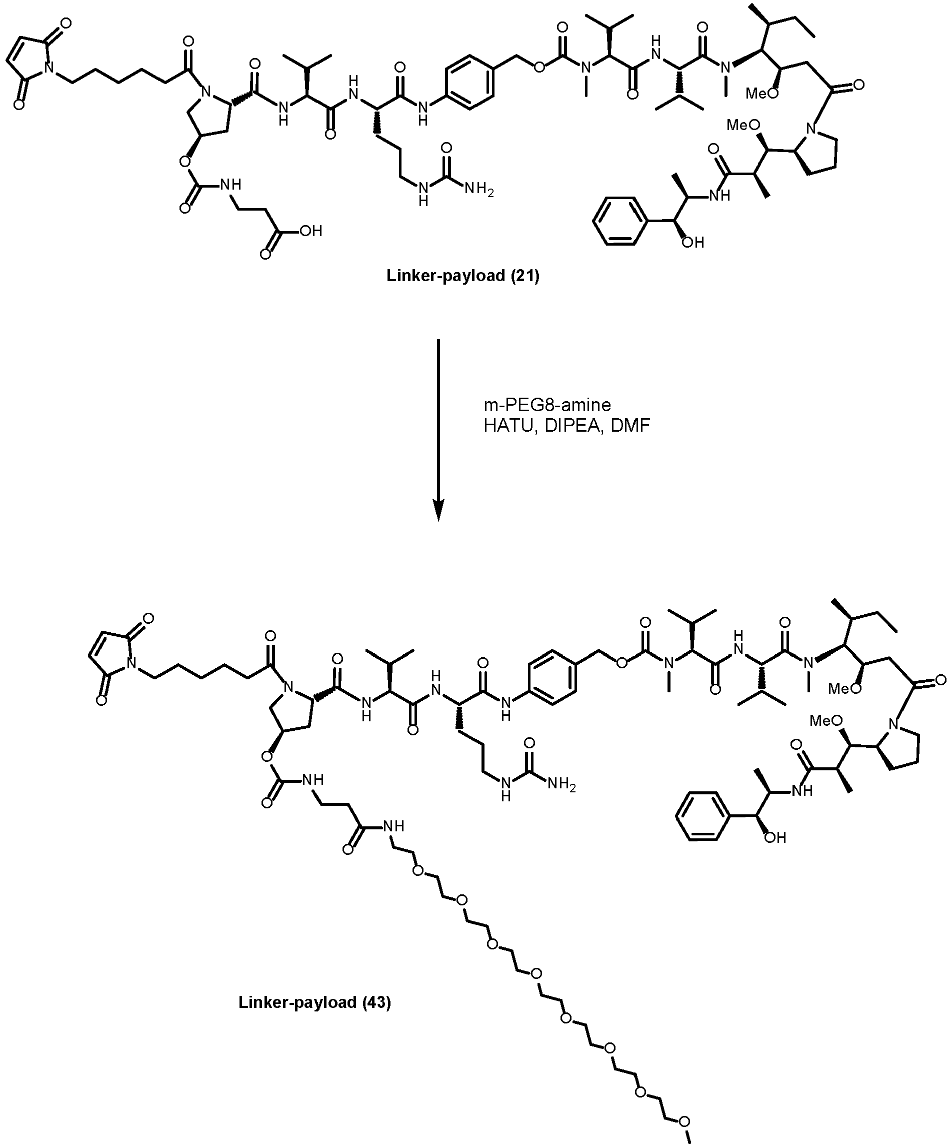

Linker-payload (21) (15.6 mg, 0.074 mmol), HATU (5.4 mg, 0.014 mmol), and DIPEA (4.5 µL, 0.026 mmol) were dissolved in DMF (0.5 mL) and stirred at room temperature for 1 min to obtain a reaction solution. m-PEG8-amine(BroadPharm #BP-21111)(7.5mg,0.0194mmol) was added to the reaction solution, stirred at room temperature for 20 minutes, diluted with a 1 : 1 = water : acetonitrile solution (containing 0.05% formic acid), and purified by reverse phase preparative chromatography. A fraction comprising the product was collected and concentrated under reduced pressure to remove acetonitrile. The residue was freeze-dried to obtain Linker-payload (43) (5.2 mg, 0.0027 mmol) .
MS(ESI)m/z:955.2[M+2H]²⁺

### (1-4) Synthesis of Linker-payload mimic (44)

Linker-Payload mimic (44) was synthesized as follows.

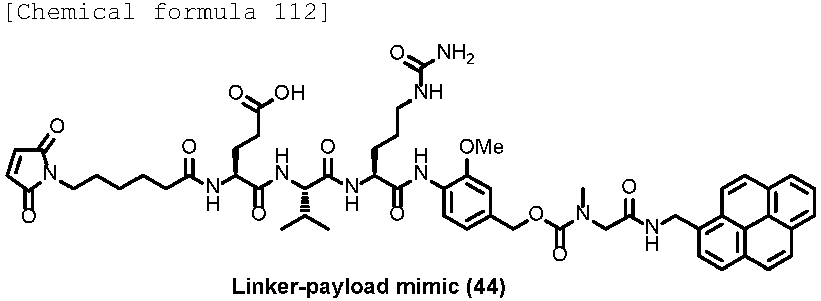

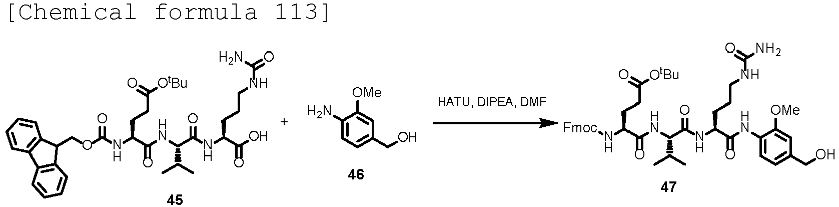

Compound (45) (20.2mg, 0.0293 mmol), HATU (13.4 mg, 0.0352 mmol), and DIPEA (6.0µL, 0.035mmol) were dissolved in DMF (0.3 mL) and stirred at room temperature for 1 min. Compound (46) (5.4 mg, 0.035 mmol) was added thereto. The mixture was stirred at room temperature for 17 h to obtain a reaction solutuon. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Compound (47) (23.9 mg, quant).
MS(ESI)m/z:817.4[M+H]⁺

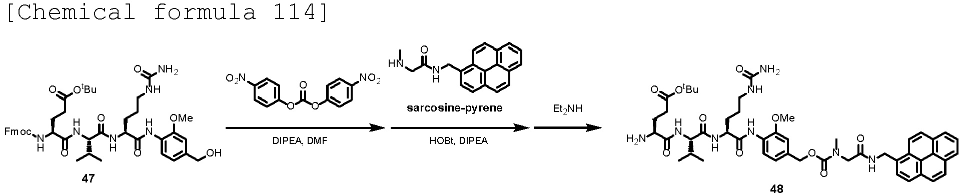

Compound (47) (23.9 mg, 0.0293 mmol) was dissolved in DMF (0.45 mL, and bis (4-nitrophenyl) carbonate (27.3 mg, 0.0879 mmol) and DIPEA (11.4 µL, 0.0657 mmol were added thereto. The mixture was stirred at room temperature for 3 hours to obtain a reaction solution. Sarucosine-pyrene (44.3 mg, 0.147 mmol), HOBt(5.9 mg, 0.044 mmol), and DIPEA (39.4 µL, 0.227 mmol) were added to the reaction solution, followed by stirring at room temperature for 20 h. Then, diethylamine (93.0 µL, 0.882 mmol) was added to the reaction solution, and stirred for 3 hours at room temperature, concentrated under reduced pressure. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Compound (48) (1.9 mg, 0.0021 mmol).
MS(ESI)m/z:923.5[M+H]⁺

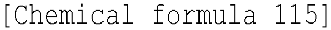

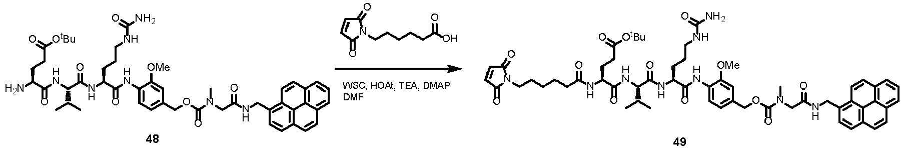

Compound (48) (1.9 mg, 0.0021 mmol) was dissolved in DMF (0.4 mL), 4-maleimidohexanoic acid (0.7 mg,0.003 mmol), HOAt (0.4 mg, 0.003 mmol), WSC·HCl (0.9 mg, 0.005 mmol), triethylamine (0.89 µL, 0.0063 mmol), and DMAP (0.1 mg, 0.001 mmol) are added thereto. The mixture was stirring at room temperature for 5 h to obtain a reaction solution. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Compound (49) (0.3 mg, 0.0003 mmol).
MS(ESI)m/z:1116.5[M+H]⁺

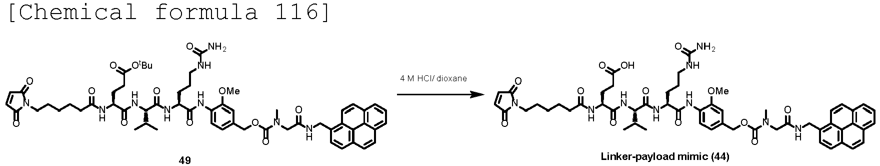

Compound (49) (0.3mg, 0.0003 mmol) was dissolved in 1,4-dioxane (0.3 mL), and then 4N hydrochloride dioxane (315 µL, 1.26 mmol) was added thereto. The mixture was stirred at room temperature for 5 hours to obtain a reaction solutuiono. The reaction solution was cooled to 0°C, added DIPEA (238 µL, 1.39 mmol), and then stirred at room temperature for 1 minute, concentrated, and diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload (44) (0.3 mg, 0.0003 mmol) .
MS(ESI)m/z:1060.5[M+H]⁺

### (1-5) Synthesis of Linker-payload mimic (50)

Linker-Payload mimic (50) was synthesized as follows.

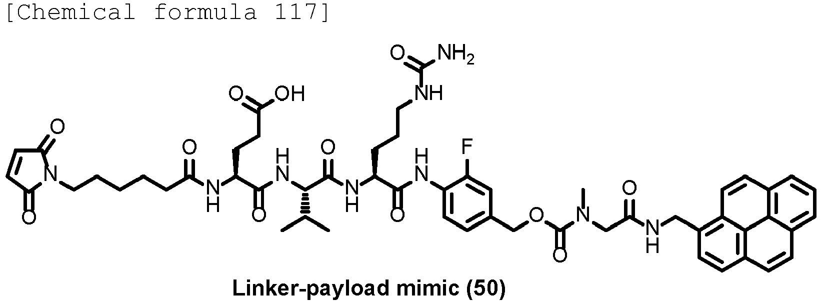

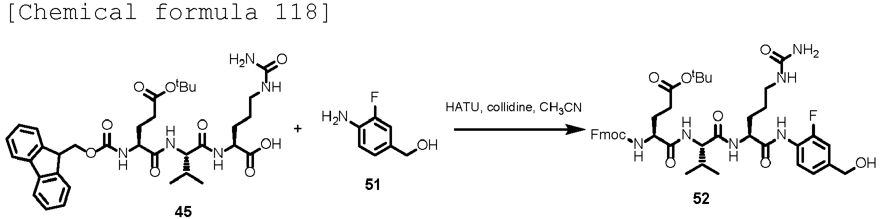

Compound (45) (24.8 mg, 0.0367 mmol), HATU (16.7 mg, 0.0440 mmol) and collidine (5.8 µL, 0.044 mmol) were suspended in acetonitrile (0.5mL). The suspension was stirred at room temperature for 1 min, and Compound (51) (6.2 mg, 0.044 mmol) was added thereto. The mixture was stirred at room temperature for 24 h to obtain a reaction solution. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Compound (52) (11.4 mg, 0.0142 mmol).
MS(ESI)m/z:805.4[M+H]⁺

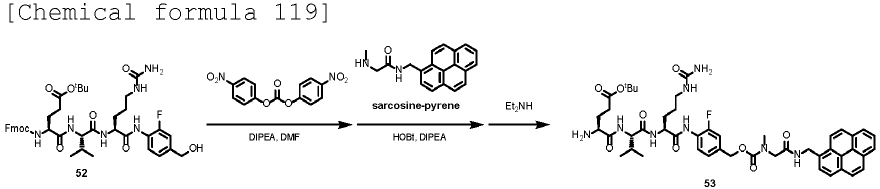

Compound (52) (11.4 mg, 0.0142 mmol) was dissolved in DMF (0.3 mL), and bis (4-nitrophenyl)carbonate (12.6 mg, 0.0426 mmol, and DIPEA (5.4 µL, 0.0318 mmol were added thereto. The mixture was stirred at room temperature for 4 hours.

Further, bis (4-nitrophenyl) carbonate (6.5 mg,0.0213 mmol), DIPEA (2.7 µL,0.016 mmol) was added to the mixture. The mixture was stirred at room temperature for 3 hours to obtain a reaction solution. Sarucosine-pyrene (42.9 mg, 0.142 mmol), HOBt (2.9 mg, 0.021 mmol), and DIPEA(38.4 µL, 0.226 mmol) were added to the reaction solution, followed by stirring at room temperature for 16 h. Diethylamine (29.7 µL, 0.284 mmol) was added to the reaction solution. The reaction solution was stirred for 3 hours at room temperature, and concentrated under reduced pressure. The reaction solution was diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Compound (53) (10.0 mg, 0.0110 mmol).
MS(ESI)m/z:911.4[M+H]⁺

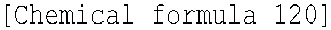

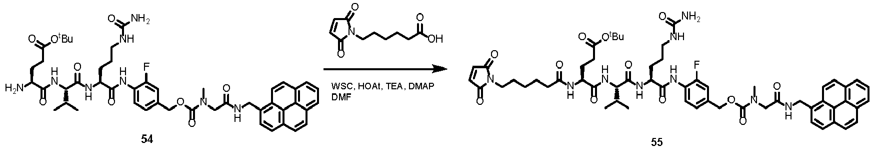

Compound (54) (10.0 mg, 0.0110 mmol) was dissolved in DMF (1 mL), and then 4-maleimidohexanoic acid (3.5 mg, 0.017 mmol), HOAt (2.2 mg, 0.017 mmol), WSC·HCl (4.8 mg,0.025 mmol), triethylamine (4.6 µL, 0.033 mmol), and DMAP (0.3 mg,0.002 mmol) was added thereto. The mixture was stirred at room temperature for 4 hours to obtain a reaction solution. The reaction solution was diluted with a 1 : 1 = water : acetonitrile solution, and purified by reverse phase preparative chromatography.
A fraction comprising the product was collected and concentrated under reduced pressure to remove acetonitrile. The residue was freeze-dried to obtain Compound (55) (1.6 mg, 0.0014 mmol).
MS(ESI)m/z:1104.5[M+H]⁺

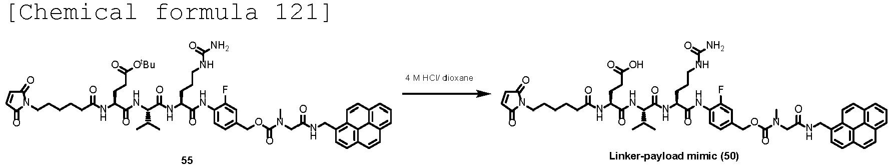

Compound (55) (1.6 mg, 0.0014 mmol) was suspended in 1,4-dioxane (0.2mL), and then 4N hydrochloric acid (181 µL, 724 mmol) was added thereto. The mixture was stirred at room temperature for 5 hours to obtain a reaction solution. The reaction solution was cooled to 0°C, and DIPEA (136 µL, 798 mmol) was added thereto, followed by stirring at room temperature for 1 minute, concentrated, and then, diluted with a 1 : 1 = water : acetonitrile solution, and purified by reverse phase preparative chromatography. A fraction comprising the product was collected and concentrated under reduced pressure to remove acetonitrile. The residue was freeze-dried to obtain Linker-payload (50) (0.81 mg, 0.00077 mmol).
MS(ESI)m/z:1048.5[M+H]⁺

### COMPARATIVE EXAMPLE 1: Synthesis of Linker-payload mimic (28)

Maleimide-VC-Pyrene (28) was synthesized as follows. Maleimide-VC-Pyrene (28) was synthesized in one step from commercially available MC-VC-PAB-PNP (CAS No: 159857-81-5) and known Sarcosine-pyridine (WO 2018218004 A1).

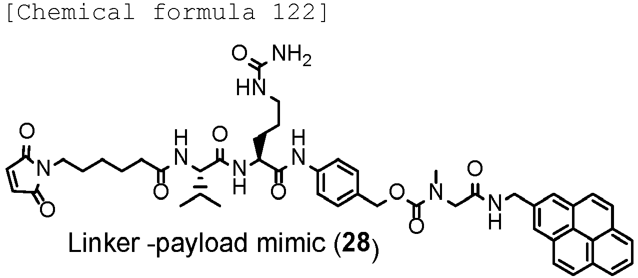

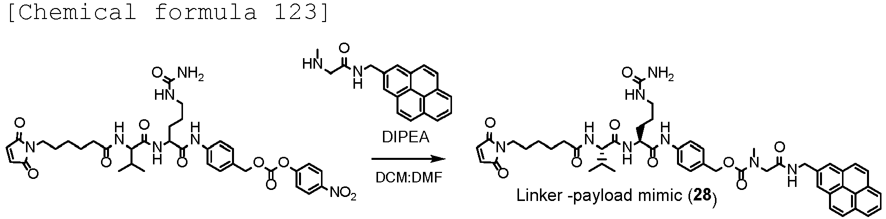

Commercially available MC-VC-PAB-PNP (CAS No: 159857-81-5) (15.5 mg, 0.021 mmol) was dissolved in dichloromethane (1 mL), and a dimethylformamide solution (0.5 mL) of N,N-diisopropylethylamine (0.025 mL, 0.142 mmol) and known Sarcosine-pyrene (WO 2018218004 A1) (7.6 mg, 0.025 mmol) was added thereto. The mixture was stirred for 17 hours. After purification by reverse phase preparative chromatography, a fraction comprising a product was collected and concentrated under reduced pressure to remove acetonitrile. The residue was freeze-dried to obtain Linker-payload mimic (28) (7.3 mg, 0.008 mmol).
¹H NMR(400 MHz, DMSO-d6)δ9.98(s, 1H), 8.34(d, J=9.2Hz, 2H), 8.32-8.23(m, 4H), 8.16(s, 2H), 8.10-8.00(m, 4H), 7.80(d, J=8.8Hz, 1H), 7.59(d, J=8.4Hz, 2H), 7.31(d, J=8.0Hz, 2H), 6.99(s, 2H), 5.96(m, 1H), 5.40(s, 2H), 5.01(s, 2H), 4.95(d, J=6.0Hz, 2H), 4.38(m, 1H), 4.19(m, 1H), 3.03-2.92(m, 3H), 2.67(m, 1H), 2.33(m, 1H), 2.20-2.07(m, 2H), 1.97(m, 1H), 1.67(m, 1H), 1.59(m, 1H), 1.51-1.45(m, 6H), 1.26-1.15(m, 3H), 0.83(dd, J=12.8, 6.8Hz, 6H)
MS(ESI)m/z:901.45[M+H]⁺

### COMPARATIVE EXAMPLE 2: Synthesis of Linker-payload

### (2-1) Synthesis of Linker-Payload (29)

Linker-Payload (29) was synthesized as follows.

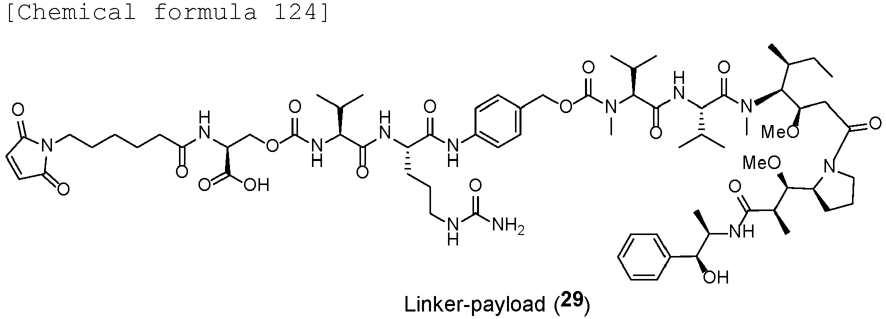

Linker-Payload (29) was synthesized according to the following scheme.

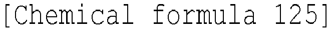

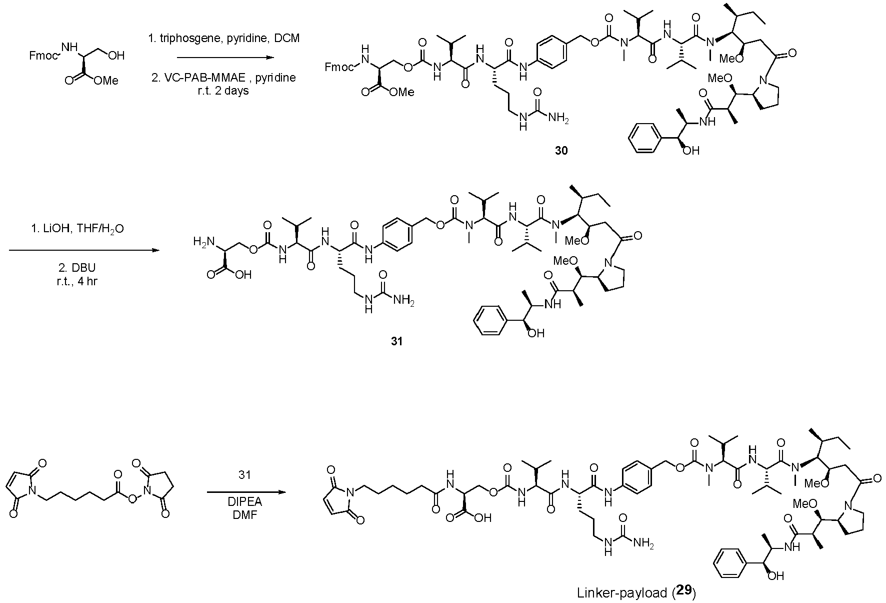

MS analysis results of Linker-Payload (29) were as follows.
MS(ESI)m/z:1447.8[M+H]⁺

### EXAMPLE 3: Synthesis of ADC mimic

### (3-1) Synthesis of ADC mimic

In the following Comparative Examples and Examples, an antibody derivative (thiol group-introduced trastuzumab) described in Example 81-7 of WO 2019/240287 A1 was used as a thiol group-introduced antibody. This antibody derivative has the following structure in which a thiol group is regioselectively introduced into trastuzumab (humanized IgG1 antibody) via an amino group of a side chain of a lysine residue at position 246 or 248 of an antibody heavy chain (the position of the lysine residue is in accordance with EU numbering). 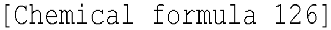 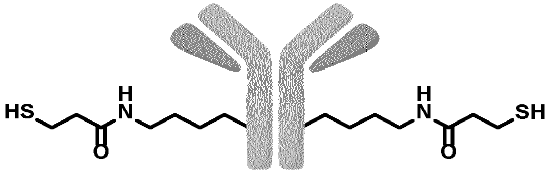 (In the above structure, NH-CH₂-CH₂-CH₂-CH₂- extending from the antibody heavy chain corresponds to a side chain of a lysine residue, and HS-CH₂-CH₂-C(=O) as a thiol-comprising group is added to an amino group in the side chain of the lysine residue.)

To a buffer (pH 7.4 PBS buffer) solution (20 µM) of the thiol group-introduced antibody, 10 equivalents of a DMF solution (1.25 mM) of Linker-payload mimic synthesized in Comparative Example 1 and Example 1 was added, and the mixture was allowed to stand at room temperature for two hours, and then purified using NAP-5 Columns (manufactured by GE Healthcare) to obtain an ADC mimic.

ADC mimic 1 having the following structure was synthesized from Linker-payload mimic (1) synthesized in Example 1-1 and the thiol-comprising antibody. ESI-TOFMS analysis was performed. For the reaction product, a peak was observed at 150641 indicating a product with two Linker-payload mimics (1) introduced.

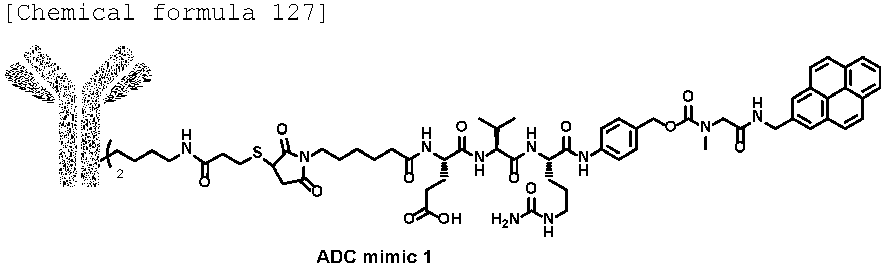

Similarly, ADC mimic 2 having the following structure was synthesized from Linker-payload mimic (5) of Example 1-2 and the thiol-comprising antibody. ESI-TOFMS analysis was performed. For the reaction product, a peak was observed at 150803 indicating a product with two Linker-payload mimics (5) introduced.

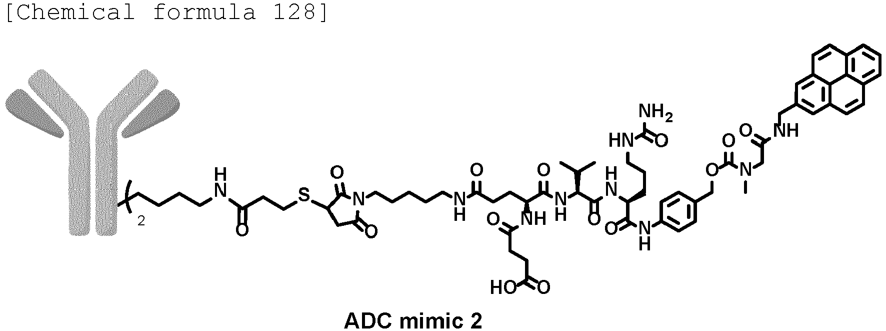

Similarly, ADC mimic 3 having the following structure was synthesized from Linker-payload mimic (9) of Example 1-3 and the thiol-comprising antibody. ESI-TOFMS analysis was performed. For the reaction product, a peak was observed at 150620 indicating a product with two Linker-payload mimics (9) introduced.

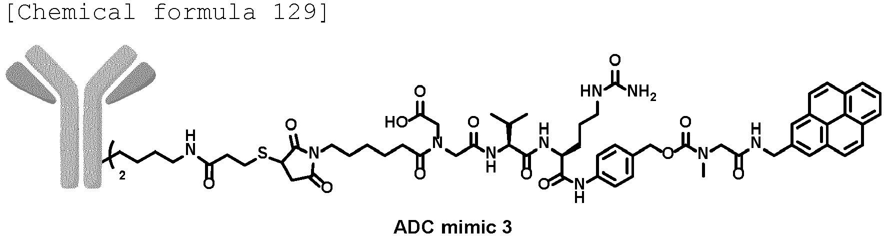

Similarly, ADC mimic 4 having the following structure was synthesized from Linker-payload mimic (28) of Comparative Example 1 and the thiol-comprising antibody. ESI-TOFMS analysis was performed. For the reaction product, a peak was observed at 150244 indicating a product with two Linker-payload mimics (28) introduced.

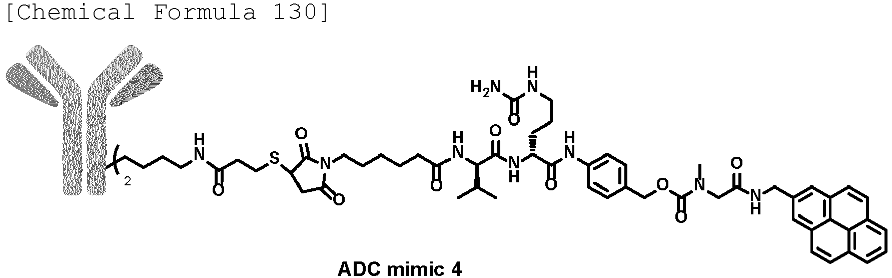

### (3-2) DAR analysis of ADC mimic

ADC mimic synthesized in Example 3-1 was subjected to ESI-TOFMS analysis according to a previous report (WO 2019/240287 A1) and confirmed to have a DAR of 2.

**Table 1. DAR of ADC mimic**

| | Linker-payload mimic | EXAMPLE/ COMPARATIVE EXAMPLE | DAR |
|---|---|---|---|
| ADC mimic 1 | Linker-payload mimic (1) | EXAMPLE 1-1 | 2 |
| ADC mimic 2 | Linker-payload mimic (5) | EXAMPLE 1-2 | 2 |
| ADC mimic 3 | Linker-payload mimic (9) | EXAMPLE 1-3 | 2 |
| ADC mimic 4 | Linker-payload mimic (28) | COMPARATIVE EXAMPLE 1 | 2 |

### EXAMPLE 4: Synthesis of ADC

### (4-1) Synthesis of ADC

To a buffer (pH 7.4 PBS buffer) solution (20 µM) of the thiol group-introduced antibody, 10 equivalents of a DMF solution (1.25 mM) of Linker-payload mimic synthesized in Comparative Example 2 and Example 1 was added, and the mixture was allowed to stand at room temperature for two hours, and then purified using NAP-5 Columns (manufactured by GE Healthcare) to obtain an ADC.

ADC 1 having the following structure was synthesized from Linker-payload (11) synthesized in Example 2-1 and the thiol-comprising antibody. ESI-TOFMS analysis was performed. For the reaction product, a peak was observed at 151230 indicating a product with two Linker-payloads (11) introduced.

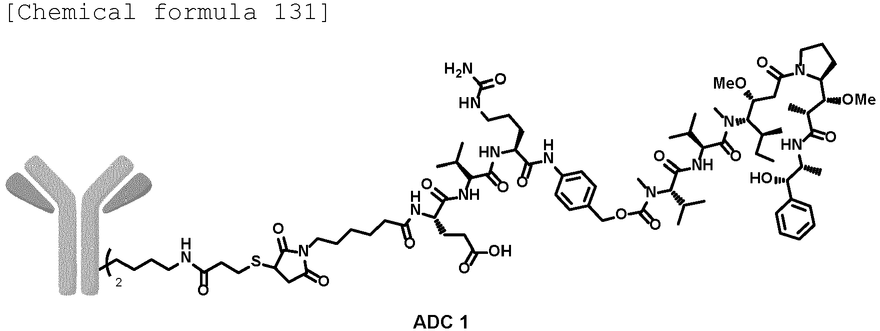

ADC 2 having the following structure was synthesized from Linker-payload (12) synthesized in Example 2-2 and the thiol-comprising antibody. ESI-TOFMS analysis was performed. For the reaction product, a peak was observed at 151443 indicating a product with two Linker-payloads (12) introduced.

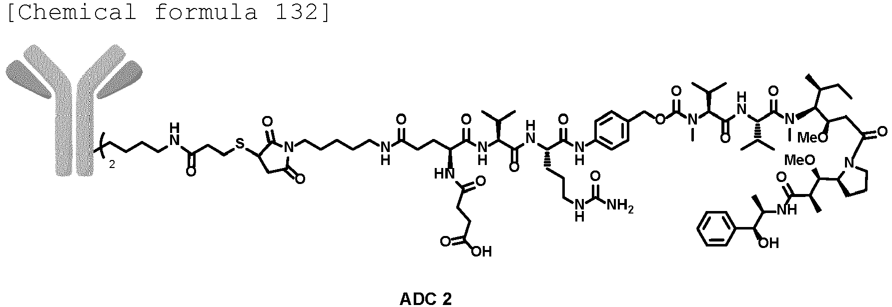

ADC 3 having the following structure was synthesized from Linker-payload (18) synthesized in Example 2-3 and the thiol-comprising antibody. ESI-TOFMS analysis was performed. For the reaction product, a peak was observed at 152173 indicating a product with two Linker-payloads (18) introduced.

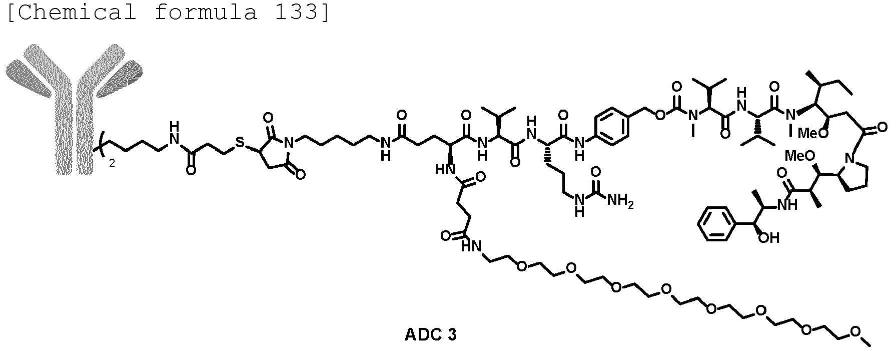

ADC 4 having the following structure was synthesized from Linker-payload (19) synthesized in Example 2-4 and the thiol-comprising antibody. ESI-TOFMS analysis was performed. For the reaction product, a peak was observed at 151270 indicating a product with two Linker-payloads (19) introduced.

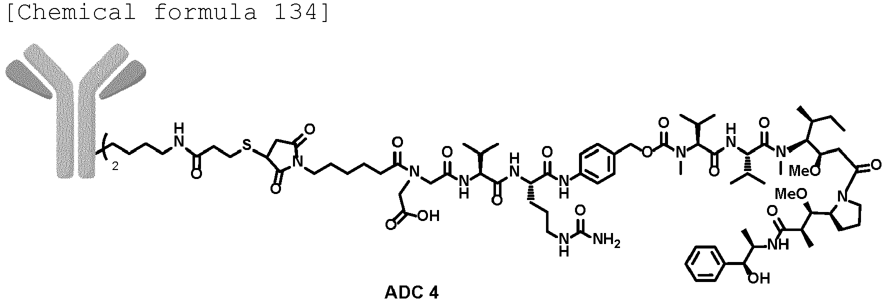

ADC 5 having the following structure was synthesized from Linker-payload (20) synthesized in Example 2-5 and the thiol-comprising antibody. ESI-TOFMS analysis was performed. For the reaction product, a peak was observed at 152005 indicating a product with two Linker-payloads (20) introduced.

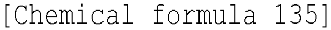

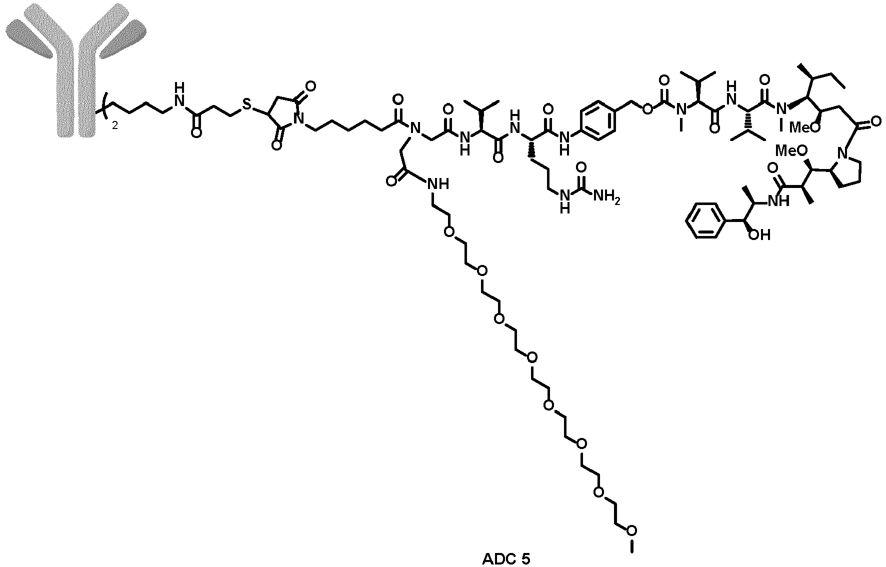

ADC 6 having the following structure was synthesized from Linker-payload (21) synthesized in Example 2-6 and the thiol-comprising antibody. ESI-TOFMS analysis was performed. For the reaction product, a peak was observed at 151492 indicating a product with two Linker-payloads (21) introduced.

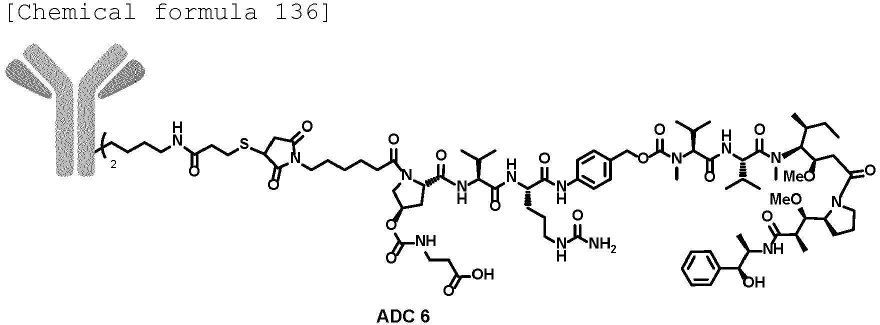

ADC 7 having the following structure was synthesized from Linker-payload (29) synthesized in Comparative Example 2 and the thiol-comprising antibody. ESI-TOFMS analysis was performed. For the reaction product, a peak was observed at 151295 indicating a product with two Linker-payloads (29) introduced.

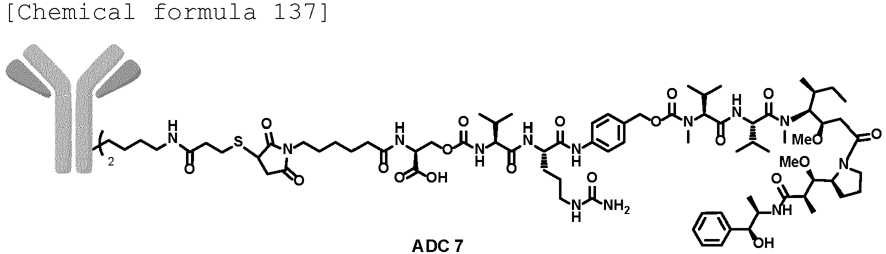

ADC 8 having the following structure was synthesized from commercially available MC-VC-MMAE (CAS no: 646502-53-6) and the thiol-comprising antibody. ESI-TOFMS analysis was performed. For the reaction product, a peak was observed at 151091 indicating a product with two MC-VC-MMAEs introduced.

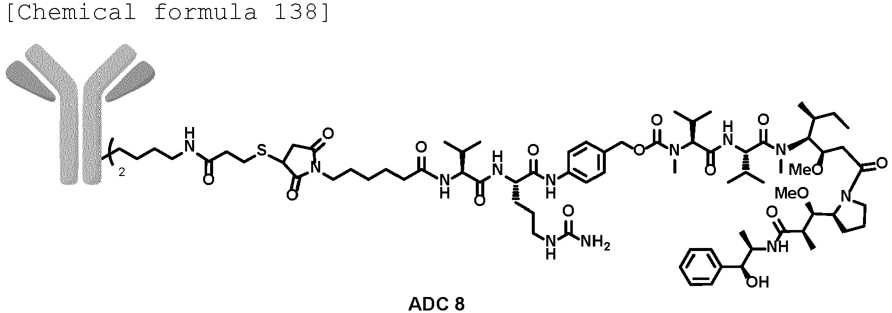

Similarly, ADC mimic 9 having the following structure was synthesized from Linker-payload (32) synthesized in Example 2-7 and the thiol-comprising antibody. ESI-TOFMS analysis was performed. For the reaction product, a peak was observed at 152261 indicating a product with two Linker-payloads (32) introduced.

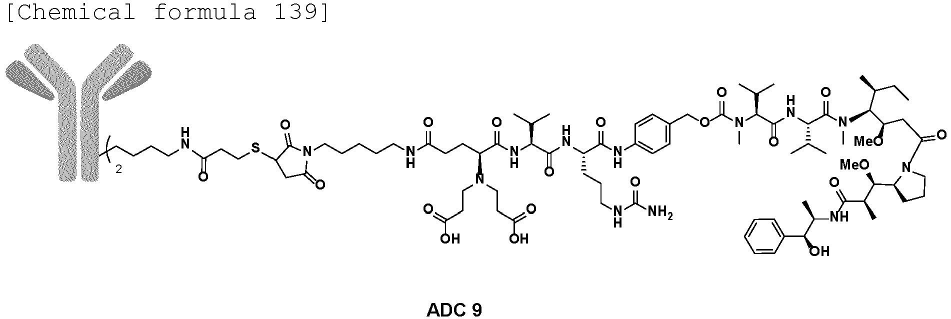

### (4-2) DAR analysis of ADC

The ADC synthesized in Example 4-1 was subjected to ESI-TOFMS analysis according to a previous report (WO 2019/240287 A1) and confirmed to have a DAR of 2.

**Table 2. List of synthesized ADCs and ADC mimics and DAR analysis results**

| | Linker-payload | EXAMPLE/ COMPARATIVE EXAMPLE | DAR |
|---|---|---|---|
| ADC 1 | Linker-payload (11) | EXAMPLE 2-1 | 2 |
| ADC 2 | Linker-payload (12) | EXAMPLE 2-2 | 2 |
| ADC 3 | Linker-payload (18) | EXAMPLE 2-3 | 2 |
| ADC 4 | Linker-payload (19) | EXAMPLE 2-4 | 2 |
| ADC 5 | Linker-payload (20) | EXAMPLE 2-5 | 2 |
| ADC 6 | Linker-payload (21) | EXAMPLE 2-6 | 2 |
| ADC 9 | Linker-payload (32) | EXAMPLE 2-7 | 2 |
| ADC 7 | Linker-payload (29) | COMPARATIVE EXAMPLE 2 | 2 |
| ADC 8 | MC-VC-MMAE | Commercial product (CAS no: 646502-53-6) | 2 |

### EXAMPLE 5: Evaluation of hydrophobicities of ADC and ADC mimic by hydrophobic column chromatography (HIC-HPLC)

According to a previous report (Anal. Chem., 2019, 91, 20, 12724-12732), HIC-HPLC analysis was performed. Measurement was performed using the following conditions. The hydrophobicity of an ADC can be evaluated by retention time of the ADC in HIC chromatogram.
Measurement system: Chromaster (registered trademark) (manufactured by Hitachi, Ltd.)
Column: Tosoh Biobuthyl NPR 2.5 um 4.6 × 35 mm column manufactured by Tosoh Bio-Sciences Inc.
Gradient: linear gradient of eluent A/B
Flow rate: 0.8 mL/min
Eluent A: 1.1 M (NH₄)₂SO₄, 25mM Na₂HPO₄/NaH₂PO₄ (pH 6.0)
Eluent B: 25 mM Na₂HPO₄/NaH₂PO₄ (pH 6.0, 25 v/v% isopropanol added)
Detector: UV (280 nm)

**Table 3. Evaluation of hydrophobicities of ADC and ADC mimic using HIC-HPLC**

| | Linker-payload mimic or Linker-payload | EXAMPLE/ COMPARATIVE EXAMPLE | Retention time |
|---|---|---|---|
| ADC mimic 1 | Linker-payload mimic (1) | EXAMPLE 1-1 | 12.8 min |
| ADC mimic 2 | Linker-payload mimic (5) | EXAMPLE 1-2 | 11.6 min |
| ADC mimic 3 | Linker-payload mimic (9) | EXAMPLE 1-3 | 12.7 min |
| ADC mimic 4 | Linker-payload mimic (28) | COMPARATIVE EXAMPLE 1 | 13.1 min |
| ADC 1 | Linker-payload (11) | EXAMPLE 2-1 | 12.2 min |
| ADC 2 | Linker-payload (12) | EXAMPLE 2-2 | 11.7 min |
| ADC 3 | Linker-payload (18) | EXAMPLE 2-3 | 12.0 min |
| ADC 4 | Linker-payload (19) | EXAMPLE 2-4 | 12.0 min |
| ADC 5 | Linker-payload (20) | EXAMPLE 2-5 | 11.8 min |
| ADC 6 | Linker-payload (21) | EXAMPLE 2-6 | 12.3 min |
| ADC 9 | Linker-payload (32) | EXAMPLE 2-7 | 11.2 min |
| ADC 7 | Linker-payload (29) | COMPARATIVE EXAMPLE 2 | 11.8 min |
| ADC 8 | MC-VC-MMAE | Commercial product (CAS no: 646502-53-6) | 12.4 min |

### EXAMPLE 6: Evaluation of aggregation ratios of ADC and ADC mimic by size exclusion chromatography (SEC-HPLC)

SEC-HPLC analysis was performed according to a previous report (Chemistry Select, 2020, 5, 8435-8439). Measurement was performed using the following conditions.
Measurement system: 1260 HPLC system (manufactured by Agilent)
Column: AdvanceBio SEC 300 Å, 2.7 um, 4.6 mm × 150 mm, manufactured by Agilent Technologies
Flow rate: 0.25 mL/min
Eluent: 100 mM sodium dihydrogen phosphate/sodium hydrogen phosphate, aqueous solution of 250 mM sodium chloride (pH 6.8), 10% v/v isopropanol
Detector: UV (280 nm)

**Table 4. Evaluation of aggregation ratios of ADC and ADC mimic using SEC-HPLC**

| | Linker-payload mimic or Linker-payload | EXAMPLE/ COMPARATIVE EXAMPLE | aggregation ratio |
|---|---|---|---|
| ADC mimic 1 | Linker-payload mimic (1) | EXAMPLE 1-1 | 3.0% |
| ADC mimic 2 | Linker-payload mimic (5) | EXAMPLE 1-2 | 2.9% |
| ADC mimic 3 | Linker-payload mimic (9) | EXAMPLE 1-3 | 2.9% |
| ADC mimic 4 | Linker-payload mimic (28) | COMPARATIVE EXAMPLE 1 | 6.2% |
| ADC 1 | Linker-payload (11) | EXAMPLE 2-1 | 2.10 |
| ADC 2 | Linker-payload (12) | EXAMPLE 2-2 | 1.8% |
| ADC 3 | Linker-payload (18) | EXAMPLE 2-3 | 1.8% |
| ADC 4 | Linker-payload (19) | EXAMPLE 2-4 | 2.0% |
| ADC 5 | Linker-payload (20) | EXAMPLE 2-5 | 2.0% |
| ADC 6 | Linker-payload (21) | EXAMPLE 2-6 | 2.0% |
| ADC 9 | Linker-payload (32) | EXAMPLE 2-7 | 1.2% |
| ADC 7 | Linker-payload (29) | COMPARATIVE EXAMPLE 2 | 2.2% |
| ADC 8 | MC-VC-MMAE | Commercial product (CAS no: 646502-53-6) | 5.1% |

### EXAMPLE 7: Evaluation of ADC mimics using enzyme cathepsin B

Cleave abilities for various ADC mimics by cathepsin B were evaluated by analyzing the amount of fluorescent molecules released from the ADC mimics as described below.

### (7-1) Cathepsin B cleavability test

A cathepsin B cleavability test was performed as follows according to a previous report (Nature Communications 2018, 9, 2512). An ADC mimic was added to 180 µL of a MES buffer (10 mM MES, 40 µM DTT, pH 5.0) so as to have a concentration of 1 mg/mL, and then 30 µL of the solution was poured into each of six Eppendorf tubes. To each of three of the six samples, 100 µL of acetonitrile was immediately added at 0°C. The mixture was stirred by vortex, and then centrifuged to obtain a precipitate. The resulting supernatant solution was collected and subjected to HPLC analysis. The remaining three samples were incubated at 37°C for six hours. To each of the samples, 100 µL of acetonitrile was added. The mixture was stirred by vortex, and then centrifuged to obtain a precipitate. The resulting supernatant solution was collected and subjected to HPLC analysis.

### (7-2) Analysis of amount of released fluorescent molecules using HPLC analysis

For the measurement, the amount of fluorescent molecules released from an ADC mimic was measured using a liquid chromatography/fluorescence detection method. The three samples to which acetonitrile was immediately added at 0°C in Example 7-1 were taken as 0 hour samples, and the three samples incubated at 37°C for six hours as described in Example 7-1 were taken as 6 hour samples. A difference in fluorescence intensity between the 6 hour samples and the 0 hour samples was analyzed.

Separately, a correlation between the area of a fluorescence intensity area by HPLC and a concentration was calculated using Pyrene. The difference in fluorescence intensity between the ADC mimics was converted into a concentration using the calculation formula. When the concentration at 0 hour was set to 100%, a ratio of the above-described difference in fluorescence intensity was calculated as a releasing ratio.

**Table 5. Evaluation of cleave ability for ADC mimic by cathepsin B**

| | Linker-payload mimic | EXAMPLE/ COMPARATIVE EXAMPLE | Releasing ratio of fluorescent molecule for 6 hours |
|---|---|---|---|
| ADC mimic 1 | Linker-payload mimic (1) | EXAMPLE 1-1 | 88% |
| ADC mimic 2 | Linker-payload mimic (5) | EXAMPLE 1-2 | 89% |
| ADC mimic 3 | Linker-payload mimic (9) | EXAMPLE 1-3 | 100% |
| ADC mimic 4 | Linker-payload mimic (28) | COMPARATIVE EXAMPLE 1 | 53% |

As a result, the ADC mimic synthesized in Example 1 had high reactivity to cathepsin and immediately emitted fluorescent molecules, whereas the ADC mimics synthesized in Comparative Examples 1-1 and 1-2 had low reactivity to cathepsin.

### EXAMPLE 8: Evaluation of ADC using enzyme cathepsin B

Cleave abilities for various ADCs by cathepsin B were evaluated by analyzing the amount of fluorescent molecules released from the ADCs as described below.

### (8-1) Cathepsin B cleavability test

A cathepsin B cleavability test was performed according to a previous report (Nature Communications 2018, 9, 2512).

### (8-2) Analysis of amount of released payload using HPLC analysis

The amount of payload released from an ADC was measured using liquid chromatography mass spectrometry (comprising tandem mass spectrometry). The three samples to which acetonitrile was immediately added at 0°C in Example 8-1 were taken as 0 hour samples, and the three samples incubated at 37°C for six hours in Example 8-1 were taken as 6 hour samples. MS intensities of payloads detected from the 6 hour samples and the 0 hour samples were calculated by an extracted ion chromatogram, and a difference therebetween was analyzed.

Separately, a correlation between the area of a TIC are by HPLC and a concentration was calculated using MMAE. TIC of a fluorescence intensity of each of the ADCs was converted into a concentration using the calculation formula. When the concentration at Day 0 was set to 100%, a ratio of the above-described difference in ion chromatogram was calculated as a releasing ratio.

**Table 6. Evaluation of cleave ability for ADC by cathepsin B**

| | Linker-payload | EXAMPLE/ COMPARATIVE EXAMPLE | Releasing ratio of payload for 6 hours |
|---|---|---|---|
| ADC 1 | Linker-payload (11) | EXAMPLE 2-1 | 112% |
| ADC 2 | Linker-payload (12) | EXAMPLE 2-2 | 193% |
| ADC 3 | Linker-payload (18) | EXAMPLE 2-3 | 181% |
| ADC 4 | Linker-payload (19) | EXAMPLE 2-4 | 146% |
| ADC 5 | Linker-payload (20) | EXAMPLE 2-5 | 182% |
| ADC 6 | Linker-payload (21) | EXAMPLE 2-6 | 191% |
| ADC 9 | Linker-payload (32) | EXAMPLE 2-7 | 193% |
| ADC 7 | Linker-payload (29) | COMPARATIVE EXAMPLE 2 | 200% |
| ADC 8 | MC-VC-MMAE | Commercial product (CAS no: 646502-53-6) | 73% |

As a result, it was found that the ADC mimic synthesized in Example 2 had high reactivity to cathepsin and immediately releases payload.

### EXAMPLE 9: Evaluation of ADC mimic using mouse plasma

### (9-1) Test for stability of ADC mimic in plasma

An ADC mimic was added to 500 µL of mouse plasma (manufactured by Charles River) so as to have a concentration of 0.1 mg/mL, and then the mixture was subjected to sterile filtration. 50 µL of this solution was poured into each of six Eppendorf tubes. Three of the six samples were stored in an incubator set at 37°C for four days. The remaining three samples were stored in a freezer at -80°C for four days similarly. To each of the samples, 100 µL of acetonitrile was added. The mixture was stirred by vortex, and then centrifuged to obtain a precipitate. The resulting supernatant solution was collected and subjected to HPLC analysis.

### (9-2) Analysis of amount of released fluorescent molecules using HPLC analysis

For the measurement, the amount of fluorescent molecules released from an ADC mimic was measured using a liquid chromatography/fluorescence detection method. The three samples stored in a freezer in Example 9-1 were taken as Day = 0 samples, and the three samples stored at 37°C in Example 9-1 were taken as Day = 4 samples. A difference in fluorescence intensity between Day = 4 samples and Day = 0 samples was analyzed.

A releasing ratio of the fluorescent molecules was calculated according to Example 7-2.

Regarding the results, as illustrated in the following Table, a releasing ratio of the fluorescent molecules was evaluated.

**Table 7. Results of stability test in plasma using ADC mimic**

| | Linker-payload mimic | EXAMPLE/ COMPARATIVE EXAMPLE | Releasing ratio of payload on Day 4 |
|---|---|---|---|
| ADC mimic 1 | Linker-payload mimic (1) | EXAMPLE 1-1 | 8% |
| ADC mimic 2 | Linker-payload mimic (5) | EXAMPLE 1-2 | 5% |
| ADC mimic 3 | Linker-payload mimic (9) | EXAMPLE 1-3 | 9% |
| ADC mimic 4 | Linker-payload mimic (28) | COMPARATIVE EXAMPLE 1 | 38% |

### EXAMPLE 10: Evaluation of ADC using mouse plasma (10-1) Test for stability of ADC in plasma

An ADC mimic was added to 500 µL of mouse plasma (manufactured by Charles River) so as to have a concentration of 0.1 mg/mL, and then the mixture was subjected to sterile filtration. 50 µL of this solution was poured into each of six Eppendorf tubes. Three of the six samples were stored in an incubator set at 37°C for four days. The remaining three samples were stored in a freezer at -80°C for four days similarly. To each of the samples, 100 µL of acetonitrile was added. The mixture was stirred by vortex, and then centrifuged to obtain a precipitate. The resulting supernatant solution was collected and subjected to HPLC analysis.

### (10-2) Analysis of amount of released payload using HPLC analysis

The amount of payload released from an ADC was measured using liquid chromatography mass spectrometry (comprising tandem mass spectrometry). The three samples to which acetonitrile was immediately added at 0°C in Example 10-1 were taken as 0 hour samples, and the three samples incubated at 37°C for six hours in Example 8-1 were taken as 6 hour samples. MS intensities of payloads detected from the 6 hour samples and the 0 hour samples were calculated by an extracted ion chromatogram, and a difference therebetween was analyzed. A releasing ratio of payload was calculated according to Example 8-2.

**Table 8. Results of stability test in plasma using ADC**

| | Linker-payload | EXAMPLE/ COMPARATIVE EXAMPLE | Releasing ratio of payload on Day 4 |
|---|---|---|---|
| ADC 1 | Linker-payload (11) | EXAMPLE 2-1 | 6% |
| ADC 2 | Linker-payload (12) | EXAMPLE 2-2 | 3% |
| ADC 3 | Linker-payload (18) | EXAMPLE 2-3 | 7% |
| ADC 4 | Linker-payload (19) | EXAMPLE 2-4 | 8% |
| ADC 5 | Linker-payload (20) | EXAMPLE 2-5 | 10% |
| ADC 6 | Linker-payload (21) | EXAMPLE 2-6 | 4% |
| ADC 9 | Linker-payload (32) | EXAMPLE 2-7 | 2% |
| ADC 7 | Linker-payload (29) | COMPARATIVE EXAMPLE 2 | 44% |
| ADC 8 | MC-VC-MMAE | Commercial product (CAS no: 646502-53-6) | 34% |

## Claims

1. A conjugate of an antibody and a functional substance or a salt thereof, which comprises a structural unit represented by the following formula (I): 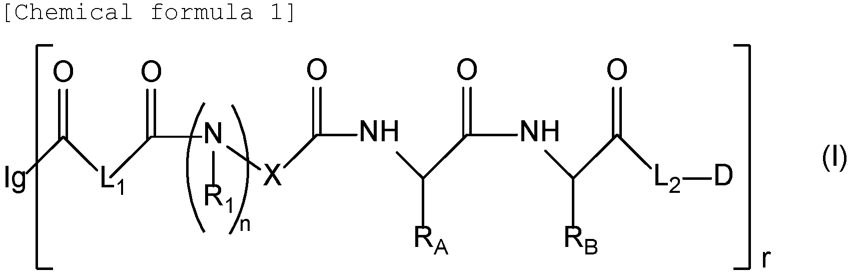 wherein
Ig represents an immunoglobulin unit comprising two heavy chains and two light chains, and regioselectively forms an amide bond with a carbonyl group adjacent to Ig via an amino group in side chains of lysine residues in the two heavy chains,
L₁ and L₂ each represent a divalent group,
R₁ represents a monovalent group optionally comprising a hydrophilic group,
X represents a divalent group optionally having a substituent, wherein the divalent group has 1 to 3 carbon atoms constituting a main chain portion linking two atoms present on both sides of X, and the substituent is a monovalent group optionally comprising a hydrophilic group,
D represents a functional substance,
R_{A} represents a side chain of a valine residue,
R_{B} represents a side chain of a citrulline residue or an alanine residue,
n is 0 or 1, wherein when n is 1, the substituent in X together with R₁ may form a ring optionally comprising a hydrophilic group, and
an average ratio r of the amide bonds per two heavy chains is 1.5 to 2.5, and
wherein at least one hydrophilic group is present in the structural unit.

2. The conjugate or a salt thereof according to claim 1, wherein the immunoglobulin unit is a human immunoglobulin unit.

3. The conjugate or a salt thereof of claim 2, wherein the human immunoglobulin unit is a human IgG antibody.

4. The conjugate or a salt thereof according to claim 1, wherein the lysine residue is present at position 246/248, position 288/290, or position 317 in accordance with Eu numbering.

5. The conjugate or a salt thereof according to claim 1, wherein r is 1.9 to 2.1.

6. The conjugate or a salt thereof according to claim 1, wherein the hydrophilic group is one or more groups selected from the group consisting of a carboxylate group, a sulfonate group, a hydroxy group, a polyethylene glycol group, a polysarcosine group, and a sugar portion.

7. The conjugate or a salt thereof according to claim 1, wherein L₁ represents a divalent group represented by the following formula (i):
-L₄-Y-L₃- (i),
wherein L₃ and L₄ each independently comprise a divalent group selected from the group consisting of - (C(R)₂)ₘ-, -(O-C(R)₂-C(R)₂)ₘ-, and -(C(R)₂-C(R)₂-O)ₘ-, and a combination thereof,
Rs each independently represent a hydrogen atom, a C₁₋₆ alkyl, a C₂₋₆ alkenyl, or a C₂₋₆ alkynyl,
m is an integer of 0 to 20, and
Y represents a divalent group generated by a reaction of two bioorthogonal functional groups capable of reacting with each other.

8. The conjugate or a salt thereof according to claim 7, wherein L₃ and L₄ each independently comprise -(C(R)₂)ₘ-.

9. The conjugate or a salt thereof according to claim 7, wherein Y is a divalent group represented by any one of the following structural formulae: 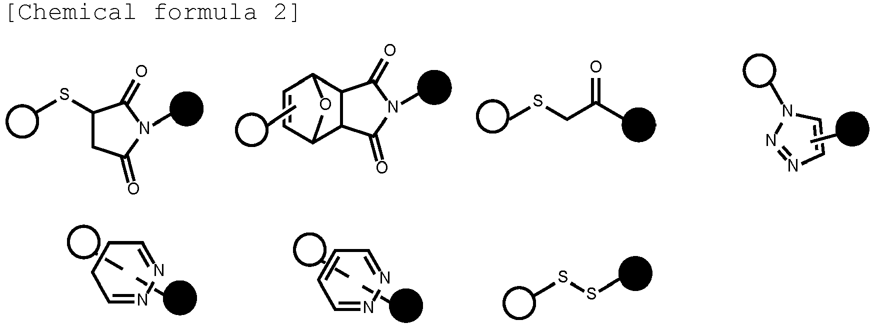
wherein a white circle and a black circle each represent a bond,
when the bond of the white circle is bonded to L₃, the bond of the black circle is bonded to L₄, and
when the bond of the white circle is bonded to L₄, the bond of the black circle is bonded to L₃.

10. The conjugate or a salt thereof according to claim 1, wherein the structural unit represented by formula (I) is a structural unit represented by the following formula (I-1), (I-2), (I-3), or (I-4): 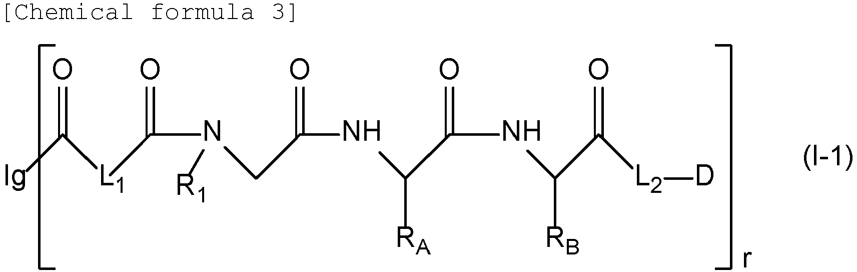 wherein
Ig, L₁, L₂, D, R_{A}, R_{B}, and r are the same as those in formula (I), and
R₁ represents a monovalent group comprising a hydrophilic group; 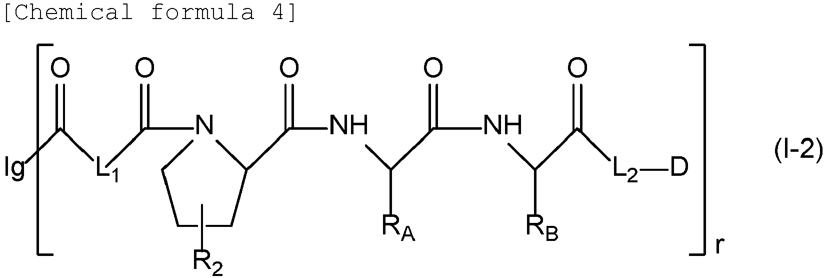
wherein
Ig, L₁, L₂, D, R_{A}, R_{B}, and r are the same as those in formula (I), and
R₂ represents a monovalent group comprising a hydrophilic group; 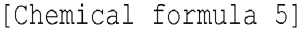 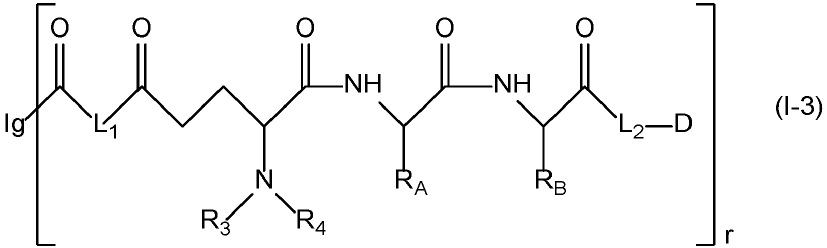
wherein
Ig, L₁, L₂, D, R_{A}, R_{B}, and r are the same as those in formula (I),
R₃ represents a hydrogen atom, a C₁₋₆ alkyl, or a monovalent group comprising a hydrophilic group and
R₄ represents a monovalent group comprising a hydrophilic group; or 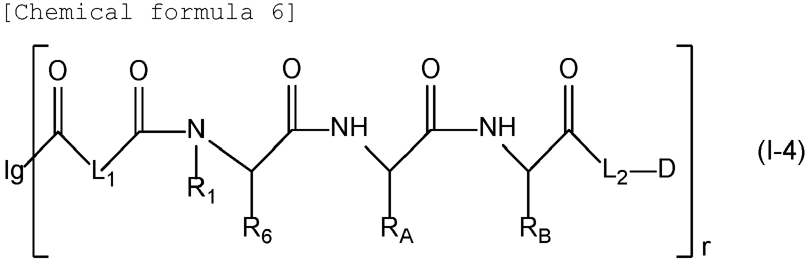
wherein
Ig, L₁, L₂, D, R_{A}, R_{B}, and r are the same as those in formula (I),
R₁ represents a hydrogen atom or a C₁₋₆ alkyl, and
R₆ represents a monovalent group comprising a hydrophilic group.

11. The conjugate or a salt thereof according to claim 10, wherein the structural unit represented by formula (I) is a structural unit represented by formula (I-1), (I-2), or (I-3) .

12. The conjugate or a salt thereof according to claim 10, wherein the structural unit represented by formula (I-1), (I-2), (I-3), or (I-4) is a structural unit represented by the following formula (I-1a'), (I-1b'), (I-1c'), (I-2'), (I-3a'), (I-3b'), (I-4a'), (I-4b'), or (I-4c'): 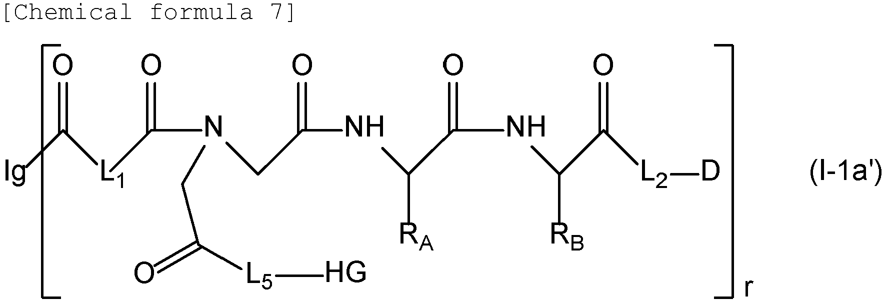 wherein
Ig, L₁, L₂, D, R_{A}, R_{B}, and r are the same as those in formula (I),
L₅ represents a bond or a divalent group, and
HG represents a hydrophilic group; 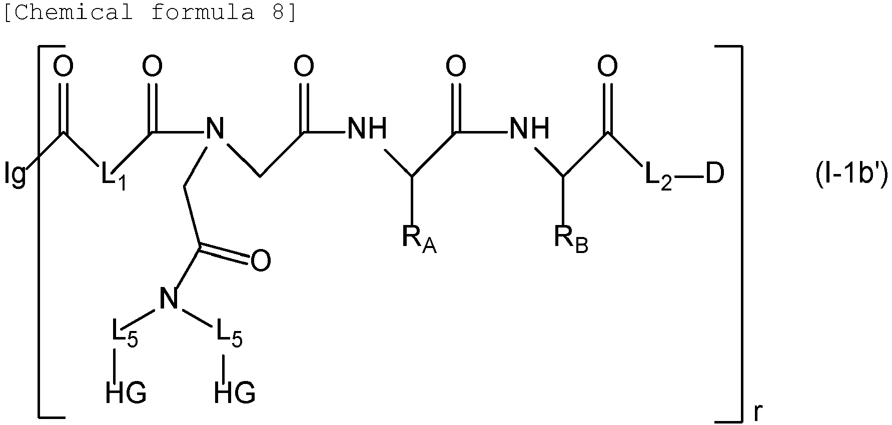
wherein
Ig, L₁, L₂, D, R_{A}, R_{B}, and r are the same as those in formula (I),
two L₅ each independently represent a bond or a divalent group, and
two HG each independently represent a hydrophilic group; 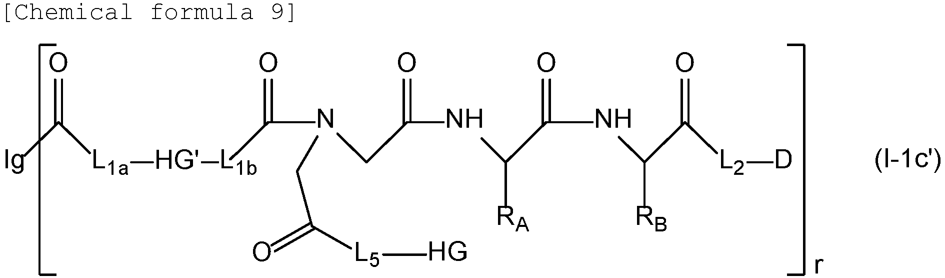
wherein
Ig, L₂, D, R_{A}, R_{B}, and r are the same as those in formula (I),
L₅ represents a bond or a divalent group,
HG represents a hydrophilic group,
L₁ₐ and L_{1b} each independently represent a bond or a divalent group, and
HG' represents a divalent hydrophilic group; 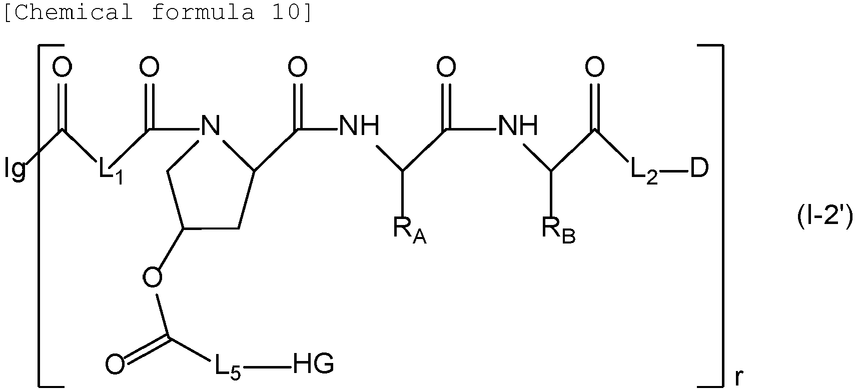
wherein
Ig, L₁, L₂, D, R_{A}, R_{B}, and r are the same as those in formula (I),
L₅ represents a bond or a divalent group, and
HG represents a hydrophilic group; 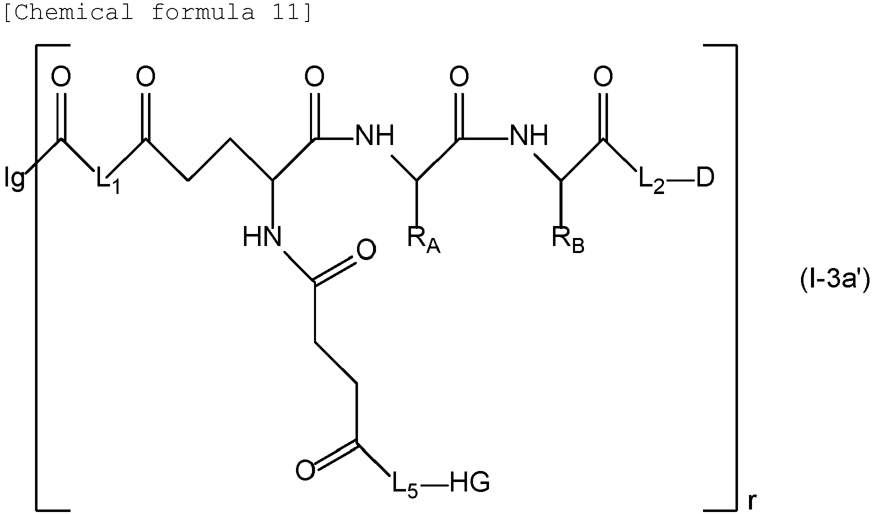
wherein
Ig, L₁, L₂, D, R_{A}, R_{B}, and r are the same as those in formula (I),
L₅ represents a bond or a divalent group, and
HG represents a hydrophilic group; 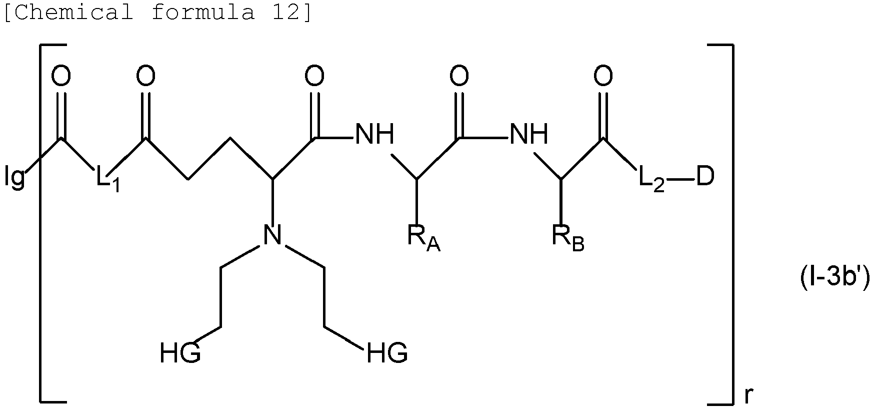
wherein
Ig, L₁, L₂, D, R_{A}, R_{B}, and r are the same as those in formula (I), and
HG represents a hydrophilic group; 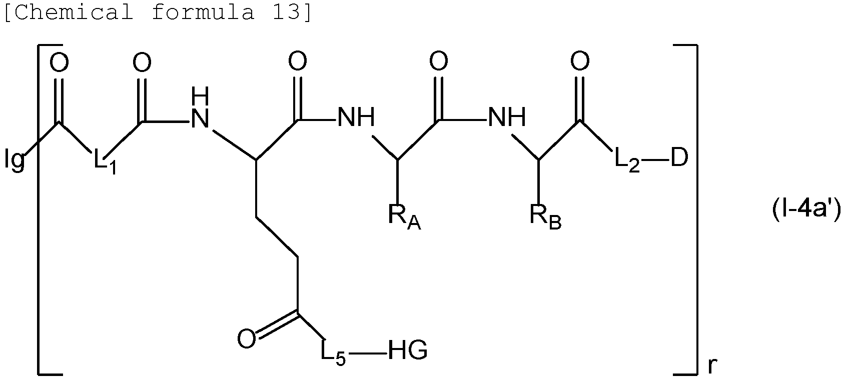
wherein
Ig, L₁, L₂, D, R_{A}, R_{B}, and r are the same as those in formula (I),
L₅ represents a bond or a divalent group, and
HG represents a hydrophilic group; 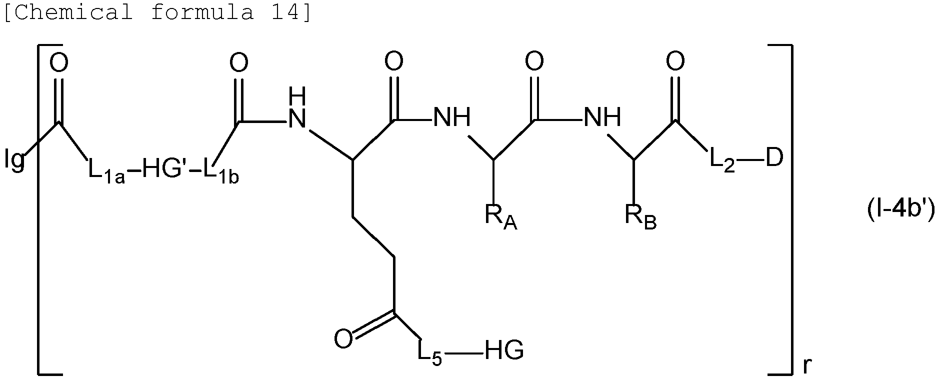
wherein
Ig, L₂, D, R_{A}, R_{B}, and r are the same as those in formula (I),
L₅ represents a bond or a divalent group,
HG represents a hydrophilic group,
L₁ₐ and L_{1b} each independently represent a bond or a divalent group,
HG' represents a divalent hydrophilic group; or 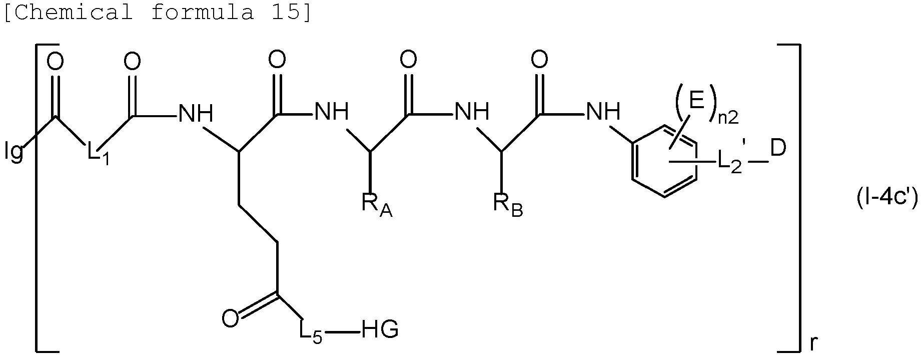
wherein
Ig, L₁, D, R_{A}, R_{B}, and r are the same as those in formula (I),
L₅ represents a bond or a divalent group,
HG represents a hydrophilic group,
L₂' represents a bond or a divalent group,
E represents an electron-withdrawing group, and
n2 is an integer of 1 to 4.

13. The conjugate or a salt thereof according to claim 12, wherein the structural unit represented by formula (I-1a'), (I-1b'), (I-1c'), (I-2'), (I-3a'), (I-3b'), (I-4a'), (I-4b') or (I-4c') is a structural unit represented by the following formula (I-1a'-1), (I-1a'-2), (I-1b'-1), (I-1c'-1), (I-2'-1), (I-2'-2), (I-3a'-1), (I-3a'-2), (I-3b'-1), (I-4a'-1) , (I-4b'-1), (I-4c'-1), or (1-4c'-2): 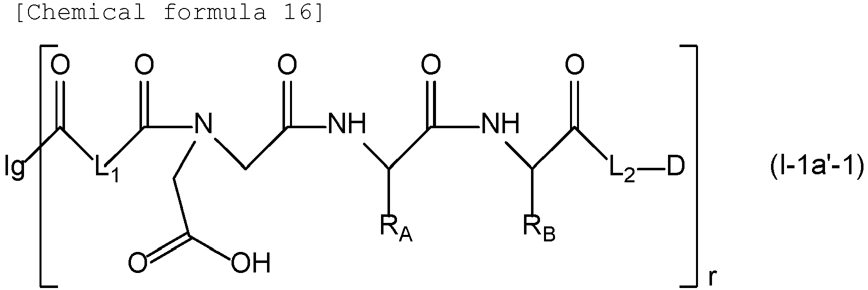
wherein Ig, L₁, L₂, D, R_{A}, R_{B}, and r are the same as those in formula (I); 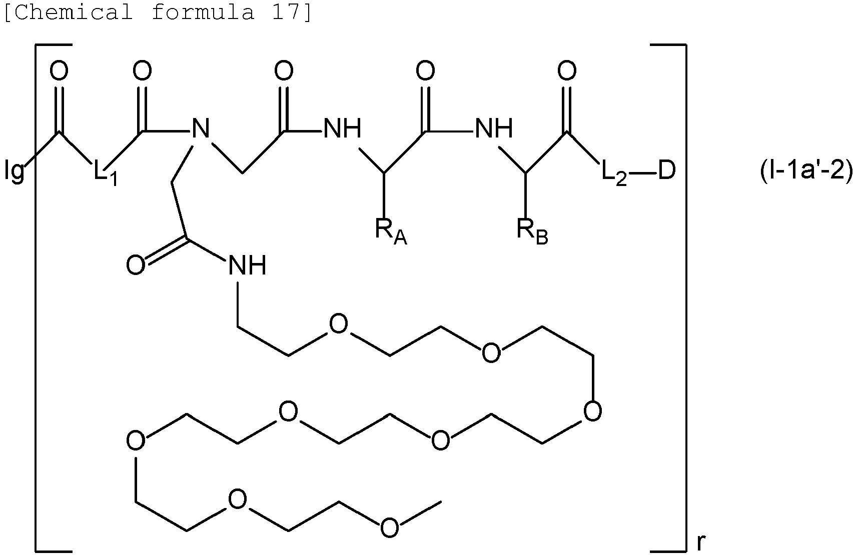
wherein
Ig, L₁, L₂, D, R_{A}, R_{B}, and r are the same as those in formula (I); 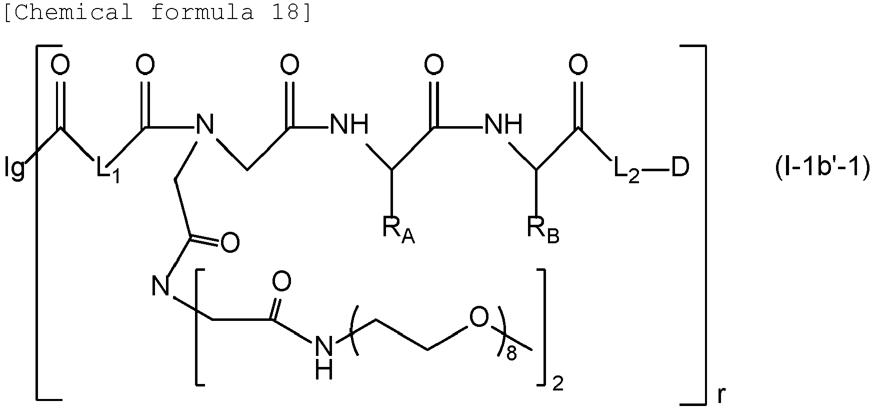
wherein Ig, L₁, L₂, D, R_{A}, R_{B}, and r are the same as those in formula (I); 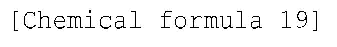 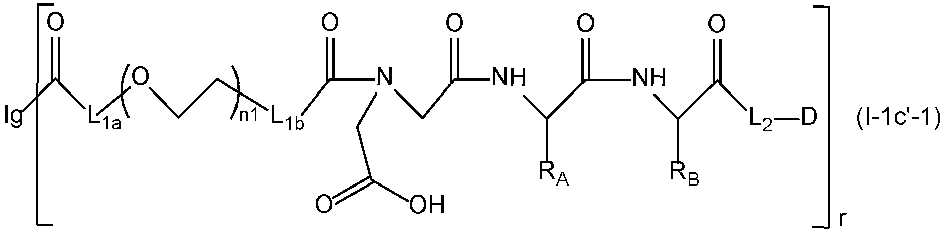
wherein
Ig, L₂, D, R_{A}, R_{B}, and r are the same as those in formula (I)
L₁ₐ and L_{1b} each independently represent a bond or a divalent group, and
n1 is an integer of 3 to 20; 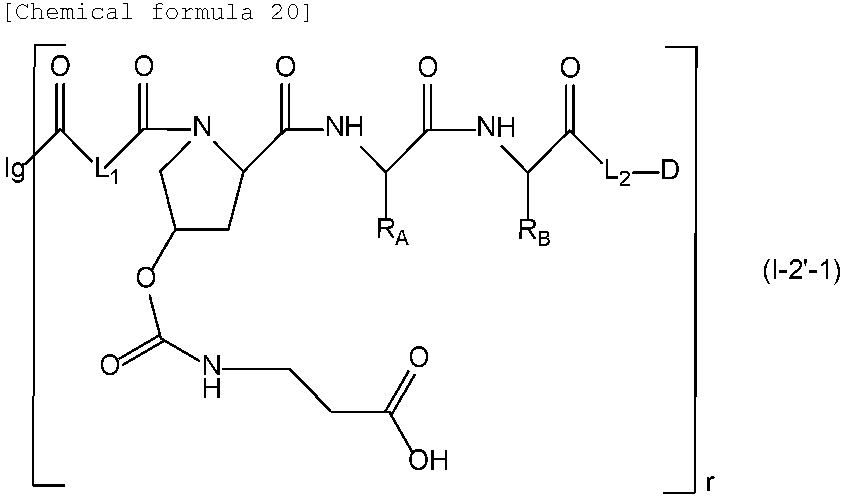
wherein
Ig, L₁, L₂, D, R_{A}, R_{B}, and r are the same as those in formula (I); 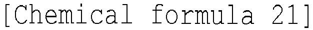 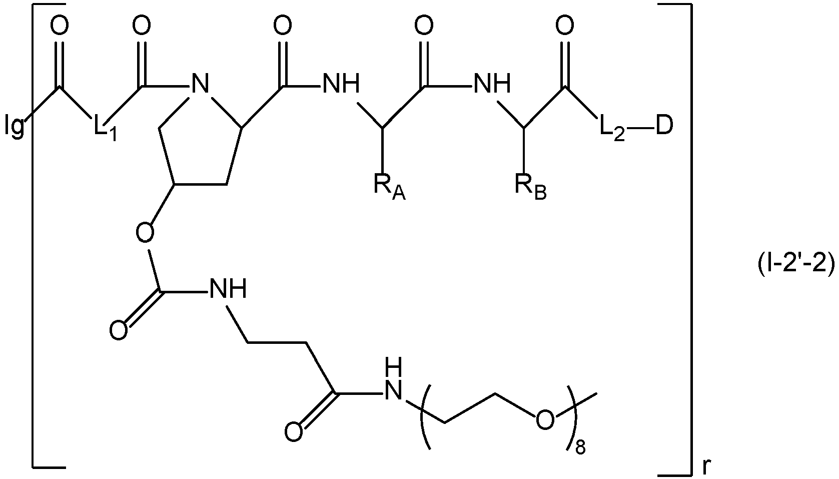
wherein Ig, L₁, L₂, D, R_{A}, R_{B}, and r are the same as those in formula (I); 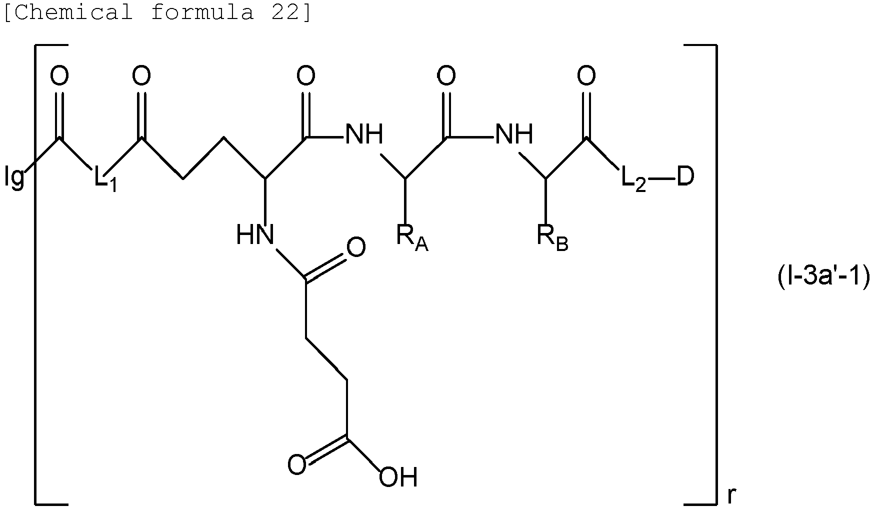
wherein Ig, L₁, L₂, D, R_{A}, R_{B}, and r are the same as those in formula (I); 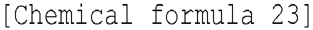 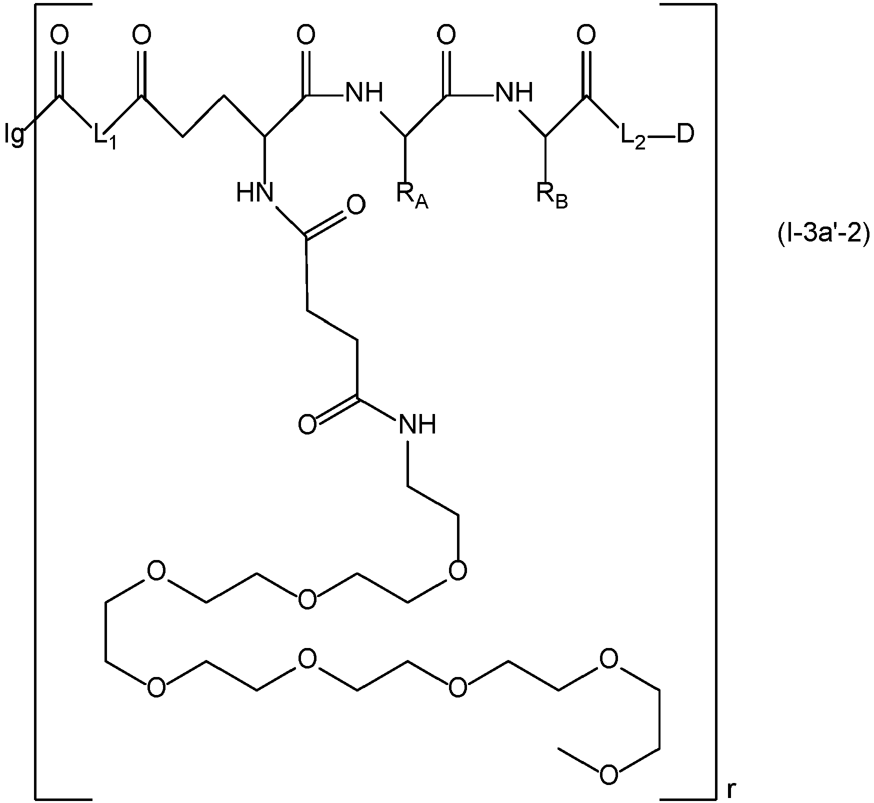
wherein Ig, L₁, L₂, D, R_{A}, R_{B}, and r are the same as those in formula (I); 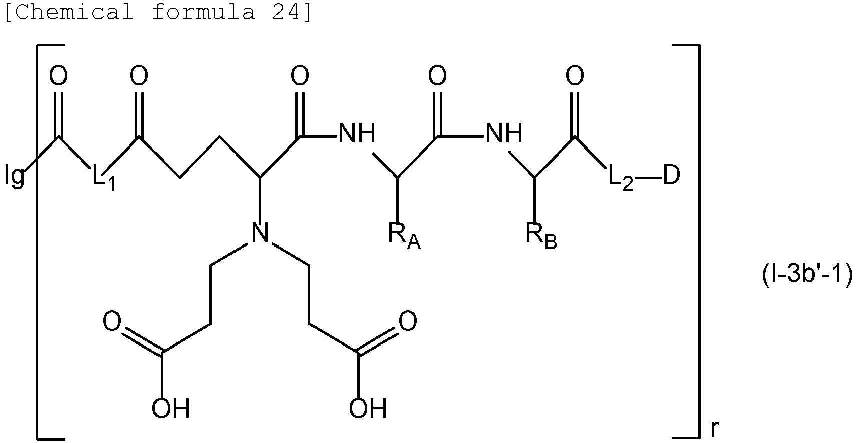
wherein Ig, L₁, L₂, D, R_{A}, R_{B}, and r are the same as those in formula (I); 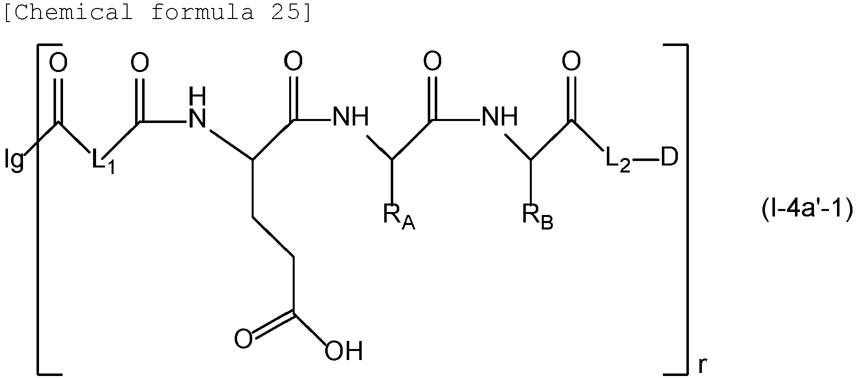
wherein Ig, L₁, L₂, D, R_{A}, R_{B}, and r are the same as those in formula (I); 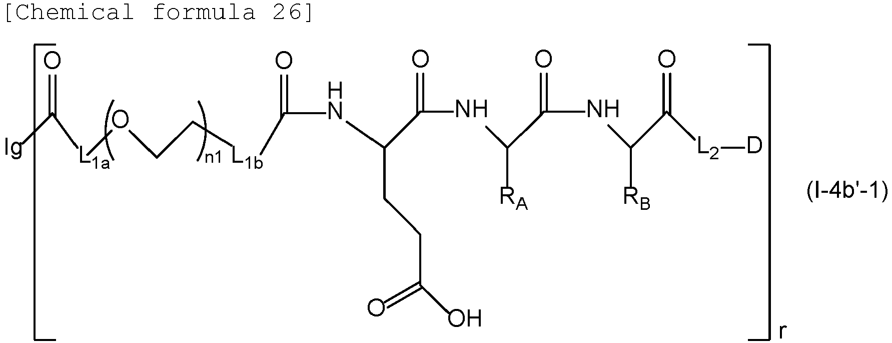
wherein
Ig, L₂, D, R_{A}, R_{B}, and r are the same as those in formula (I),
L₁ₐ and L_{1b} each independently represent a bond or a divalent group, and
n1 is an integer of 3 to 20; 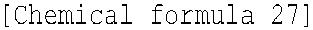 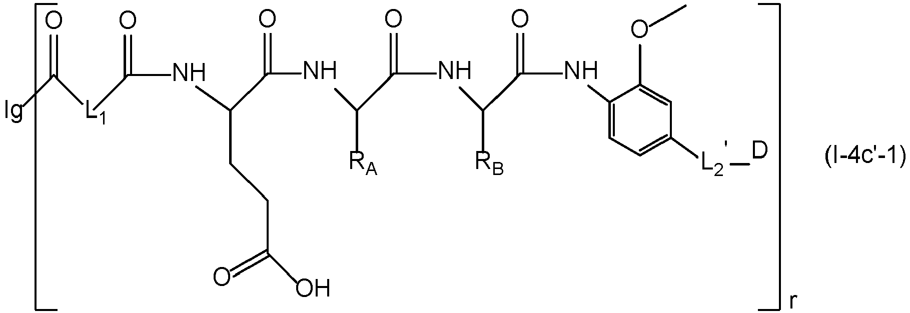
wherein
Ig, L₁, D, R_{A}, R_{B}, and r are the same as those in formula (I);
L₂' represent a bond or a divalent group; or 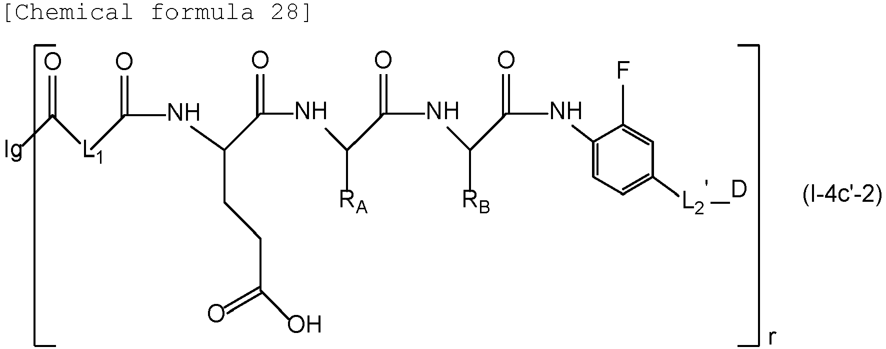
wherein
Ig, L₁, D, R_{A}, R_{B}, and r are the same as those in formula (I);
L₂' represent a bond or a divalent group.

14. The conjugate or a salt thereof according to any one of claims 1 to 13, wherein L₂ comprises a divalent group represented by the following structural formula: 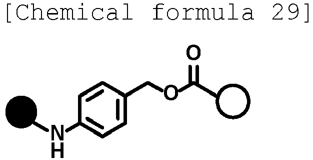 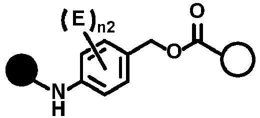
wherein a black circle and a white circle each represent a bond,
the bond of the black circle is bonded to a carbonyl group adjacent to L₂,
the bond of the white circle is bonded to D,
E represents an electron-withdrawing group, and
n2 is an integer of 1 to 4.

15. A compound or a salt thereof represented by the following formula (II-1), (II-2), (II-3), (II-4b'), or (II-4c'): 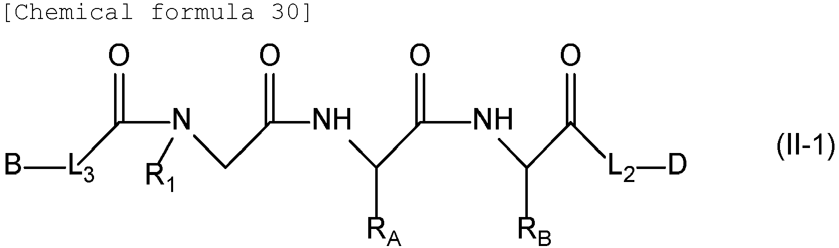 wherein
L₂ and L₃ each represent a divalent group,
R₁ represents a monovalent group optionally comprising a hydrophilic group,
D represents a functional substance,
R_{A} represents a side chain of a valine residue,
R_{B} represents a side chain of a citrulline residue or an alanine residue, and
B represents a bioorthogonal functional group; 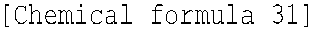 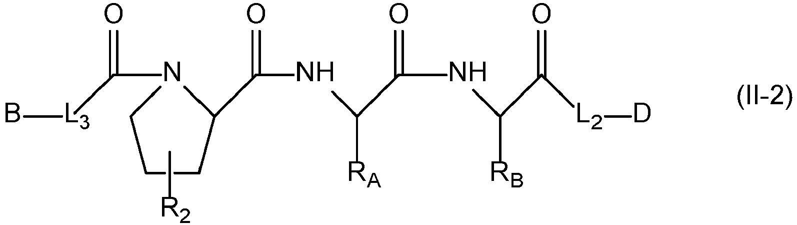
wherein
L₂, L₃, D, R_{A}, R_{B}, and B are the same as those in formula (II-1), and
R₂ represents a monovalent group optionally comprising a hydrophilic group; 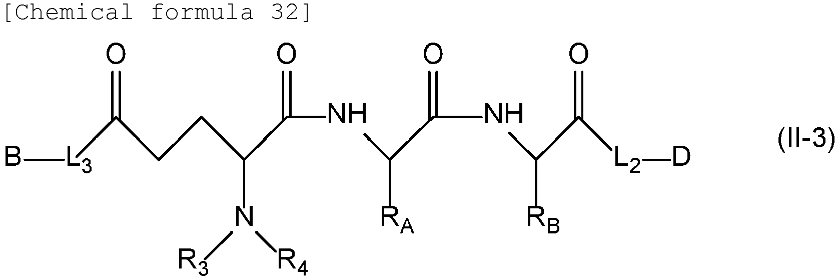
wherein
L₂, L₃, D, R_{A}, R_{B}, and B are the same as in formula (II-1),
R₃ represents a hydrogen atom, a C₁₋₆ alkyl, or a monovalent group comprising a hydrophilic group, and
R₄ represents a monovalent group comprising a hydrophilic group; 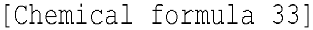 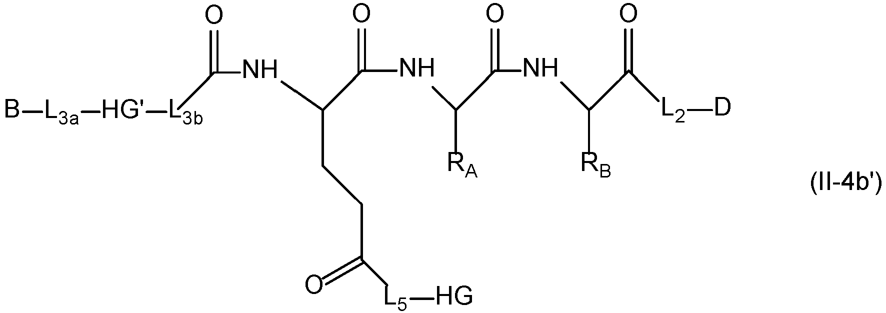
wherein
L₂, D, R_{A}, R_{B}, and B are the same as those in formula (II-1),
L₅ represents a bond or a divalent group,
HG represents a hydrophilic group,
L₃ₐ and L_{3b} each independently represent a bond or a divalent group, and
HG' represents a divalent hydrophilic group.; or 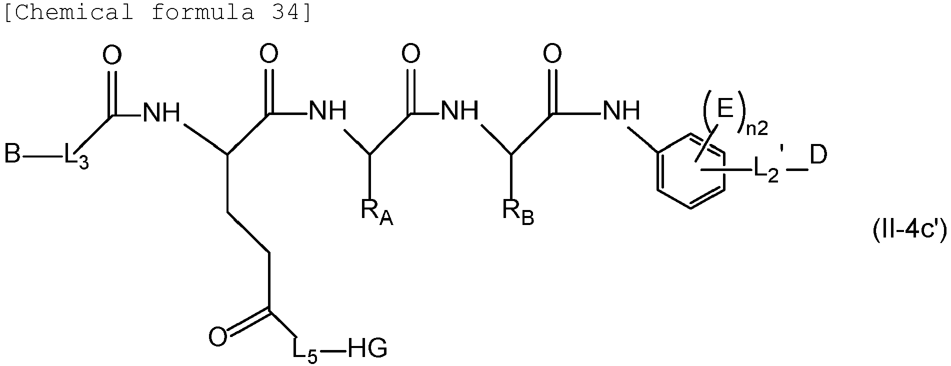
wherein
L₃, D, R_{A}, R_{B}, and B are the same as those in formula (II-1),
L₅ represents a bond or a divalent group,
HG represents a hydrophilic group,
L₂' represent a bond or a divalent group, and
E represents an electron-withdrawing group, and
n2 is an integer of 1 to 4.

16. The compound or a salt thereof according to claim 15, wherein the bioorthogonal functional group is a maleimide residue, a thiol residue, a furan residue, a halocarbonyl residue, an alkene residue, an alkyne residue, an azide residue, or a tetrazine residue.

17. The compound or a salt thereof according to claim 15 or 16, wherein the compound represented by formula (II-1), (II-2), or (II-3) is represented by the following formula (II-1a'), (II-1b'), (II-1c'), (II-2'), (II-3a'), or (II-3b'): 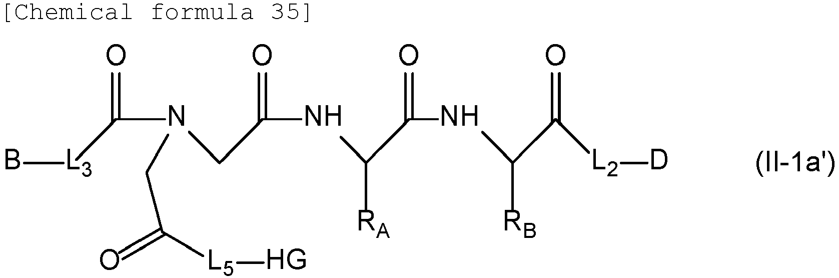 wherein
L₂, L₃, D, R_{A}, R_{B}, and B are the same as those in formula (II-1),
L₅ represents a bond or a divalent group, and
HG represents a hydrophilic group; 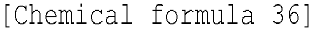 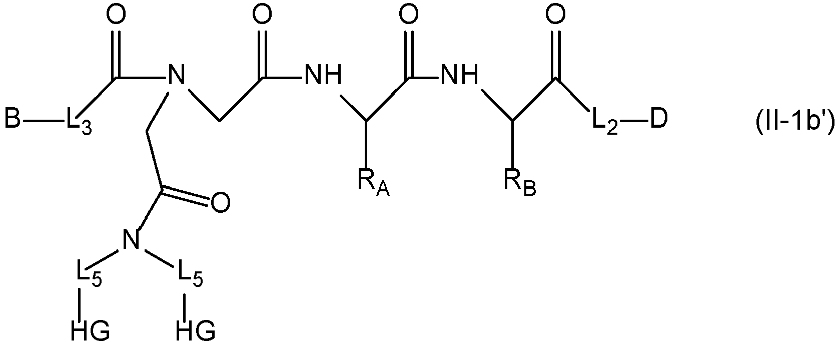
wherein
L₂, L₃, D, R_{A}, R_{B}, and B are the same as those in formula (II-1),
two L₅ each independently represent a bond or a divalent group, and
two HG each independently represent a hydrophilic group. 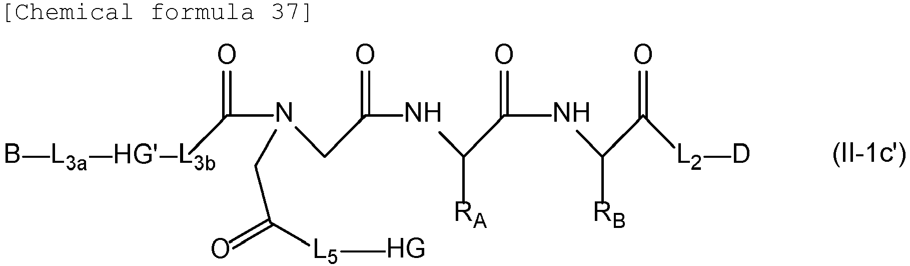
wherein
L₂, D, R_{A}, R_{B}, and B are the same as those in formula (II-1),
L₅ represents a bond or a divalent group,
HG represents a hydrophilic group,
L₃ₐ and L_{3b} each independently represent a bond or a divalent group, and
HG' represents a divalent hydrophilic group 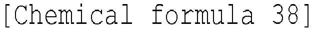 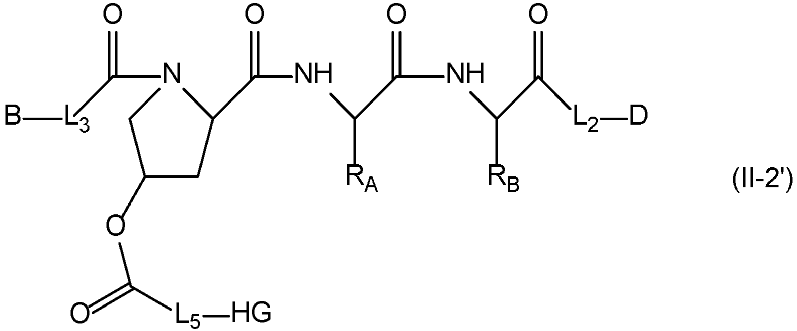
wherein
L₂, L₃, D, R_{A}, R_{B}, and B are the same as those in formula (II-1),
L₅ represents a bond or a divalent group, and
HG represents a hydrophilic group; or 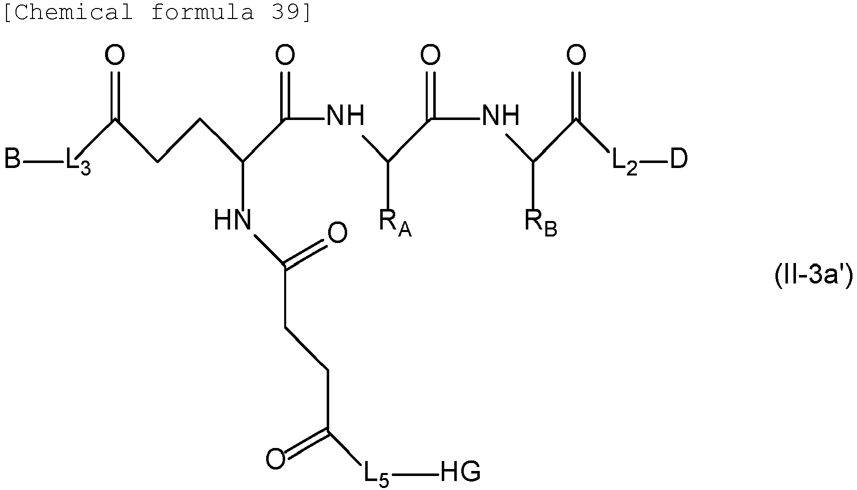
wherein
L₂, L₃, D, R_{A}, R_{B}, and B are the same as those in formula (II-1),
L₅ represents a bond or a divalent group, and
HG represents a hydrophilic group; or 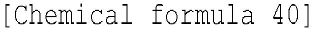 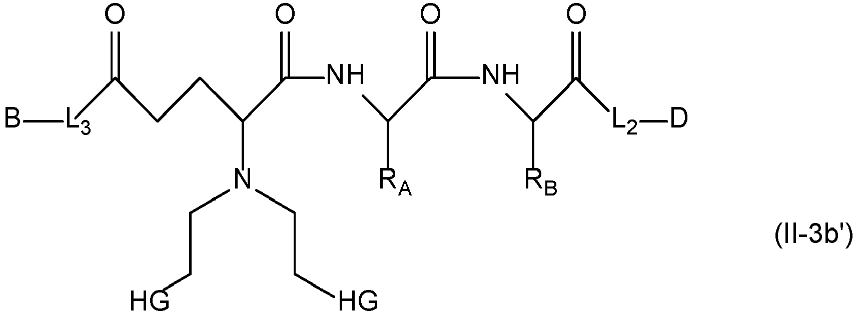
wherein
L₂, L₃, D, R_{A}, R_{B}, and B are the same as those in formula (II-1), and
HG represents a hydrophilic group.

18. The compound or a salt thereof according to any one of claims 15 to 17, wherein the compound represented by formula (II-1a'), (II-1b'), (II-1c'), (II-2'), (II-3a'), (II-3b'), (II-4b'), or (II-4c') is represented by the following formula (II-1a'-1), (II-1a'-2), (II-1b'-1), (II-1c'-1), (II-2'-1), (II-2'-2), (II-3a'-1), (II-3a'-2) , (II-3b'-1), (II-4b'-1) , (II-4c'-1) or (II-4c'-2): 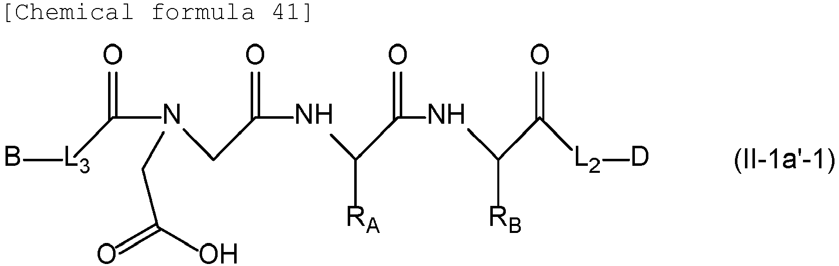
wherein L₂, L₃, D, R_{A}, R_{B}, and B are the same as those in formula (II-1); 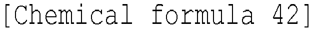 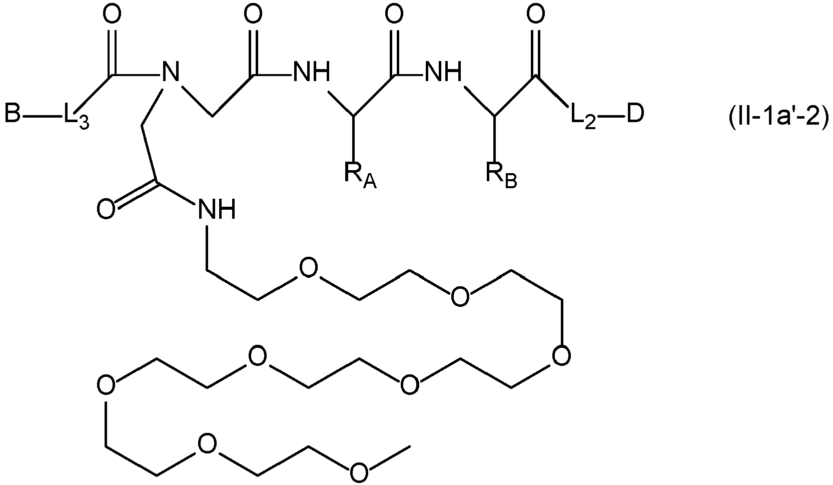
wherein L₂, L₃, D, R_{A}, R_{B}, and B are the same as those in formula (II-1); 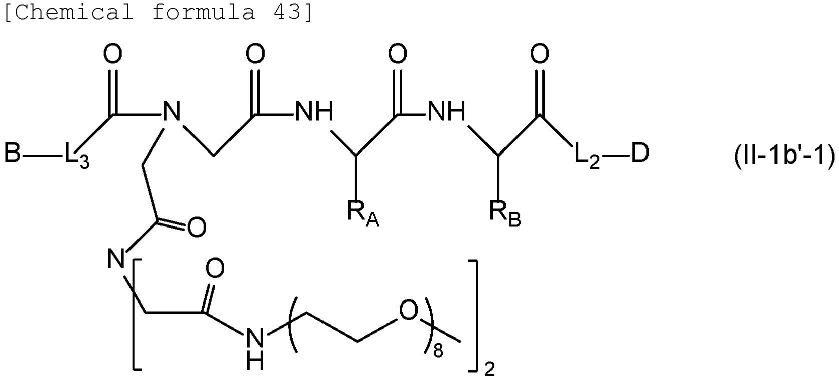
wherein
L₂, L₃, D, R_{A}, R_{B}, and B are the same as those in formula (II-1); 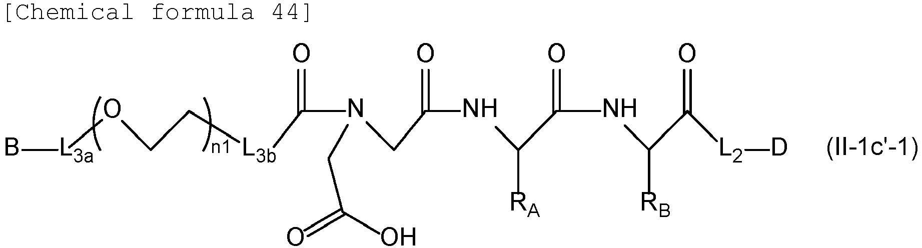
wherein
L₂, D, R_{A}, R_{B}, and B are the same as those in formula (II-1),
L₃ₐ and L_{3b} each independently represent a bond or a divalent group, and
n1 is a integer of 3 to 20; 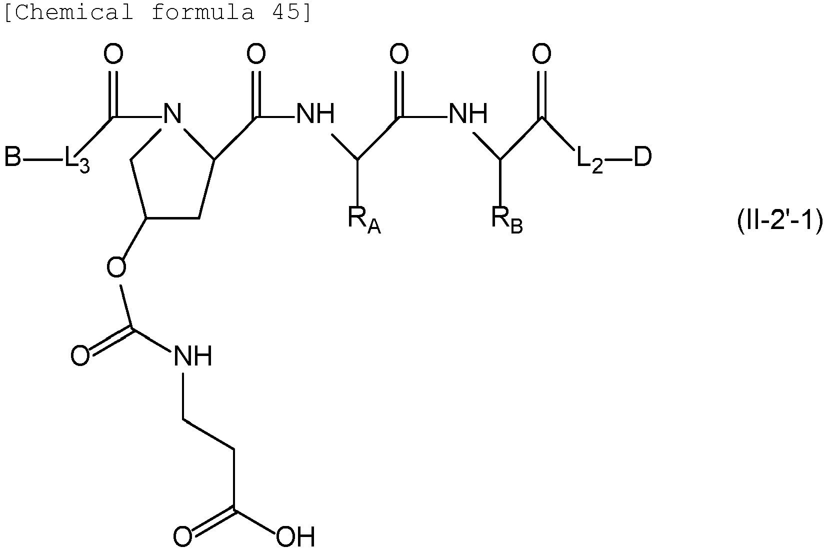
wherein
L₂, L₃, D, R_{A}, R_{B}, and B are the same as those in formula (II-1); 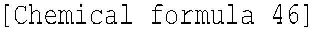 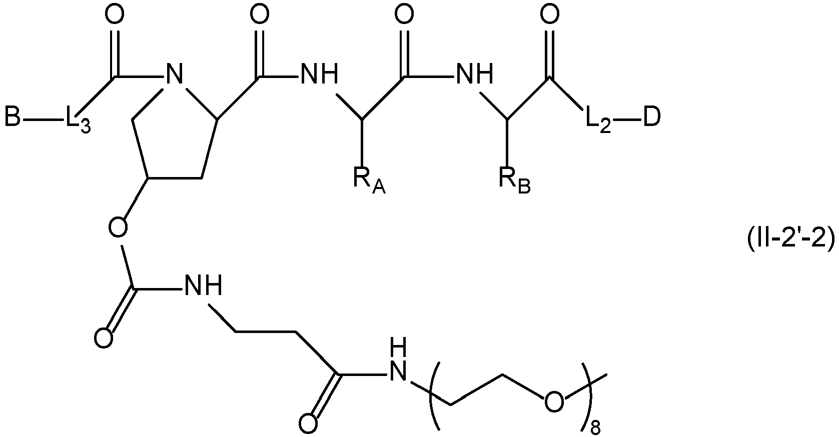
wherein L₂, L₃, D, R_{A}, R_{B}, and B are the same as those in formula (II-1); 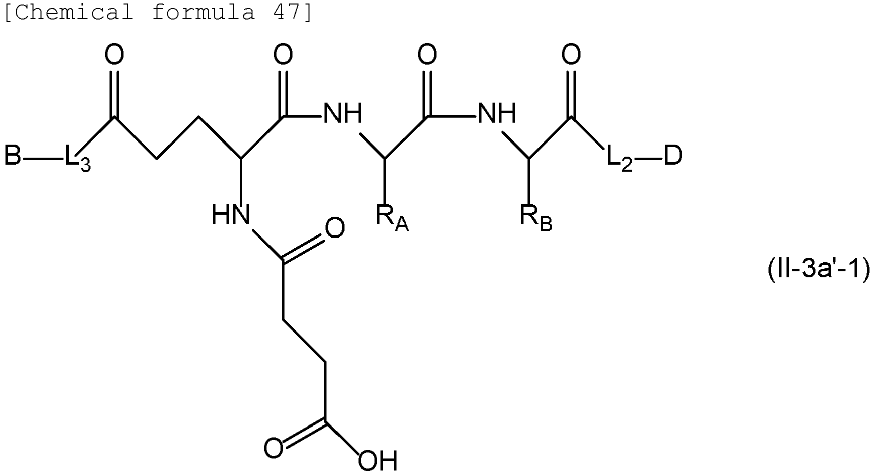
wherein
L₂, L₃, D, R_{A}, R_{B}, and B are the same as those in formula (II-1); 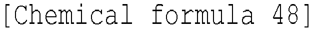 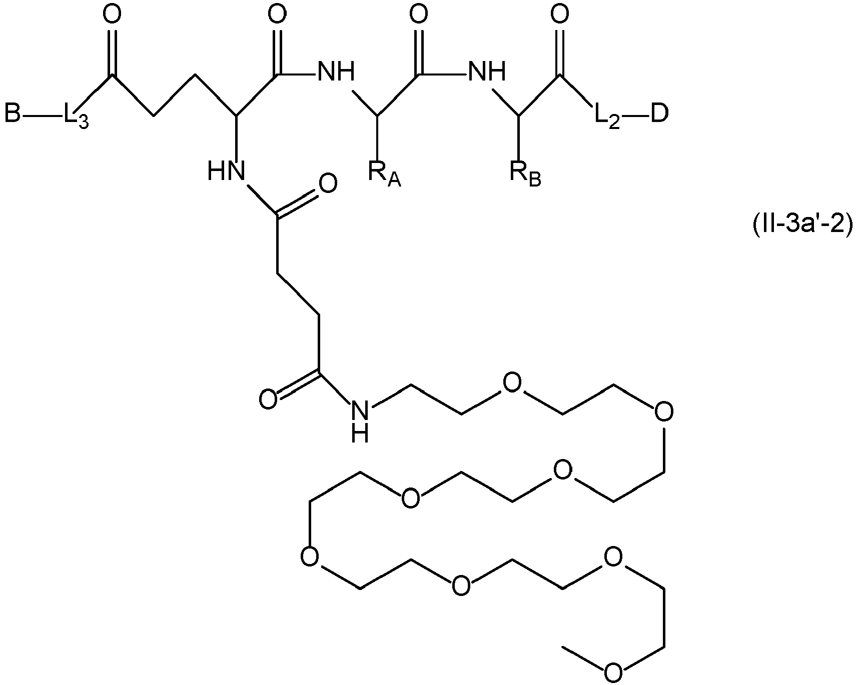
wherein L₂, L₃, D, R_{A}, R_{B}, and B are the same as those in formula (II-1); 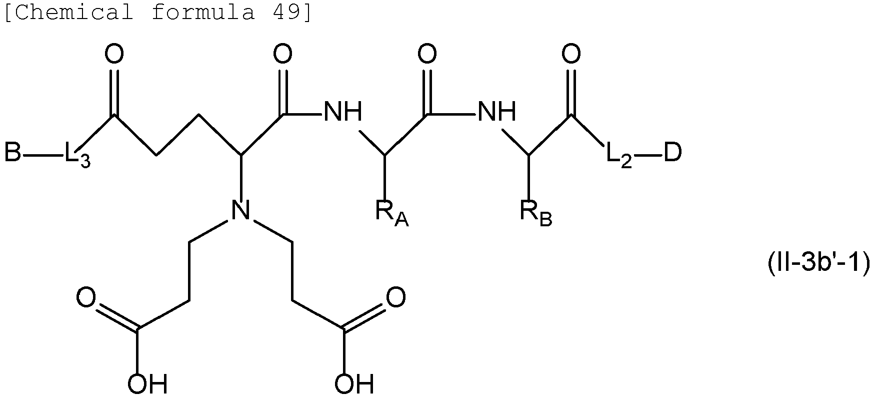
wherein
L₂, L₃, D, R_{A}, R_{B}, and B are the same as those in formula (II-1); 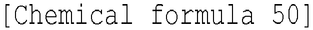 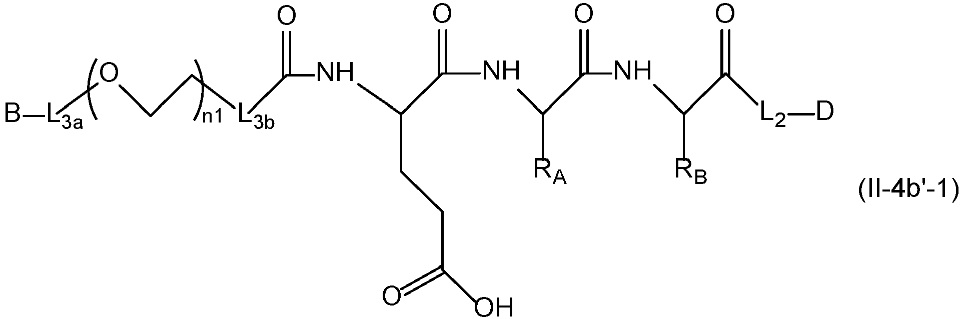
wherein
L₂, D, R_{A}, R_{B}, and B are the same as those in formula (II-1),
L₃ₐ and L_{3b} each independently represent a bond or a divalent group,
n1 is a integer of 3 to 20; 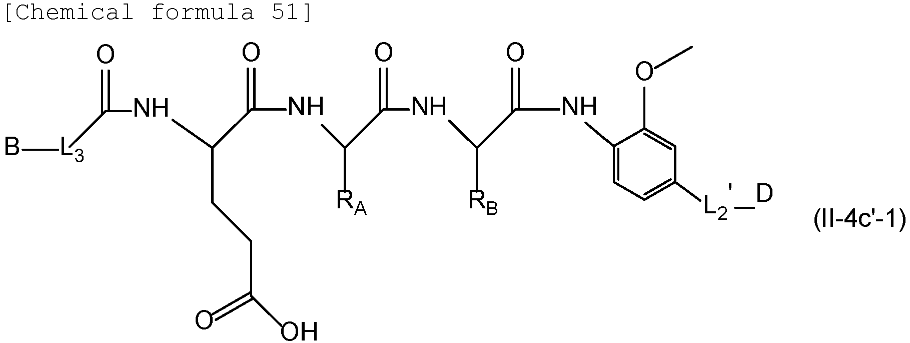
wherein
L₃, D, R_{A}, R_{B}, and B are the same as those in formula (II-1), and
L₂' represents a bond or a divalent group; or 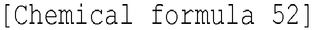 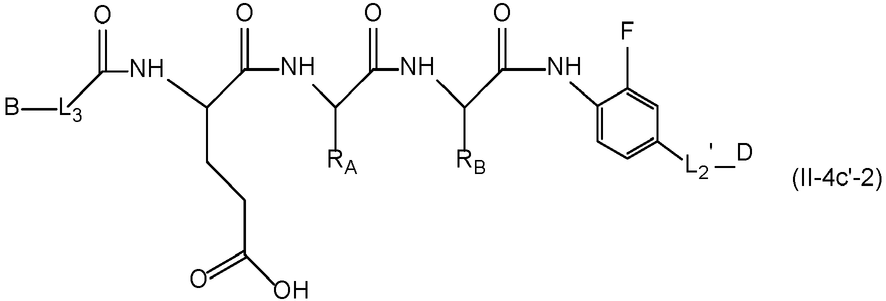
wherein
L₃, D, R_{A}, R_{B}, and B are the same as those in formula (II-1), and
L₂' represents a bond or a divalent group.

19. The compound or a salt thereof according to claim 15, wherein L₂ comprises a divalent group represented by the following structural formula: 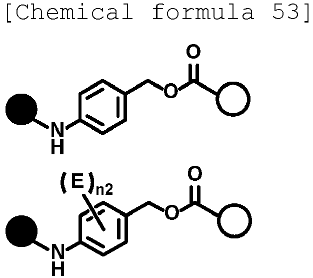
wherein, a black circle and a white circle each represent a bond,
the bond of the black circle is bonded to a carbonyl group adjacent to L₂, and
the bond of the white circle is bonded to D,
E represents an electron-withdrawing group, and
n2 is an integer of 1 to 4.

20. A reagent for derivatizing an antibody, the reagent comprising the compound or a salt thereof according to any one of claims 15 to 19.
